# EUROPEAN PATENT APPLICATION

(11) **EP 4 790 134 A1**
(43) Date of publication of application: **12.08.2026**
(21) Application number: 25382103.7
(22) Date of filing: 07.02.2025
(51) Int. Cl.: C12N 5/0789

(54) **METHOD FOR PROMOTING HEMATOPOIETIC STEM CELL (HSC) GENERATION, CELL COMPOSITION AND USES THEREOF**

(71) Applicant: Fundació Institut Hospital Del Mar D'Investigacions Mèdiques (IMIM), 08003 Barcelona (ES); Consorcio Centro de Investigación Biomédica en Red, 28029 Madrid (ES); Fundació Institut de Recerca Contra la Leucèmia Josep Carreras, 08916 Badalona, Barcelona (ES); Institució Catalana de Recerca i Estudis Avançats, 08010 Barcelona (ES)
(72) Inventor: BIGAS SALVANS, Anna, 08003 Barcelona (ES); GALÁN PALMA, Luís, 08003 Barcelona (ES); ESPINOSA BLAY, Lluís, 08003 Barcelona (ES); MENÉNDEZ BUJAN, Pablo, 08916 Badalona (ES)
(74) Representative: Hoffmann Eitle

(57) **Abstract**

The present invention pertains to the field of cell biology and regenerative medicine, particularly hematopoietic stem cell (HSC) technology and cell-based therapies. More specifically, the invention relates to methods and compositions for obtaining, enriching, and/or genetically modifying KDR⁺ cell populations, including mesodermal KDR⁺ cells and precursors thereof, for hematopoietic reconstitution, treatment of cytopenic disorders, and bone marrow regeneration in subjects affected by hematopoietic failure or undergoing myeloablative therapies.

## Description

### TECHNICAL FIELD OF THE INVENTION

The present invention pertains to the field of cell biology and regenerative medicine, particularly hematopoietic stem cell (HSC) technology and cell-based therapies. More specifically, the invention relates to methods and compositions for obtaining, enriching, and/or genetically modifying KDR⁺ cell populations, including mesodermal KDR⁺ cells and precursors thereof, for hematopoietic reconstitution, treatment of cytopenic disorders, and bone marrow regeneration in subjects affected by hematopoietic failure or undergoing myeloablative therapies.

### Background of the invention

Hematopoietic Stem Cells (HSCs) are a rare population of cells which occupy the apex of the hematopoietic hierarchy, giving rise to the entire blood system throughout the whole life ^{1,2}. Because of this crucial function, the in vitro generation of HSCs capable of full-in-vivo restoration of the blood system remains a major challenge in the field of regenerative medicine. Several strategies to generate HSCs have been tackled during the last decades (reviewed in ³), and in vitro production of multipotent HSCs that can successfully engraft in vivo has been reported⁴⁻⁹. However, the robustness and efficiency of this process is still quite limited, and the generated blood precursors are few, cannot repopulate the bone marrow at high frequency or cannot self-renew. Moreover, most approaches have relied on the ectopic expression of hematopoietic genes that can also cause leukemia, or in vivo teratoma formation, thus limiting the potential of these in vitro-generated cells to be used in the clinics ¹⁰. Recently, the importance of fine-tuning cell signaling to derive HSCs from human induced pluripotent stem cells in a transgene-free manner has been demonstrated ^{11,12}. This finding underscores the need to achieve more physiological gene activation during the specification of HSCs. In nature, HSCs are produced during a third wave of embryonic hematopoiesis from a specialized hemogenic endothelium found in the dorsal aorta, within the Aorta-Gonad-Mesonephros (AGM) region ^{13,14}. While the first hematopoietic wave produces primitive erythroid and myeloid cells, Erythroid/Myeloid progenitors (EMPs) are generated in a second wave in the yolk sac and other main vessels. However, cells with true repopulation capacity, or HSCs, are only produced during the third, intra-embryonic wave ¹⁵.

Embryoid bodies (EBs) are three-dimensional structures derived from Pluripotent Stem Cells that recapitulate certain aspects of the embryonic blood development, though they generally fail to produce bona-fide HSCs ³. Most of the hematopoietic processes in EBs resemble those occurring in the yolk sac during the first hematopoietic waves, but not the more complex processes that take place in the AGM region. Additionally, HSCs originating from the AGM region are rare and exhibit inefficient engraftment capacity, only acquiring the full features of adult HSCs later in the fetal liver ^{16,17}. Although the complexity of this developmental process has been extensively studied, many aspects remain unknown. Furthermore, the precise sequence and coordination of signals required for in vitro HSC formation are still unclear. We here conducted, for the first time, an unbiased genome-wide screen using the CRISPR activator technology (CRISPRa) ^{18,19} in mouse embryonic stem cells (mESCs) followed by an in vitro mesodermal/hemogenic specification protocol, and the in vivo transplantation of mesodermal KDR⁺ progenitors into immunocompromised mice to uncover the required components that guide the HSC fate. This novel strategy led to the identification of seven genes (*Spata2, Aass, Dctd, Eif4enif1, Guca1a, Eya2 and Net1,* collectively termed SADEiGEN) that, upon activation during mesodermal specification, enable KDR⁺ progenitors to differentiate into hematopoietic stem and progenitor cells (HSPCs) capable of serial engraftment and self-renewal in both primary and secondary transplantations. These HSPCs contribute to the erythroid, myeloid (including macrophages, neutrophils and basophils), and lymphoid (T and B cells) lineages in vivo, and they show HSC identity in vitro. Mechanistically, analysis of cell composition within the EBs after SADEiGEN activation reveals that SADEiGEN-induced EBs contain hemogenic progenitors that more closely resemble intraembryonic mesoderm specification (precursors of definitive blood) rather than extraembryonic primitive hematopoiesis. Overall, our invention highlights the crucial role of proper fate specification during early development to obtain definitive HSPCs that can multilineage engraft in primary and secondary recipients from pluripotent stem cells.

### BRIEF DESCRIPTION OF THE FIGURES

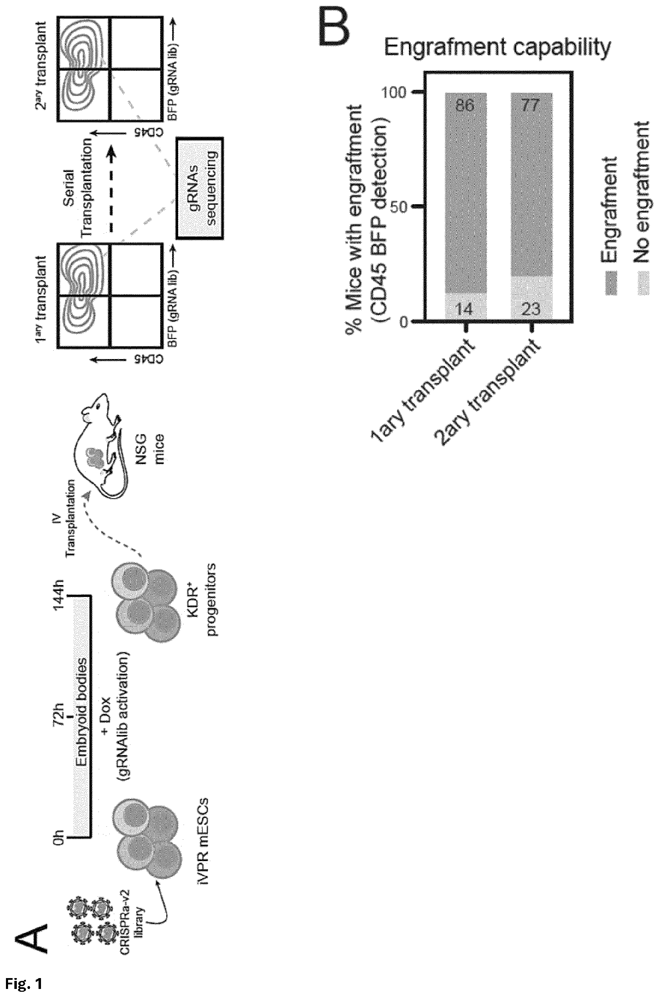

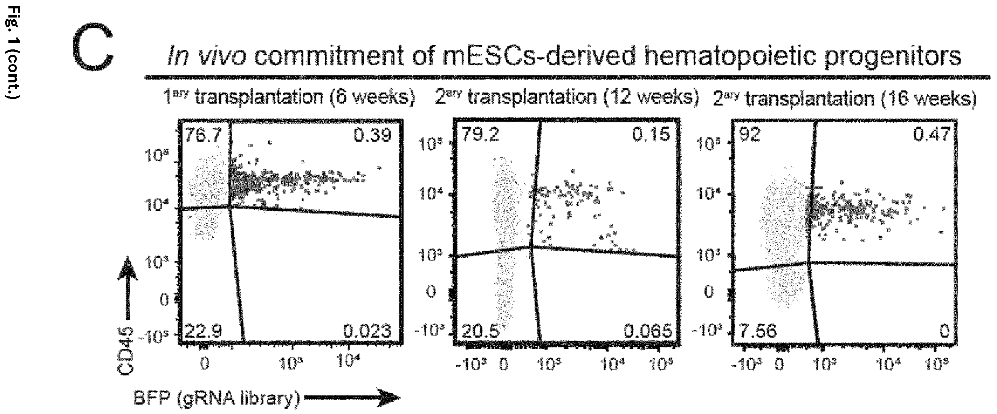

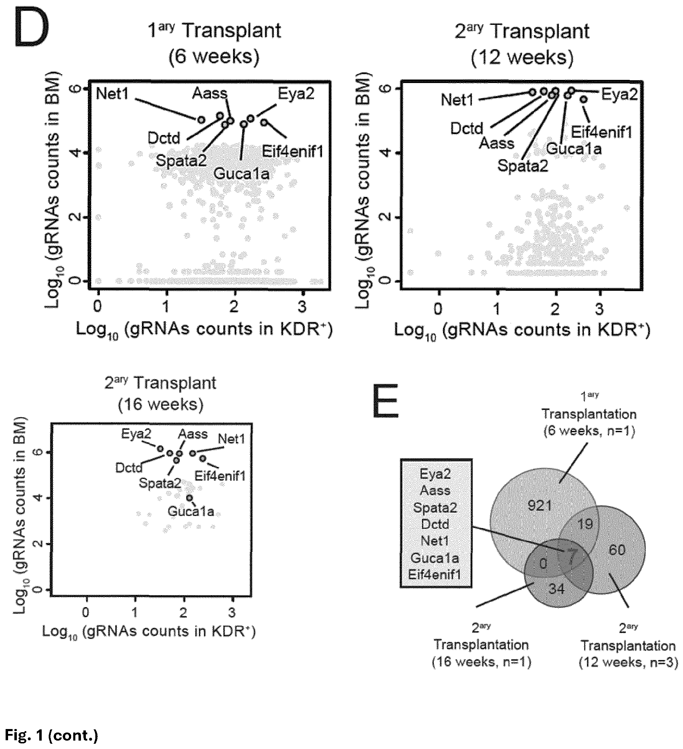

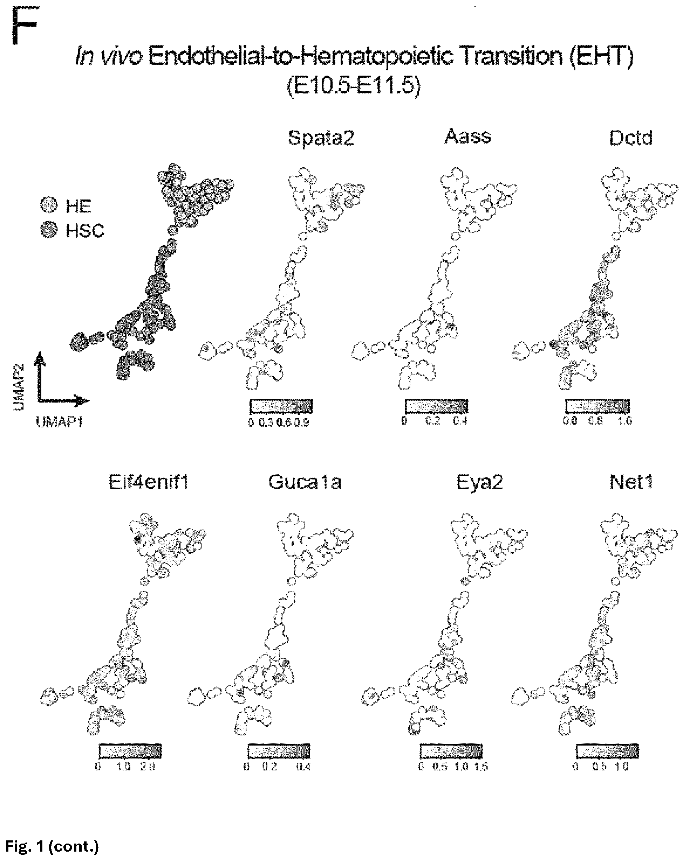

**Figure 1****. Genome-wide in vivo CRISPRa screen to uncover novel genes involved in hematopoietic stem cell specification from mouse embryonic stem cells. A.** Schematic representation of the Genome-wide in vivo CRISPRa screening. iVPR-mESCs were transduced with the CRISPRa-v2 library of gRNAs targeting gene promoters ²⁰ containing BFP in the vector (pCRISPRia-v2 backbone; Addgene Cat #84832). In each round of infection a multiplicity of infection (MOI) of 2.5 was used. gRNA library-infected iVPR-mESCs were differentiated towards KDR⁺ mesodermal progenitors by embryoid body differentiation for 144 hours. 72 hours EBs were supplemented with 1µg/mL doxycycline until 144 hours EBs to activate the VPR system. 6.5x10⁵ KDR⁺ cells from the gRNA library-induced cells were transplanted into NSG mice by retro-orbital injection (intravenous). Mice were screened for the presence of in vivo engraftment from hematopoietic cells (CD45⁺) derived from mESCs (BFP⁺) in primary recipients (6 weeks) and secondary transplantations (12 and 16 weeks). CD45⁺ BFP⁺ cells were isolated by FACS and gRNA composition in engrafted cells was sequenced. **B.** Quantification of engraftment capability in gRNA-library-iVPR-mESCs-derived KDR⁺ mesodermal progenitors in primary (n=21 mice) and secondary (n=13 mice) transplantations. Engraftment capability was assessed by the presence of CD45⁺ BFP⁺ cells in bone marrow of NSG mice. Threshold of 0.1% of positive cells was considered. **C.** Representative flow cytometry plots displayed on alive cells where BFP⁺ (mESC-derived) and CD45⁺ (hematopoietic) populations are analyzed in primary (6 weeks) and secondary (12 weeks and 16 weeks) transplants. **D.** Analysis of gRNA abundance in transplanted bone marrow in primary and secondary transplants. gRNA representation in KDR⁺ population was taken as the starting time point to analyze the gRNA enrichment in bone marrow, either in primary or secondary transplants. Normalized gRNA counts are represented in KDR⁺ (x axis) and bone marrow (y axis). Each dot represents an independent gRNA. **E.** Venn diagram to analyze common CRISPRa screen hits (gRNAs) across independent experiments in primary and secondary transplants. **F.** Expression of *Spata2, Aass, Dctd, Eif4enif1, Guca1a, Eya2, Net1* (SADEiGEN) genes in hemogenic endothelium (light pink) and hematopoietic stem and progenitor cell populations within the AGM region at E10.5-11.5 mouse embryos. Normalized counts from all cells belonging to that population are represented. AGM: Aorta-Gonad-Mesonephros region.

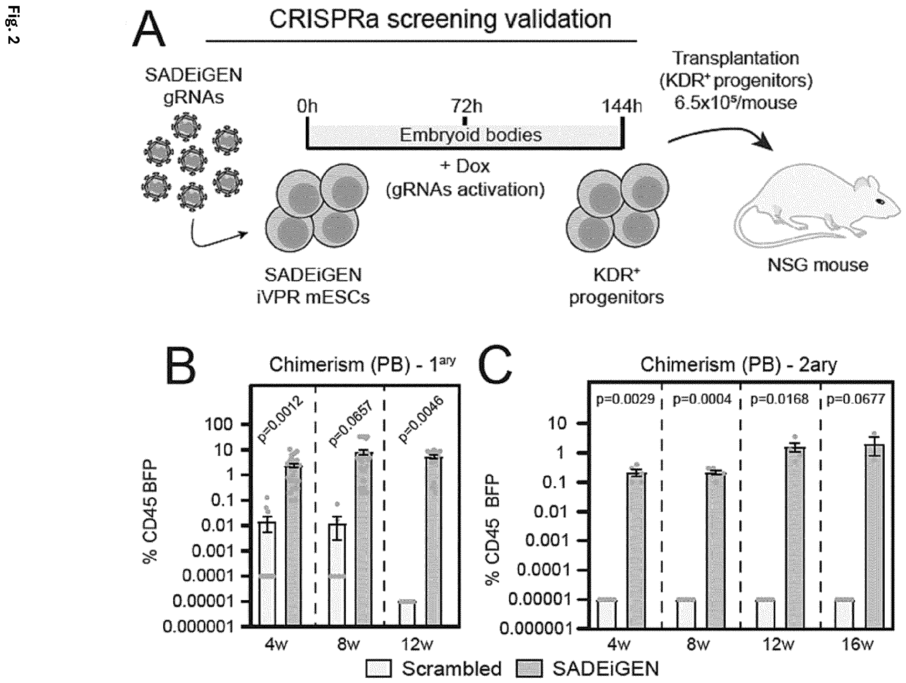

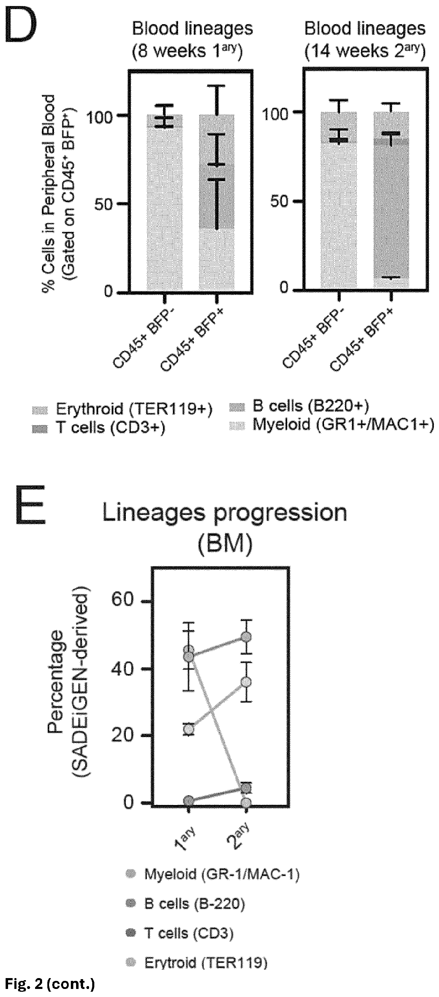

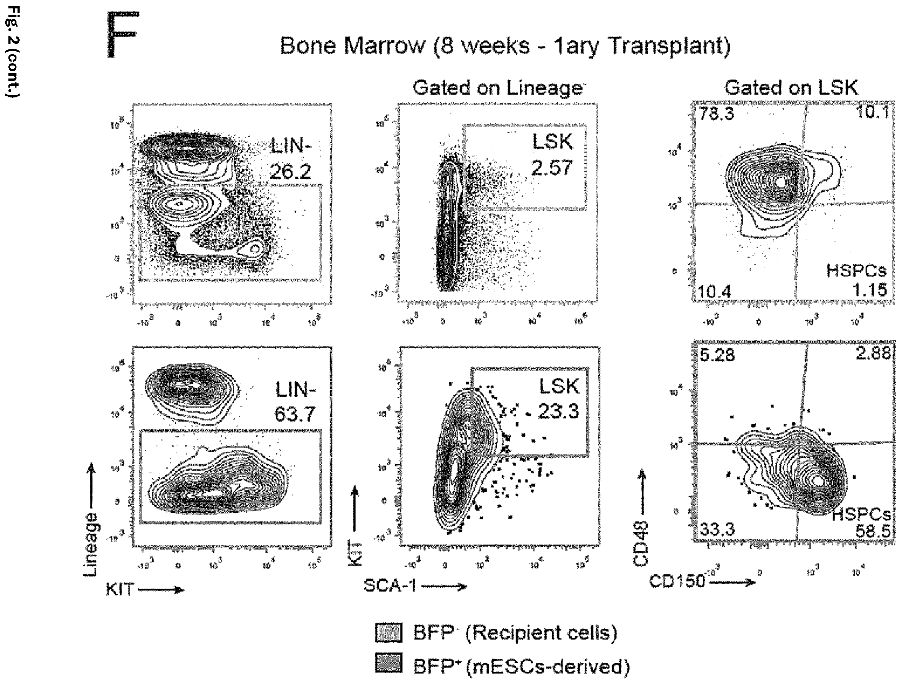

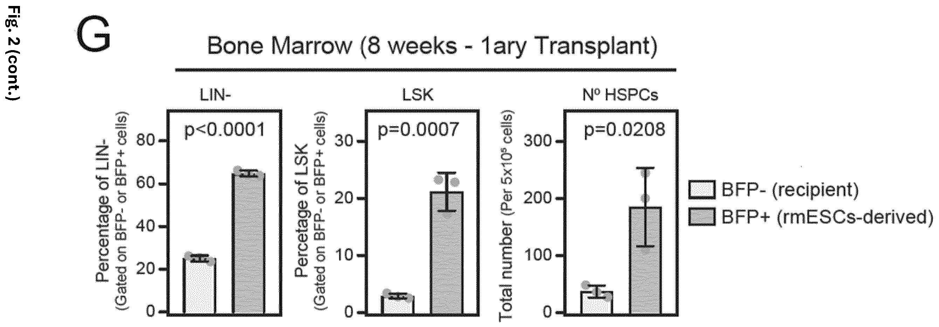

**Figure 2****. In vivo validation of CRISPRa screening confirms the generation of HSPCs which multilineage engraft from mESCs. A.** Schematics of CRISPRa screening validation. iVPR-mESCs were transduced with lentiviral vectors containing the 7 gRNAs to activate Spata2, Aass, Dctd, Eif4enif1, Guca1a, Eya2, Net1 (SADEiGEN) expression. Single-cell clones containing all 7 gRNAs into the same cell were generated (SADEiGEN iVPR-mESCs). SADEiGEN iVPR-mESCs were differentiated towards KDR⁺ progenitors for 144 hours of embryoid body-based differentiation. 72 hours EBs were supplemented with 1 µg/mL doxycycline until 144 hours EBs to induce SADEiGEN expression. KDR⁺ progenitors were isolated and transplanted into sublethally irradiated NSG mice by intratibial injection. 6.5x10⁵ KDR⁺ cells were injected per mouse. Each mouse bled at 4, 8 and 12 weeks in primary (1^{ary}) transplants and at 4, 8, 12 and 16 weeks in secondary (2^{ary}) transplants. **B-C.** Quantification of chimerism in peripheral blood (PB) in primary (B) and secondary (C) transplantation. Each dot represents a mouse. Error bars refer to standard error of the mean (SEM). **D.** Analysis of blood cell type composition in peripheral blood at 8 weeks of 1^{ary} (left panel) and 14 weeks of 2^{ary} (right panel) transplantation. Percentage was calculated based on either CD45⁺ BFP⁻ (NSG mouse recipient cells) or CD45⁺ BFP⁺ (SADEiGEN-mESCs-derived cells) gated population. Three independent mice were analyzed at each time point. Error bars represent standard deviation (SD). **E.** Analysis of blood lineage progression in bone marrow cells in 1^{ary} (8 weeks) and 2^{ary} (14 weeks) transplants. Three independent mice were analyzed at each stage. Dots represent the mean of each cell type, and error bars refer to SD. **F.** Representative flow cytometry plots of bone marrow at 8 weeks of a 1^{ary} transplant gated on BFP⁻ (left panel; recipient cells from the NSG mouse) and BFP⁺ (right panel; SADEiGEN-mESCs-derived cells) where hematopoietic stem and progenitor cells (HSPCs) are analyzed. LIN-: lineage negative cells; LSK: LIN- SCA1 + KIT+; HSPCs: LSK CD48- CD150+. **G.** Quantification of the number of LIN- (left panel), LSK (middle panel) and HSPCs (right panel) in recipient cells (BFP⁻) or SADEiGEN-derived (BFP⁺) cells from bone marrows at 8 weeks of a 1^{ary} transplant. For LIN- and LSK quantification, the total percentage based on BFP⁻ (NSG recipient) or BFP⁺ (SADEiGEN-derived) population was quantified; for HSPCs, total number of LSK CD48- CD150+ cells was quantified in 5x10⁵ events recorded by flow cytometry. Each dot represents an independent mouse. Error bars represent the SD.

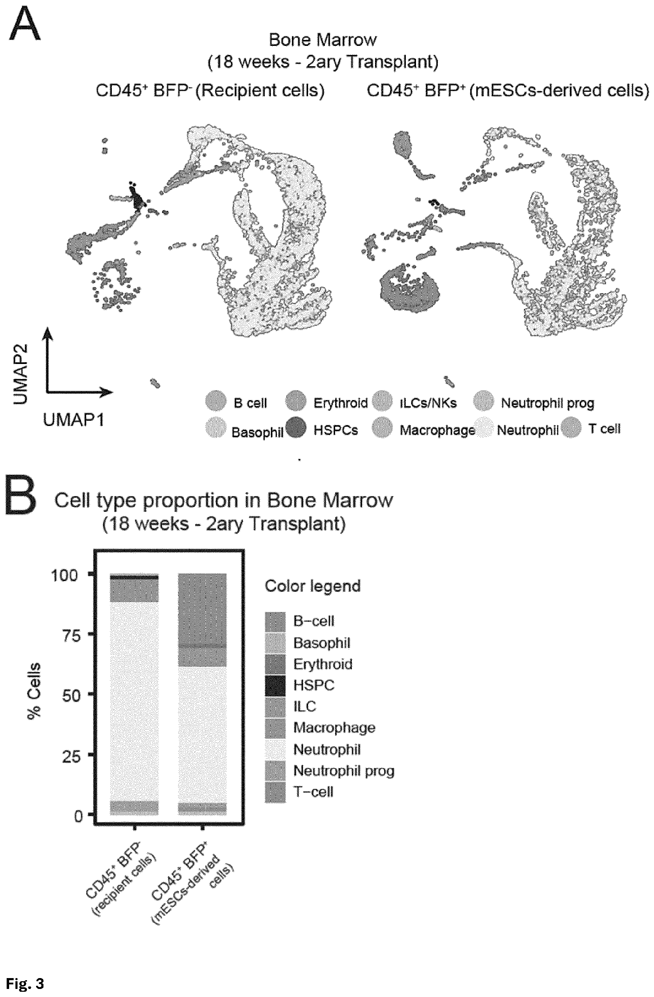

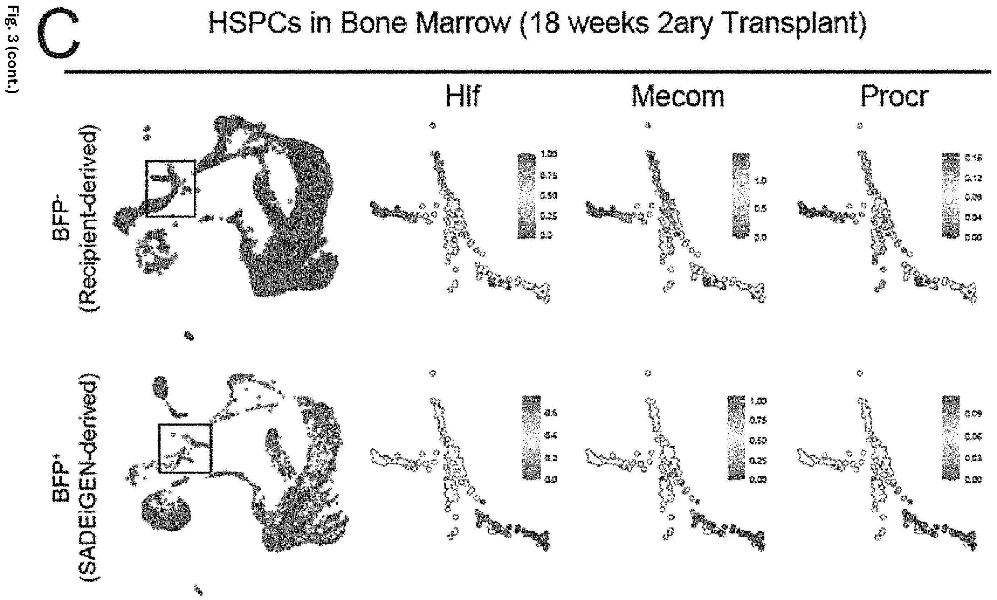

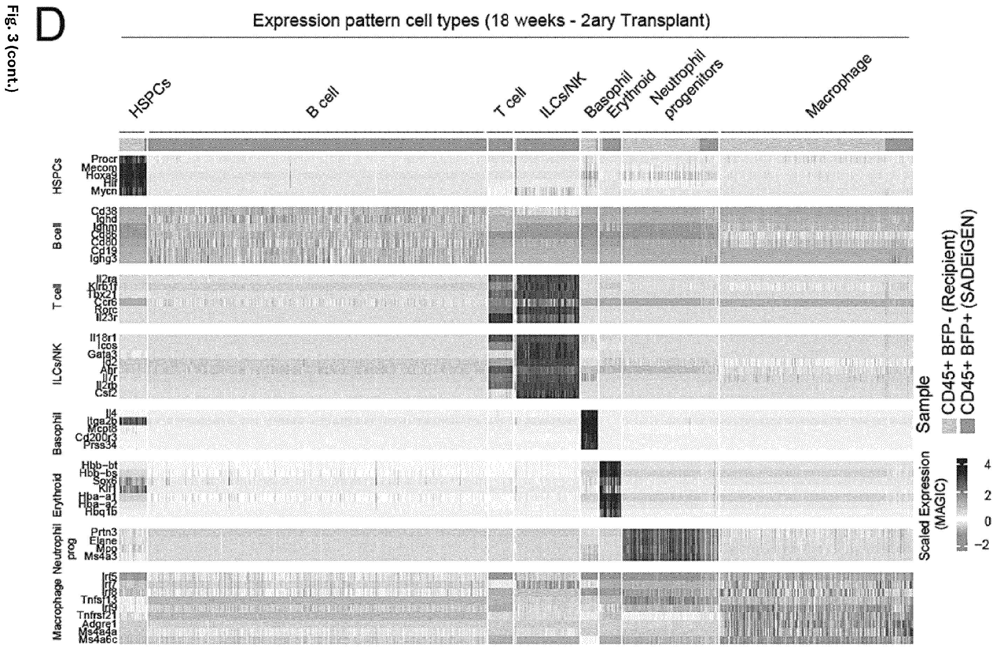

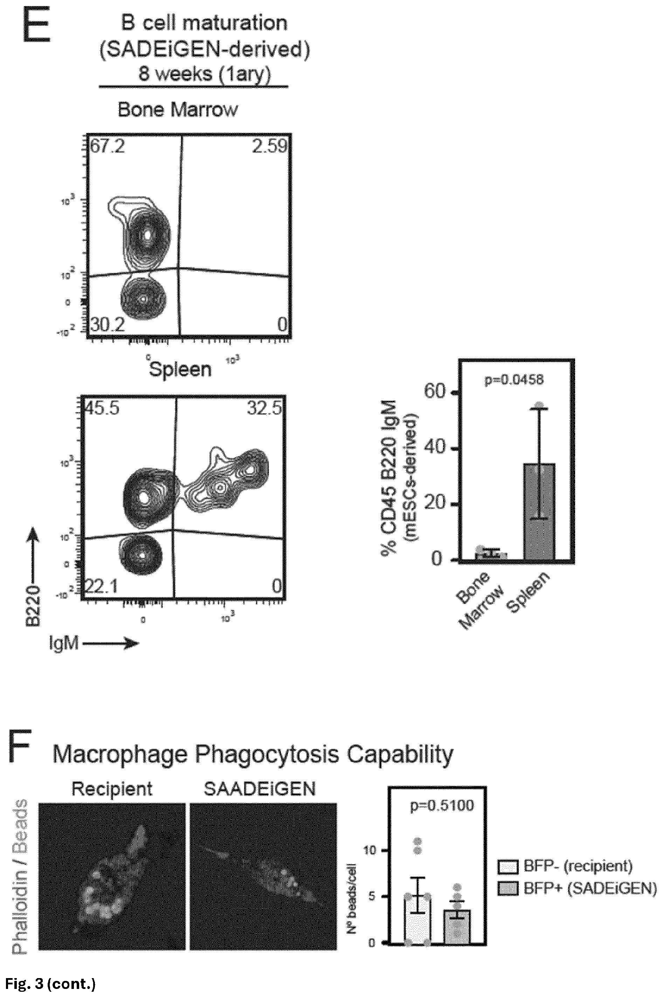

**Figure 3****. In vivo SADEiGEN-derived blood displays characteristics of functional maturation. A.** Uniform Manifold Approximation and Projection (UMAP) from sc-RNAseq of CD45⁺ bone marrow cells at 18 weeks of 2^{ary} transplantation. Both CD45⁺ BFP^{+/-} subsets are displayed in the UMAP. sc-RNAseq data was obtained by pooling CD45⁺ BFP⁻ or CD45⁺ BFP⁺ cells from 3 transplanted mice. **B.** Cell type proportion identified in the scRNA-seq dataset distinguishing between CD45 + BFP - (NSG cells, n=7,510 cells) and BFP + (SADEiGEN-derived, n=5,020 cells). **C.** Normalized and smoothed expression of HSPCs genes (Hlf, Mecom, Procr) in sc-RNAseq of bone marrow at 18 weeks (4.5 months) post-transplantation of secondary transplant. HSPC genes are considered based on specificity for HSC expression compared to multipotent progenitors (analysis based on data from Rodriguez-Fraticelli²³. Upper panel: HSPC genes from recipient cells (BFP⁻), with SADEiGEN-derived cells (BFP⁺) colored in gray. Bottom panel: HSPC genes from SADEiGEN-derived cells (BFP⁺), with recipient cells (BFP) colored in gray. Only a magnification of the cell populations marked at the left part of the general UMAP are considered for this representation. **D.** Heatmap of normalized scaled expression of marker genes associated to the different hematopoietic cell types. All cells from the scRNA-seq data from bone marrow of secondary transplanted mice at 18 weeks are included, except for those identified as neutrophils,. Cells, in columns, are sorted per identity and origin: CD45+BFP- (recipient NSG mice cells) population is displayed in gray panel; CD45+BFP+(SADEiGEN-derived cells) population is displayed in blue. Genes, in rows, are sorted per cell type association. **E.** Flow cytometry analysis of bone marrow (upper panel) and spleen (bottom panel) from 8 weeks 1ary transplanted mice. Analysis of B cell maturation was performed, and IgM expression was considered as a maturation marker. B220 marker indicates both immature precursors and more mature B lymphocytes. Right panel: quantification of percentage of mature B cells (based on % B-220+ IgM+ cells) in bone marrow and spleen at 8 weeks post-transplant (primary recipient). Only CD45 + BFP + (SADEiGEN-derived) cells were considered for the analysis. **F**. Representative confocal images of phagocytosis assays in macrophages (CD45⁺ MAC1⁺) derived from 8 weeks bone marrow cells (1ary transplantation). Left part: macrophages derived from recipient cells (BFP - ). Middle panel: macrophages derived from SADEiGEN-cells (BFP + ). Right panel: quantification of engulfment capability of recipient- or SADEiGEN-derived macrophages (CD45 + MAC-1 + BFP - /+ , respectively). Each dot represents an independent microscope field containing macrophages. Error bars correspond to ±SEM. Unpaired T-Test was performed.

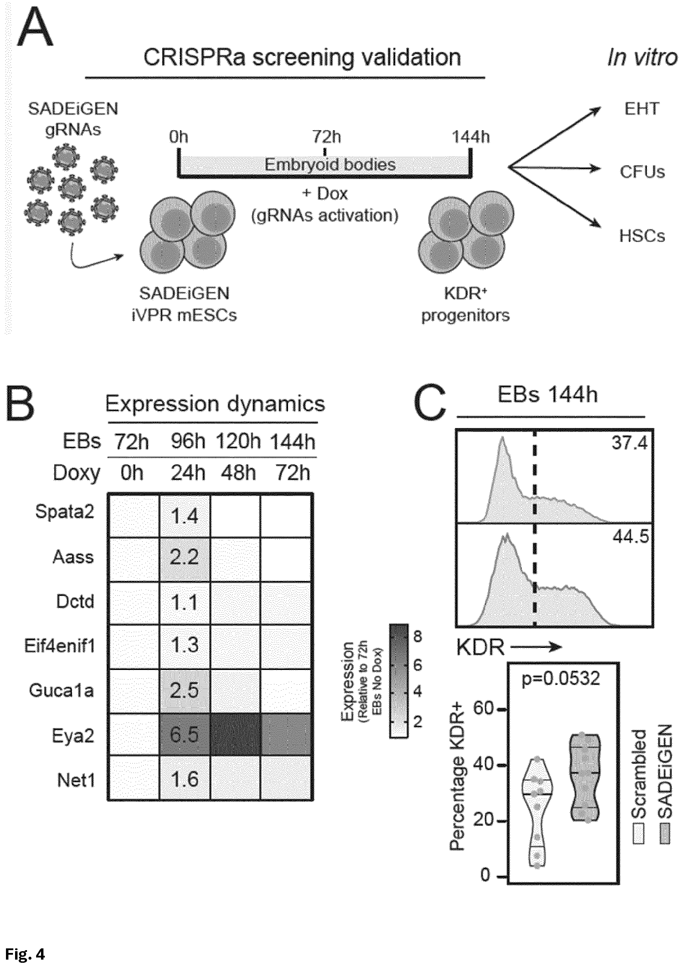

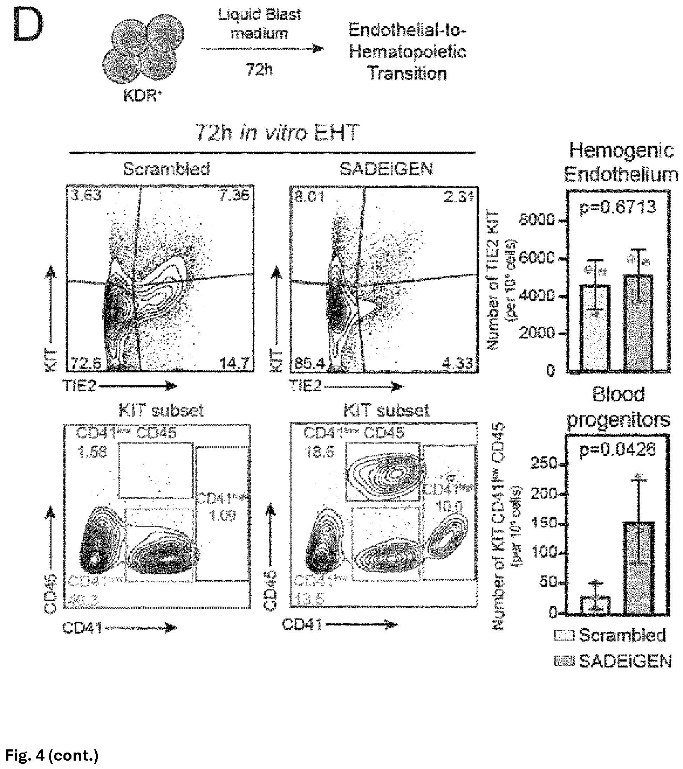

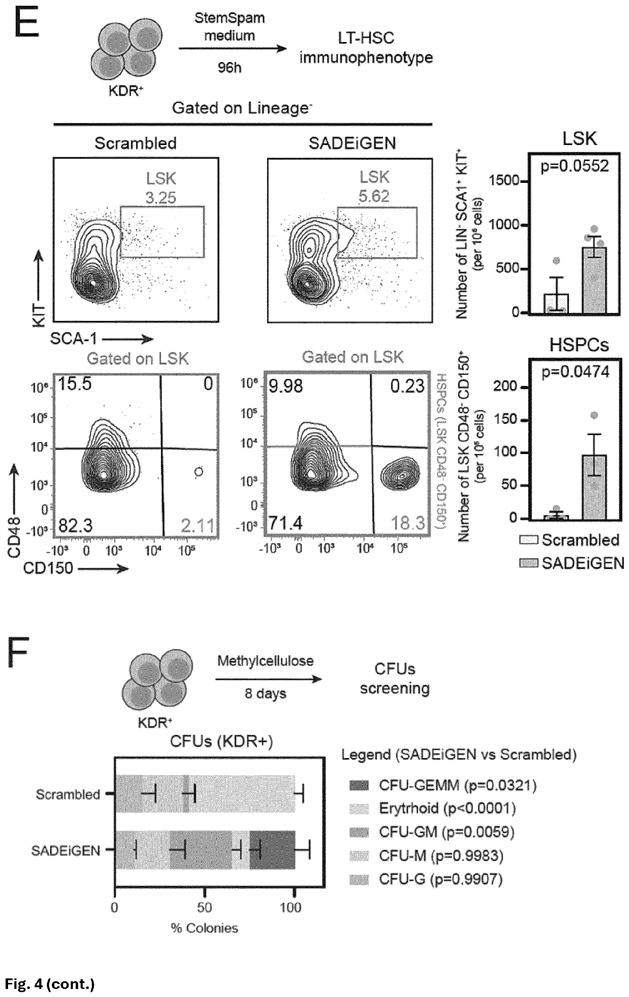

**Figure 4****. Activation of SADEiGEN during KDR⁺ differentiation induces hematopoietic stem and progenitor cell fate in vitro. A.** Schematic for testing in vitro hematopoietic potential of SADEiGEN-induced mESCs. SADEiGEN iVPR-mESCs were differentiated towards KDR⁺ progenitors for 144 hours of embryoid body-based differentiation. 72 hours EBs were supplemented with 1 µg/mL doxycycline until 144 hours EBs. KDR⁺ progenitors were isolated and further analyzed in vitro for Endothelial-to-Hematopoietic-Transition (EHT), Colony-Forming Units (CFUs) and HSC specification. **B.** Expression dynamics of SADEiGEN gene cocktail upon doxycycline-induced activation during embryoid body differentiation. Color code represents mRNA expression normalized relative to no doxycycline (0 hours, 72h EBs). *Tbp* was used as a house-keeping gene. Mean value of relative expression corresponds to average from two independent experiments. **C.** (Upper panel) Flow cytometry plots of KDR protein at 144 hours EBs from scrambled-gRNA (control; gray) and SADEiGEN (blue) activation. Gated on alive cells. (Bottom panel) Quantification of percentage of KDR⁺ at 144 hours EBs when scrambled-gRNA (control;gray) or SADEiGEN (blue) is induced. **D.** (Upper panel) Schematics of in vitro EHT induction. KDR⁺ cells are seeded in liquid blast medium (10⁵ KDR⁺ cells/9.6cm² well) for 72 hours. (Left medium panel) Flow cytometry analysis of endothelium (TIE2⁺ KIT⁻) and hemogenic endothelium (TIE2⁺ KIT⁺) populations in scrambled-gRNA and SADEiGEN-induced cells after 72 hours in liquid blast medium. (Right medium panel) Quantification of hemogenic endothelial cells in scrambled-gRNAs (gray) and SADEiGEN (blue) conditions. Each dot represents an independent experiment. (Left bottom panel) Flow cytometry analysis of early (KIT⁺ CD41⁺ CD45⁻) and more-committed hematopoietic (KIT⁺ CD41⁺ CD45^{low}) progenitor populations in scrambled-gRNA and SADEiGEN cells after 72 hours in liquid blast medium. Displayed populations are gated within KIT⁺ population. (Right bottom panel) Quantification of definitive hematopoietic progenitors (blood progenitors; KIT⁺ CD41⁺ CD45^{low}) in scrambled-gRNAs (gray) and SADEiGEN (blue) conditions. Each dot represents an independent experiment. Number of cells based on total number of cells analyzed (10⁵). **E.** (Upper panel) Schematics of in vitro HSC induction. KDR⁺ cells are seeded in StemSpan medium (3x10⁵ KDR⁺ cells/3.5cm² well) for 96 hours. (Left medium panel) Flow cytometry analysis of LSK (Lineage⁻ SCA-1⁺ KIT⁺) population in scrambled-gRNA and SADEiGEN-induced cells after 96 hours inStemSpan medium. LSK population was gated on Lineage negative population. (Right medium panel). Total number of LSK cells in scrambled-gRNAs (gray) and SADEiGEN (blue) conditions. Each dot represents an independent experiment. (Left bottom panel) Flow cytometry analysis of Hematopoietic Stem and Progenitor Cell population (HSPCs; LSK CD150⁺ CD48⁻) in scrambled-gRNA and SADEiGEN-induced cells after 96 hours inStemSpan medium. Displayed population is the percentage of cells gated within the LSK population. (Right bottom panel) Quantification of the number of HSPCs in scrambled-gRNAs (gray) and SADEiGEN (blue) conditions. Each dot represents an independent experiment. Number of cells based on total number of cells analyzed (10⁵). **F.** (Upper panel) Schematics of CFU assay. KDR⁺ cells were seeded in methylcellulose medium for 8 days. (Bottom panel) Quantification of multipotent progenitors (CFU-GEMM), oligopotent progenitors (CFU-GM), erythroid, macrophage and granulocyte colonies from KDR⁺ mesodermal progenitors from scrambled-gRNA and SADEiGEN cells. Represented data correspond to three independent experiments. A 2-way ANOVA test was performed to calculate statistical significance for each type of colony in scrambled and SADEiGEN conditions. Error bar indicates ±SD

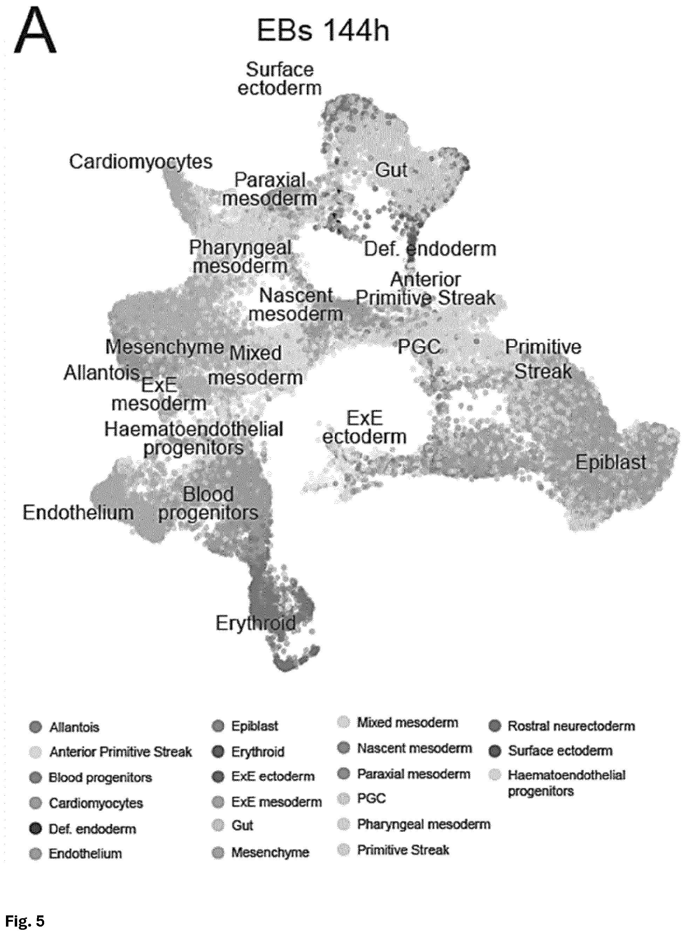

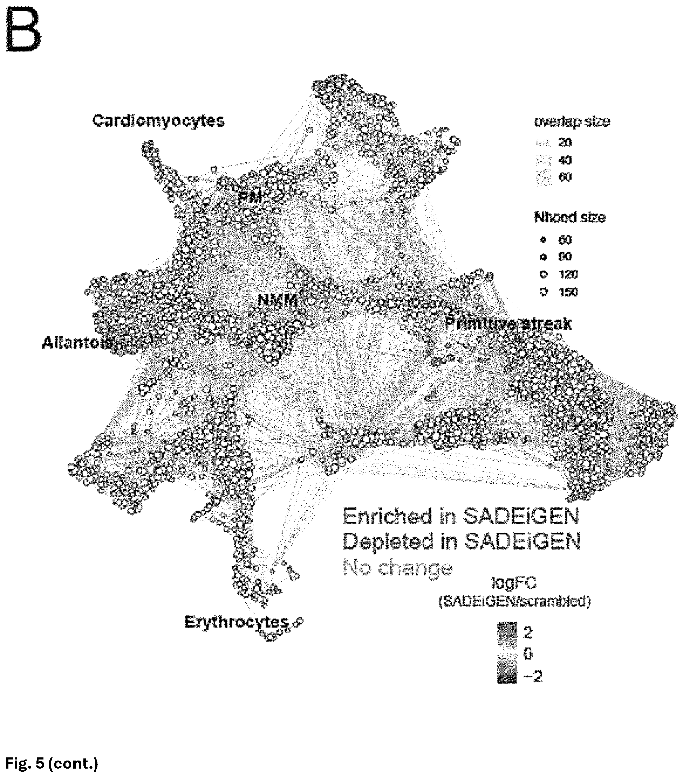

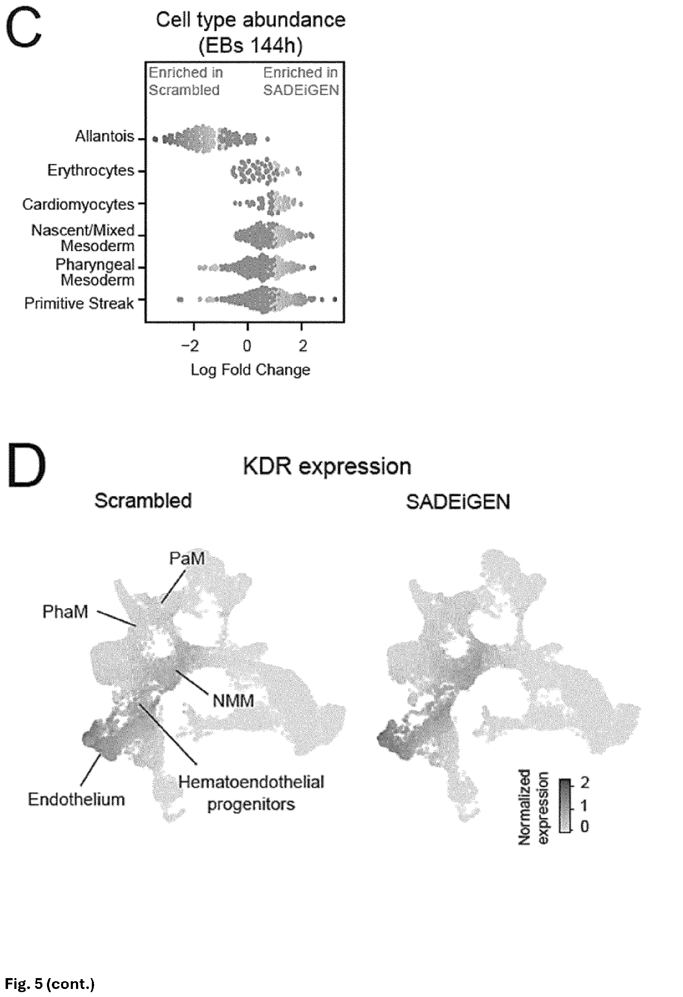

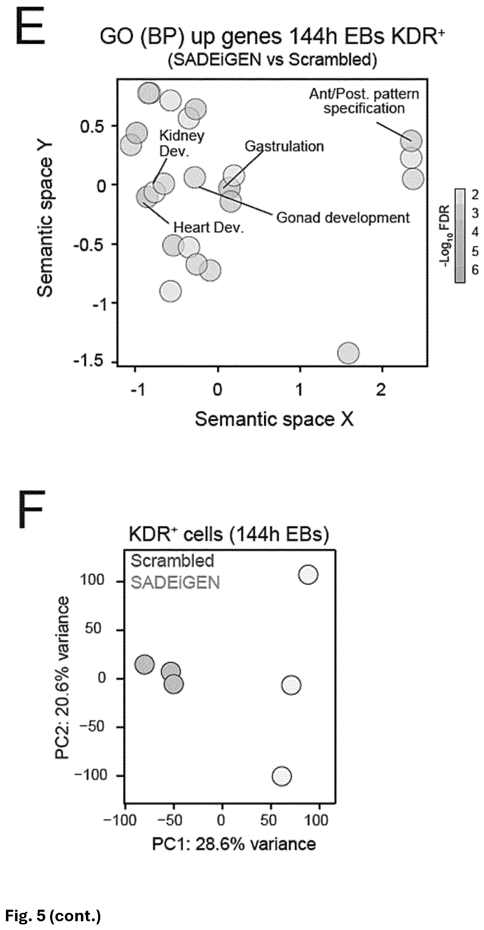

**Figure 5****. sc-RNAseq reveals the induction of intra-embryonic fate polarization of 144h SADEiGEN-EBs. A.** UMAP with the identified cell types in our sc-RNAseq 144h EBs dataset based on the mouse embryo atlas (E6.5-E8.5) annotations published in ²⁷. All dataset samples are included. **B.** Abstracted KNN graph of cell neighborhoods obtained by Milo superimposed on UMAP and colored by Log₂ Fold-change (FC) of abundance in SADEiGEN-derived samples relative to scrambled-derived. Graph edge width is proportional to cell overlap size between neighborhoods. Circle size is proportional to neighborhood size. **C.** Beeswarm plot that indicates Log₂ FC obtained from the differential abundance analysis over Milo cell neighborhoods per identified neighborhood groups of interest. **D.** UMAP showing the expression level of *kdr* gene in scrambled (left) or SADEiGEN-induced (right) samples. Replicates of each condition are displayed together for UMAP representation. Expression levels have been inferred and smoothed with MAGIC over normalized values. **E.** Semantic similarity scatter plot of Gene Ontology (GO) terms related to Biological Processes (BP) for the upregulated genes in SADEiGEN-induced 144h EBs (*Kdr*-expressing only cells). Only significant GO BP terms are represented (FDR < 0.01). Similar GO BP terms cluster together in the graph. **F**. Principal Component Analysis (PCA) based on the aggregated gene expression levels of all cells from Scrambled and SADEiGEN-induced 144h EBs samples. All expressed genes are considered.

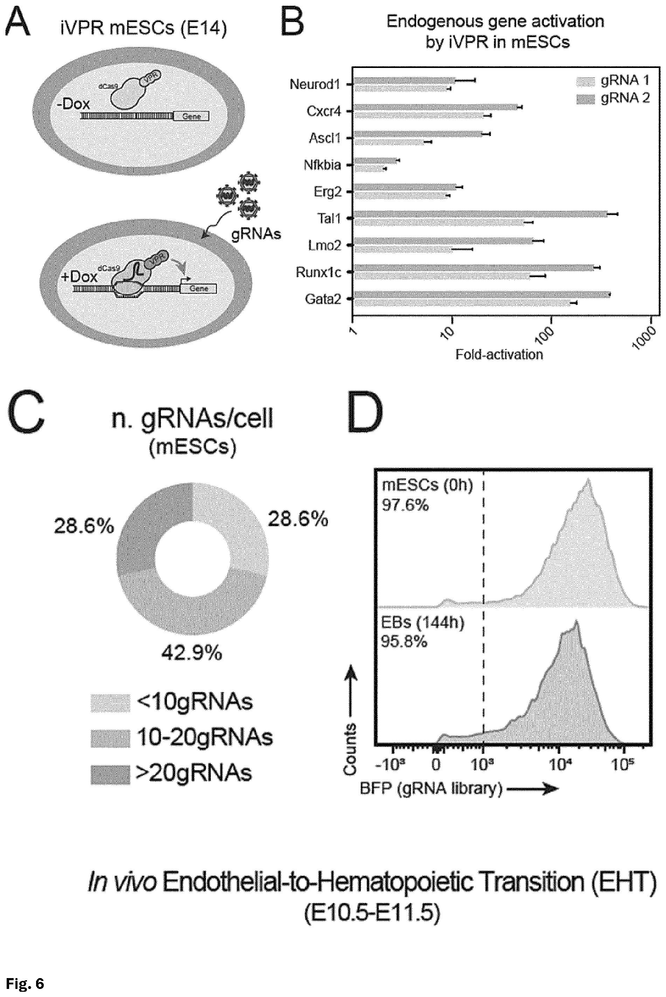

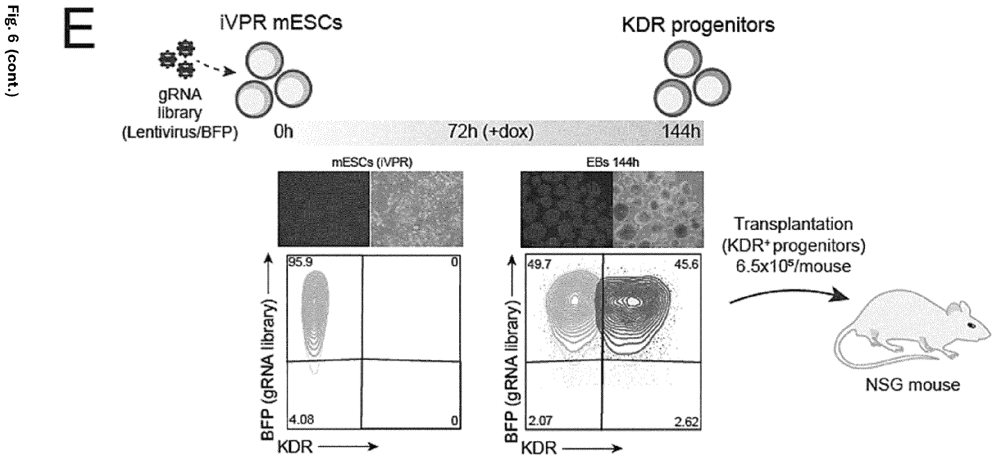

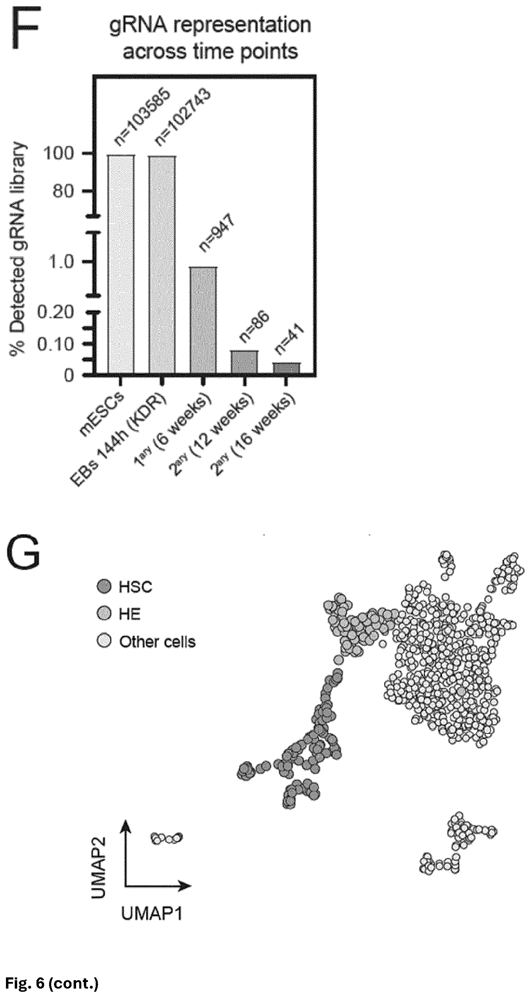

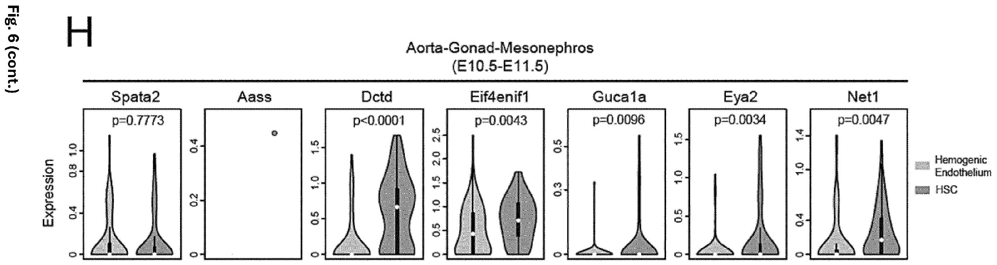

**Figure 6** **Doxycycline-inducible CRISPRa platform combined with differentiation to screen for genes which specify hematopoietic stem cell fate. A.** Schematic of doxycycline-inducible CRISPR activator (CRISPRa, VPR system) system introduced in mESC (E14 cell line) to activate endogenous genes. **B.** Endogenous gene activation of different target genes in iVPR-mESCs. Fold-activation is calculated based on relative mRNA levels to *Tbp* and untreated (no doxycycline) control. iVPR-mESCs were treated for 72 hours with 1µg/mL doxycycline. **C.** gRNA representation per single-cell clones after 4 rounds of transduction with lentiviral gRNA library. Pools of iVPR-mESCs were sorted by FACS (based on BFP expression) into individual clones after 4 rounds of infection, and number of different gRNAs were determined by sequencing. Percentage was retrieved by calculating the number of different gRNAs detected. Data come from 14 independent single-cell clones. **D.** Representative flow cytometry graphs of percentage of BFP expression (gRNAs) in mESCs (upper panel) and 144h EBs (bottom panel) after 4 rounds of infection. **E.** Schematics of the differentiation from mESCs towards KDR⁺ mesodermal progenitors. Representative images of cells and flow cytometry for BFP in each time point (mESCs and 144h EBs) are represented. 144h EBs were disaggregated, KDR⁺ cells were isolated and 6.5x10⁵ KDR⁺ cells were transplanted into NSG mice which have been sublethally irradiated (2Gy) prior to transplantation. **F.** Analysis of gRNA representation in each timepoint: mESCs, 144h EBs, primary transplantation (1.5 months or 6 weeks) and secondary transplantation (3 months or 12 weeks and 4 months or 16 weeks). Bar height represents the percentage of gRNAs detected based on the total number of gRNAs from the library. **G.** UMAP from the single-cell RNA sequencing (sc-RNA-seq) of the in vivo endothelial-to-hematopoietic transition (EHT) in embryonic days 10.5 and 11.5 from mouse embryos. Data from ²¹. HSC: Hematopoietic Stem cell; HE: Hemogenic Endothelium. **H.** UMAP from Hemogenic Endothelium (HE) and Hematopoietic Stem Cell (HSC) clusters (sc-RNA-seq) displaying expression level of *Spata2, Aass, Dctd, Eif4enif1, Guca1a, Eya2, Net1* (SADEiGEN) genes. Color code represents the expression level (normalized gene expression) of each gene.

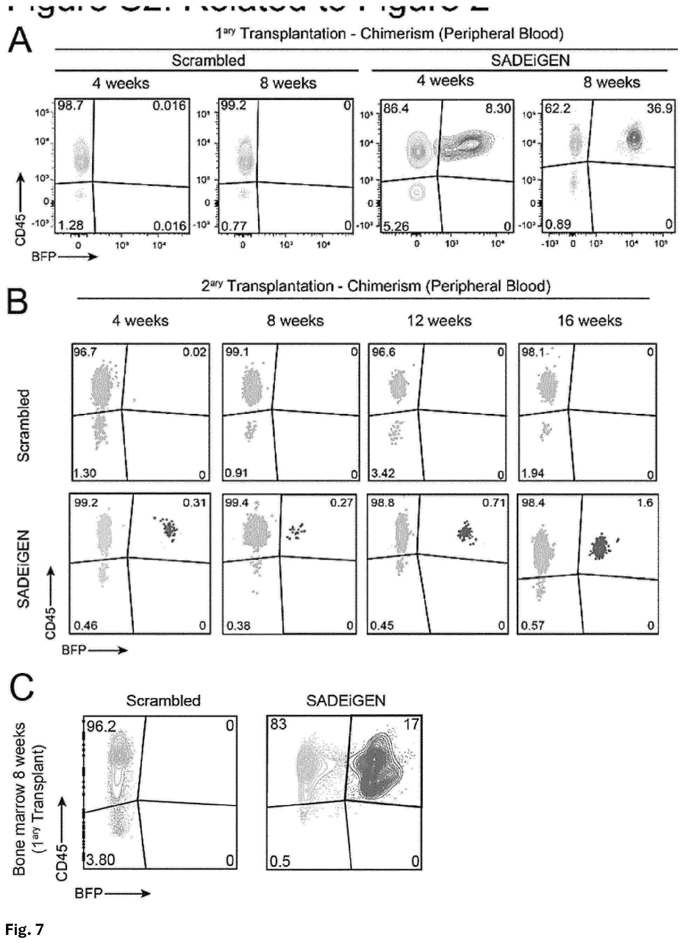

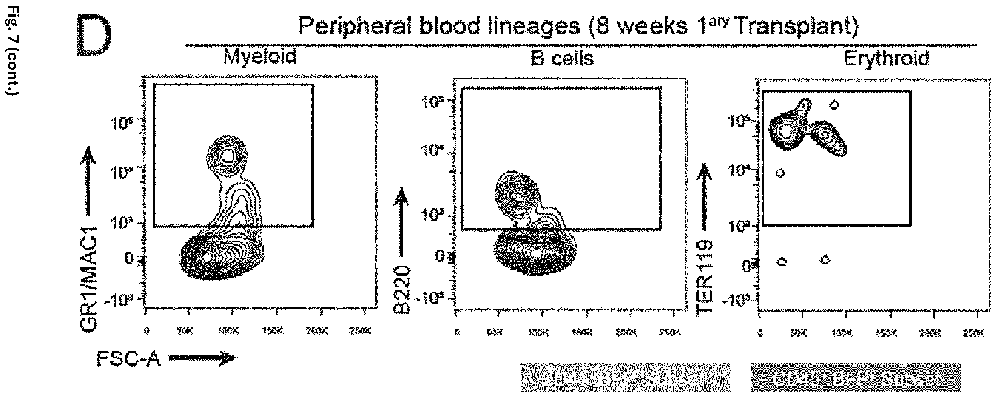

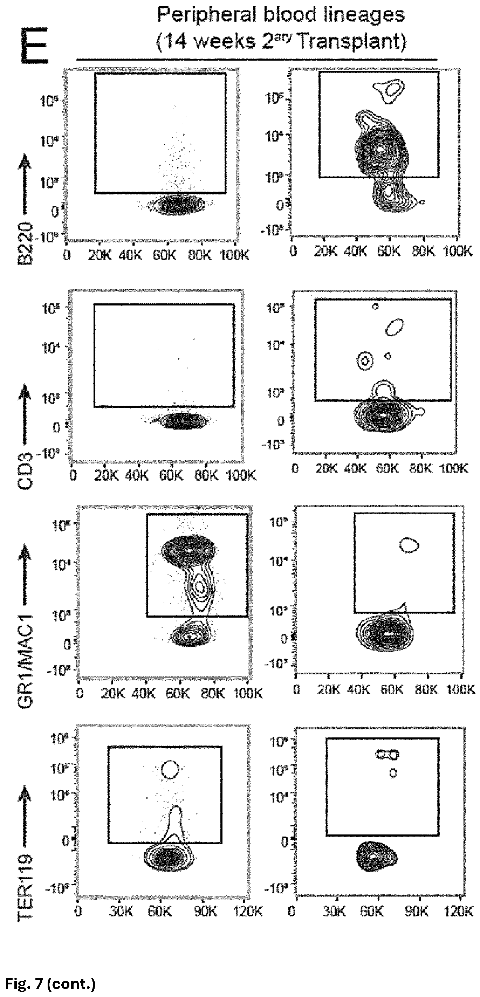

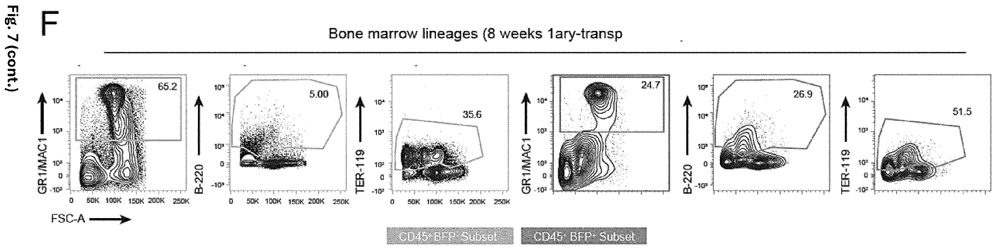

**Figure 7** **Analysis of blood chimerism upon SADEiGEN-KDR⁺ cell transplantation.** Representative flow cytometry plots of chimerism at 4 and 8 weeks in 1^{ary} **(A)** and at 4, 8, 12, 14 weeks in 2^{ary} **(B)** transplants after scrambled- KDR⁺ (control) or SADEiGEN- KDR⁺ transplantation. **C.** Representative flow cytometry graphs from bone marrow at 8 weeks after a 1^{ary} transplantation of scrambled- KDR⁺ or SADEiGEN- KDR⁺ cells. **D-E.** Representative flow cytometry plots from blood lineages detected at 8 weeks 1^{ary} (D) and at 14 weeks of 2^{ary} (E) transplants. Lineages derived from CD45⁺ BFP^{-/+} gated cells. B-220: B cells, CD-3: T cells; GR-1/MAC-1: myeloid; TER-119: erythroid. **F.** Representative flow cytometry plots from cell lineages detected at 8 weeks of 1^{ary} transplants in bone marrow. Cell lineages were determined within the CD45⁺ subset for BFP⁻ (recipient) and BFP⁺ (SADEiGEN-derived) cells.

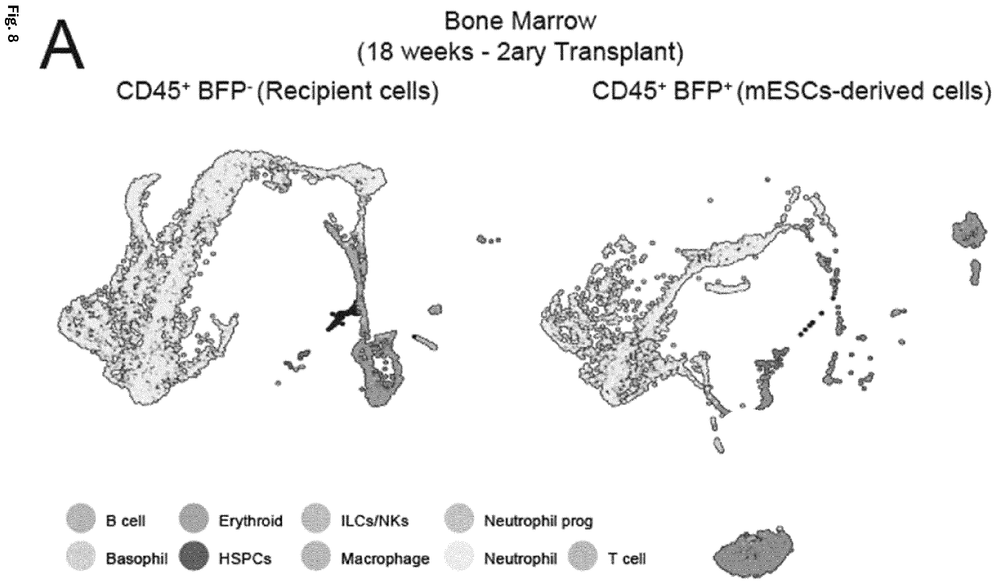

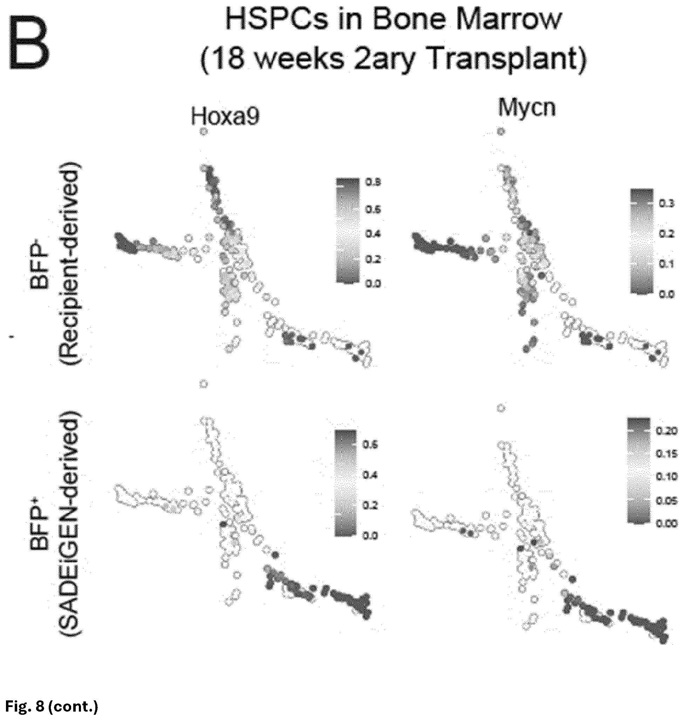

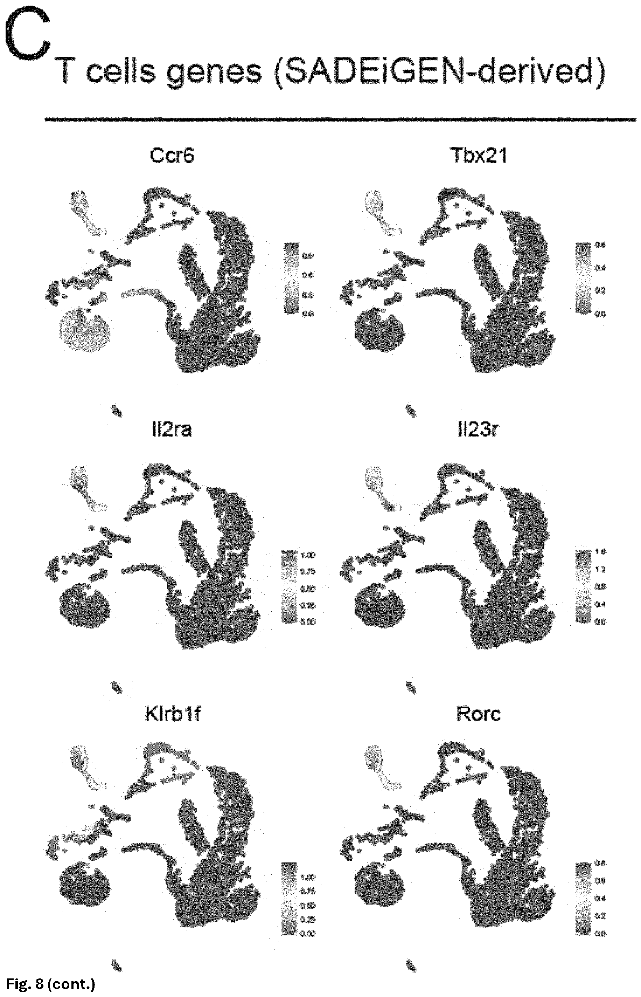

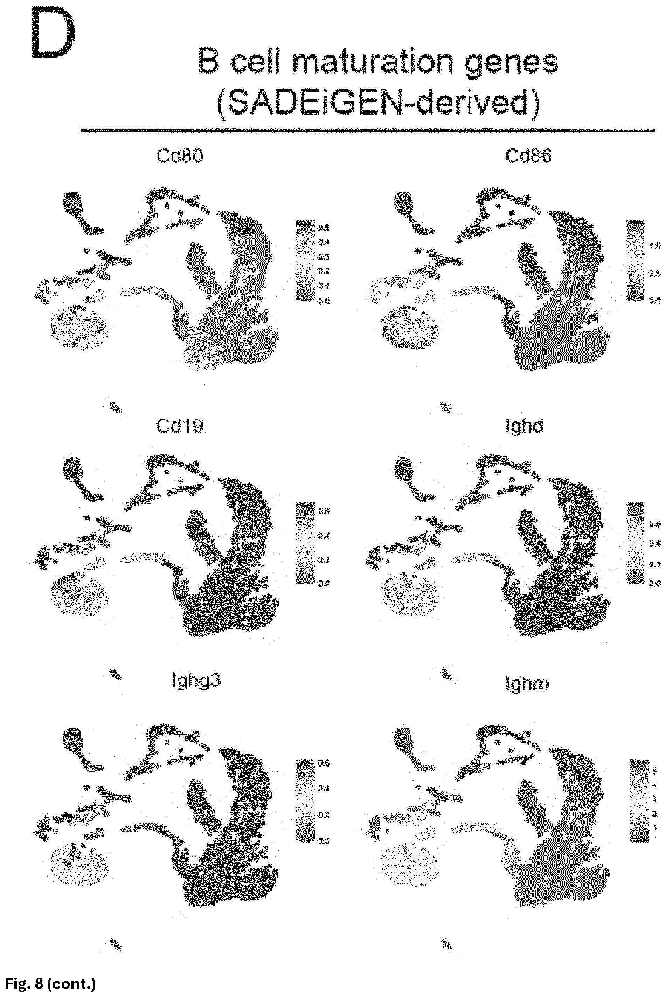

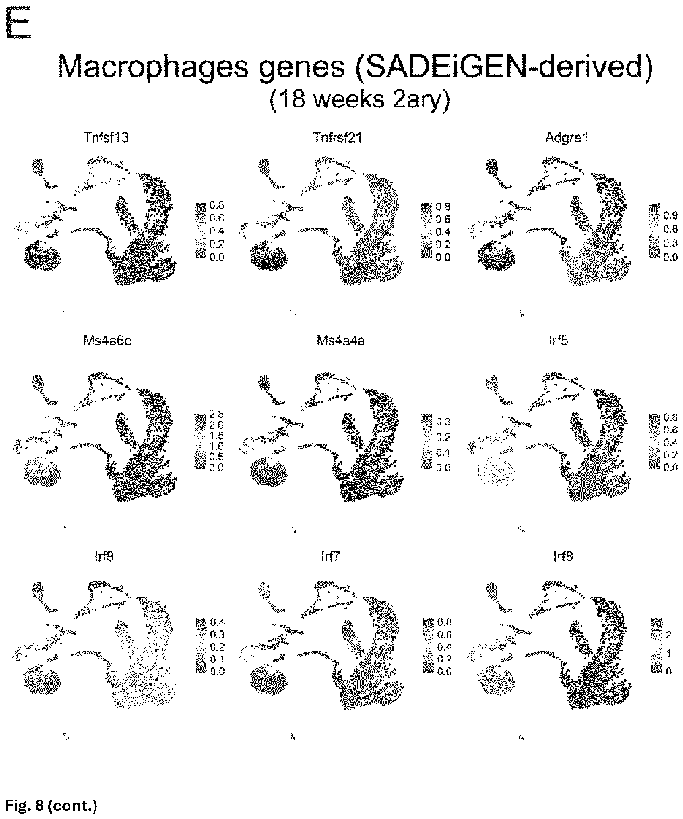

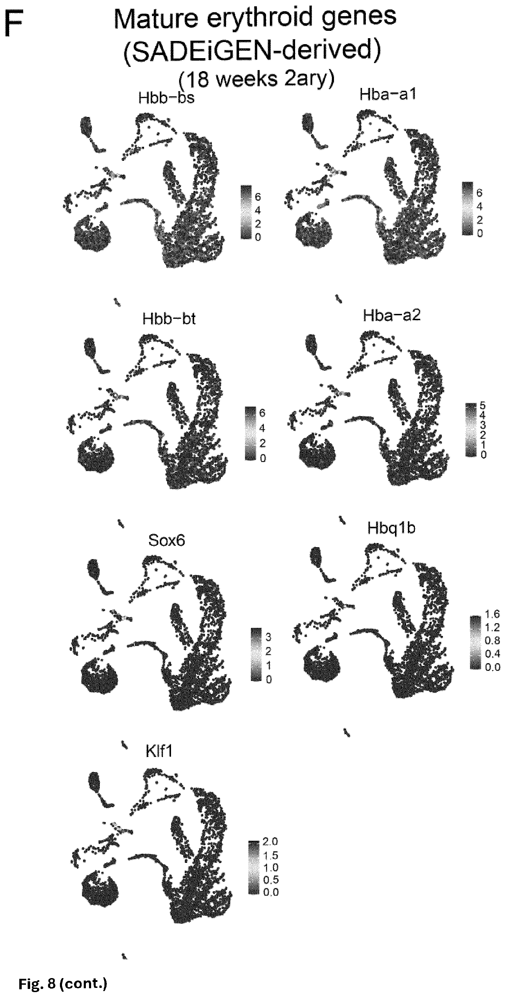

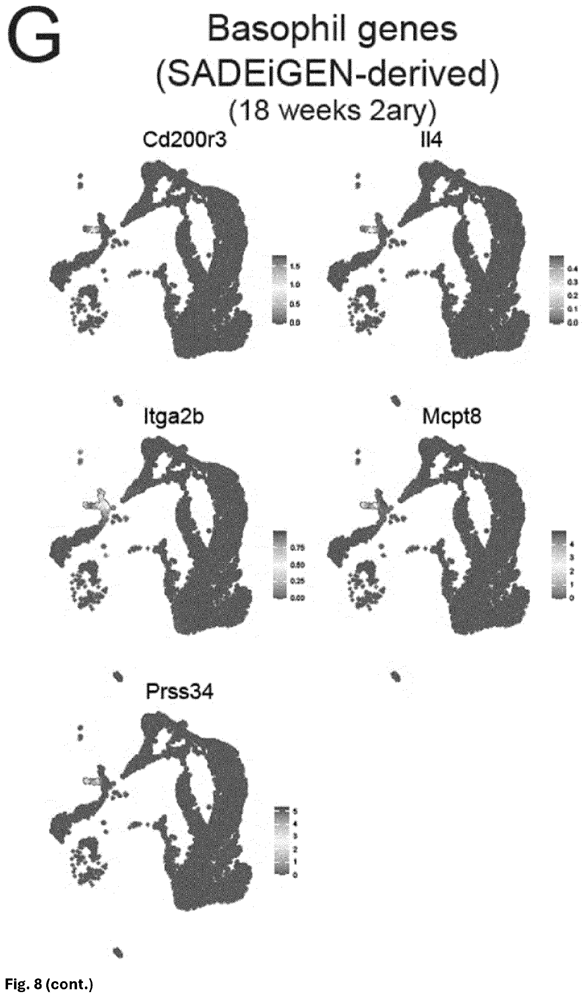

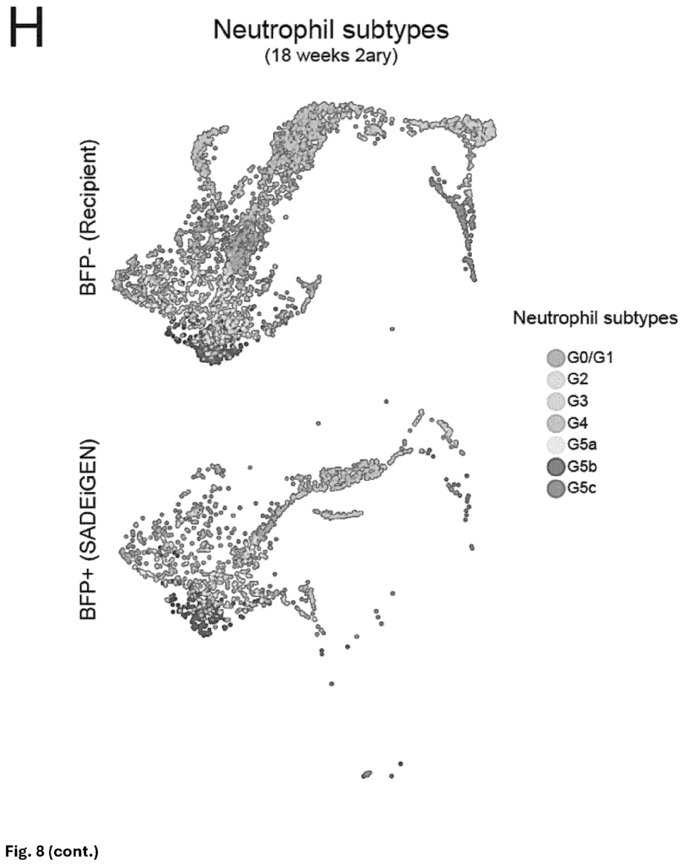

**Figure 8** **sc-RNAseq analysis of CD45⁺ bone marrow cells transplanted with SADEiGEN-KDR⁺ progenitors at 18 weeks of 2^{ary} Transplantation. A.** UMAP of the sc-RNA-seq from CD45⁺ bone marrow cells where both recipient (BFP-, left panel) and SADEiGEN-derived (BFP+, right panel) hematopoietic cells were analyzed. Each color represents a different blood cell type identified. HSPC: Hematopoietic Stem and Progenitor Cell; ILC: Innate lymphoid cell. **B.** Normalized expression of HSPCs genes (Hoxa9, Mycn) in sc-RNAseq of bone marrow at 18 weeks (4.5 months) post-transplantation of secondary transplant. Upper panel: HSPC genes from recipient cells (BFP⁻), with SADEiGEN-derived cells (BFP⁺) colored in gray. Bottom panel: HSPC genes from SADEiGEN-derived cells (BFP⁺), with recipient cells (BFP⁻) colored in gray. Only a magnification of the cell populations marked at the left part of the general UMAP are considered for this representation. **C.** Normalized expression of adult mature erythroid genes (Hbb-bs, Hba-a1, Hbq1b, Sox6, Hbb-bt, Hba-a2, Klf1) in sc-RNAseq of bone marrow at 18 weeks (4.5 months) post-transplantation of secondary recipients. Only CD45+ BFP+ cells (SADEiGEN-derived) were considered. **D.** Normalized expression of B cell maturation genes (Cd19, Cd80, Ighm, Cd86, Ighd, Ighg3) in sc-RNAseq of bone marrow at 18 weeks (4.5 months) post-transplantation of secondary recipients. Only CD45 + BFP + cells (SADEiGEN-derived) were considered. **E.** Normalized expression of macrophages genes (Tnfsf13, Tnfrsf21, Adgre1, Ms4a6c, Ms4a4a, Irf5, Irf7, Irf8, Irf9) in sc-RNAseq of bone marrow at 18 weeks (4.5 months) post-transplantation of secondary recipients. Only CD45 + BFP + cells (SADEiGEN-derived) were considered. **F.** Normalized expression of adult mature erythroid genes (Hbb-bs, Hba-a1, Hbq1b, Sox6, Hbb-bt, Hba-a2, Klf1) in sc-RNAseq of bone marrow at 18 weeks (4.5 months) post-transplantation of secondary recipients. Only CD45+ BFP+ cells (SADEiGEN-derived) were considered. **G.** Normalized expression of basophil genes (Cd200r3, Il4, Itga2b, Mcpt8, Prss34) in sc-RNAseq of bone marrow at 18 weeks (4.5 months) post-transplantation of secondary recipients. Only CD45 + BFP + cells (SADEiGEN-derived) were considered. **H**. UMAP of scRNA-seq from the neutrophil clusters where different subtypes of neutrophils are distinguished. G0-G4 neutrophils correspond to more immature subtypes, whereas G5 subclasses represent more mature stages.

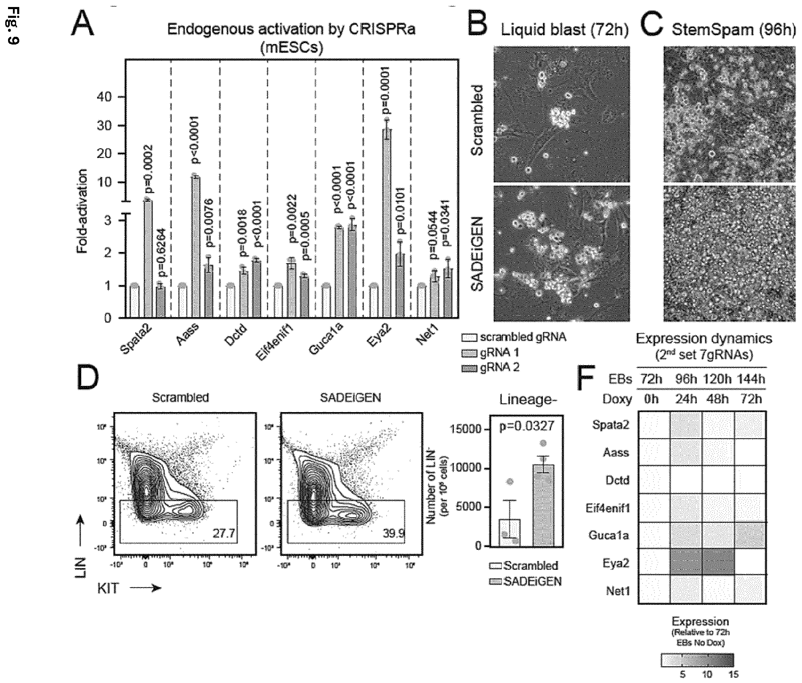

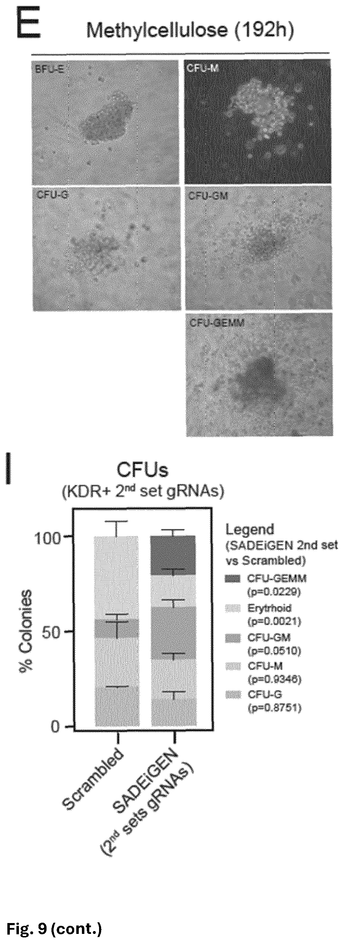

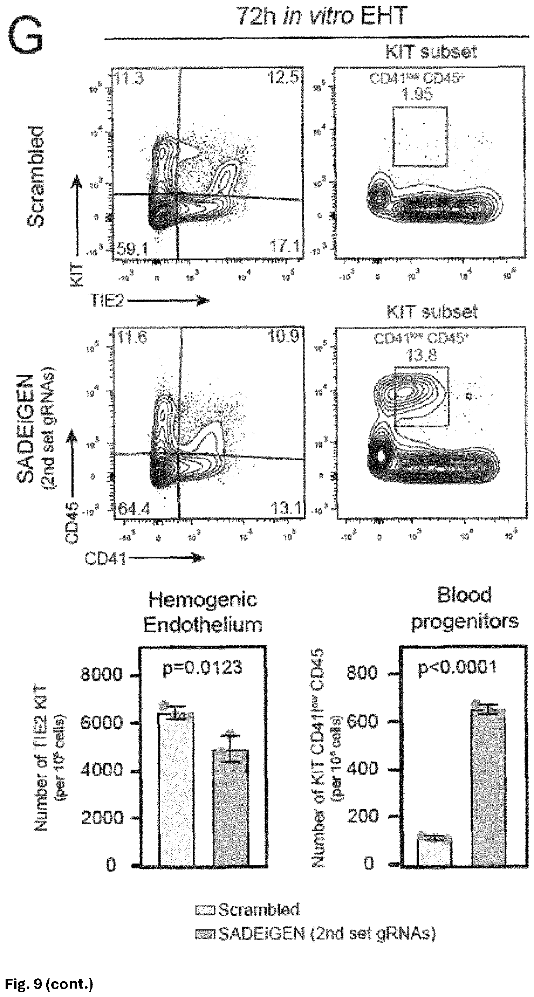

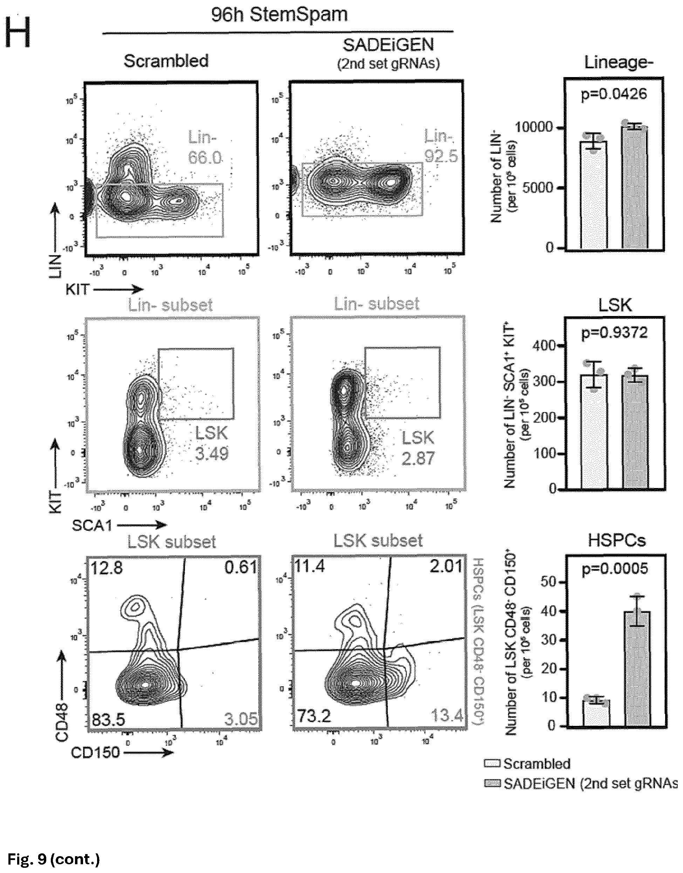

**Figure 9** **In vitro activation of SADEiGEN during KDR⁺ specification confirms the potential to form blood progenitors from mESCs in vitro. Related to** **Figure 4****. A.** Endogenous activation of *Spata2, Aass, Dctd, Eif4enif1, Guca1a, Eya2,* Net1 genes after 72 hours CRISPRa (VPR) induction in mESCs. Relative RNA levels were calculated using the house-keeping gene *Tbp,* and fold-activation levels were obtained using an uninduced control (scrambled; light gray). 2 independent gRNAs were used to target endogenous promoter regions of each gene: light blue represents gRNA 1 (identified as hit during the genome-wide CRISPRa screen) and darker blue marks gRNA2 (independent gRNA designed to target the promoter region). Bars indicate mean, whereas error bars represent ± standard deviation (SD). **B.** Representative bright-field microscopy images of scrambled-KDR⁺ and SADEiGEN-KDR⁺ cells cultured for 72h in endothelial-to-hematopoietic medium (Liquid blast). Floating clusters of primitive blood progenitors are observed budding from attached hemogenic endothelial cells. **C.** Representative bright-field microscopy images of scrambled-KDR⁺ or SADEiGEN-KDR⁺ cells seeded in HSC expansion medium (Stem Spam) for 96h. **D.** Left and middle panel: Representative flow cytometry plots of lineage negative (LIN-; percentage gated on alive cells) population from scrambled-KDR⁺ (left) and SADEiGEN-KDR+ (middle) cells seeded for 96 hours in StemSpan medium. Right panel: quantification of total number of LIN- cells (per 10⁵ cells analyzed) in scrambled and SADEiGEN conditions after culturing for 96h the KDR⁺ progenitors (derived from either scrambled or SADEiGEN conditions) in StemSpan medium. Each dot represents an independent experiment. Error bar indicates ±SEM. **E.** Representative bright-field images of the different colony-forming units (CFU) of KDR⁺ cells cultured for 192h in methylcellulose. BFU-E: burst-forming-unit-erythroid; CFU-M: colony-forming-units-monocytes; CFU-G: colony-forming-units-granulocytes; CFU-M: colony-forming-units-macrophages; CFU-GM: colony-forming-units-granulocytes-monocytes; CFU-GEMM: Colony-forming-unit-granulocytes-erythroid-monocytes-megakaryocytes. **F**. SADEiGEN induction dynamics during KDR⁺ differentiation. Relative RNA expression levels were calculated using the *Tbp* gene. Fold-change expression was considered taking into account the relative expression level of each gene at 72h EBs (0h Doxycycline treatment). Color code represents the mean expression of each gene from three independent experiments. **G.** Representative flow cytometry plots of the in vitro endothelial-to-hematopoietic-transition (EHT) experiment after 72 hours using a different set of gRNAs to activate SADEiGEN. Upper panel: scrambled condition for analysis of hemogenic endothelium (TIE2⁺KIT⁺; left panel) and primitive hematopoietic progenitors (KIT⁺CD41^{low}CD45⁺; right panel); medium panel: SADEiGEN activation condition (using a different set of gRNAs) with analysis of hemogenic endothelium (TIE2⁺KIT⁺; left panel) and primitive hematopoietic progenitors (KIT⁺CD41^{low}CD45⁺; right panel); bottom panel: quantification of total number of hemogenic endothelial cells (TIE2⁺ KIT⁺; left panel) and primitive blood progenitors (KIT⁺ CD41^{low} CD45⁺; right panel) in scrambled and SADEiGEN conditions after 72h in Li-blast medium. Each dot represents an independent experiment. Number of cells based on total number of cells analyzed (10⁵). Error bars refer to ±SD. **H.** Representative flow cytometry graphs of in vitro HSPC fate induction by culturing KDR⁺ cells in StemSpan medium for 96h. Upper panel: lineage negative (LIN⁻) population gated from alive cells in scrambled (left) or SADEiGEN (alternative set of gRNAs; medium) conditions. Quantification of the total number of LIN⁻ population in scrambled and SADEiGEN (alternative set of gRNAs) conditions (right panel); each dot represents an independent experiment, and error bar refers to ±SD. Medium panel: LIN⁻ KIT⁺ SCA-1⁺ (LSK) population gated from LIN⁻ cells in scrambled (left) or SADEiGEN (alternative set of gRNAs; medium) conditions. Quantification of the total number of LSK population (gated from LIN⁻) in scrambled and SADEiGEN (alternative set of gRNAs) conditions (right panel); each dot represents an independent experiment, and error bar refers to ±SD. Bottom panel: Long-term hematopoietic stem cell (LT-HSC; LSK CD48⁻ CD150⁺) population gated from LSK in scrambled (left) or SADEiGEN (alternative set of gRNAs; medium) conditions. Quantification of total number of LT-HSC (gated from LSK population) in scrambled and SADEiGEN (alternative set of gRNAs) conditions (right panel); each dot represents an independent experiment, and error bar refers to ±SD. Number of cells based on total number of cells analyzed (10⁵). **I.** Quantification of percentage of colony types after seeding scrambled-KDR⁺ or SADEiGEN-KDR⁺ (alternative set of gRNAs) cells in methylcellulose for 192 hours (8 days). A 2-way ANOVA test was performed to calculate statistical significance. Data derived from three independent experiments. Error bar indicates ±SD.

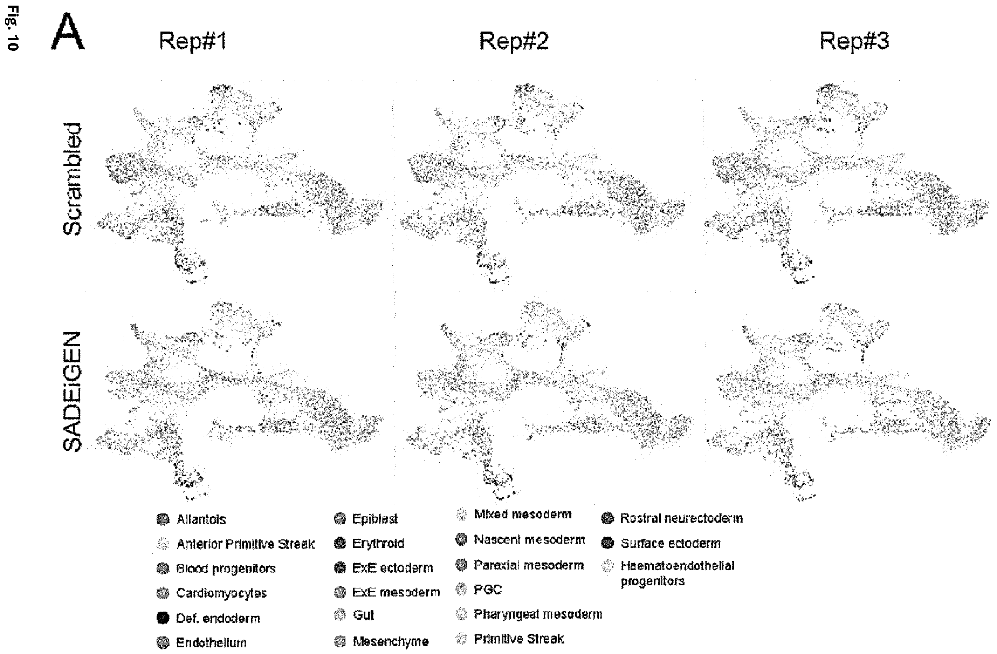

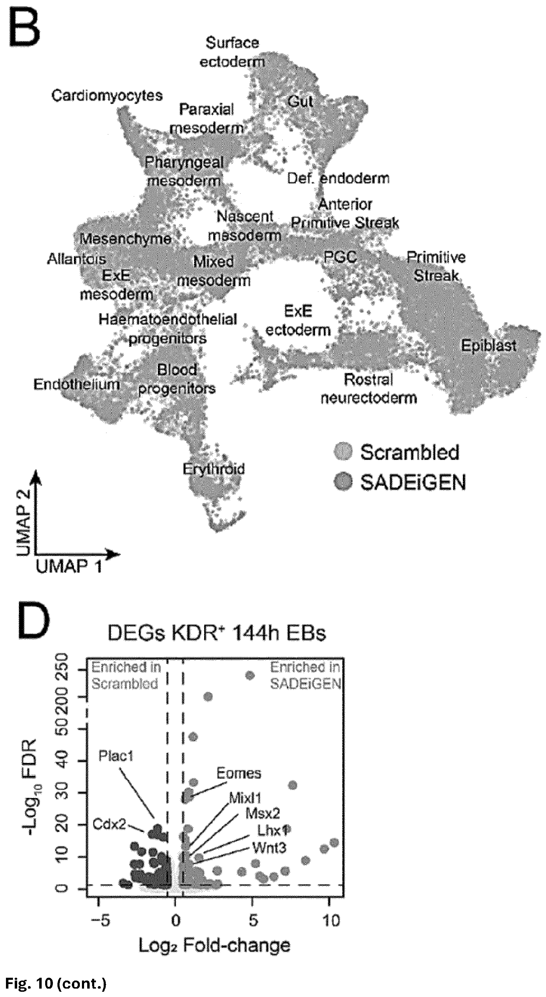

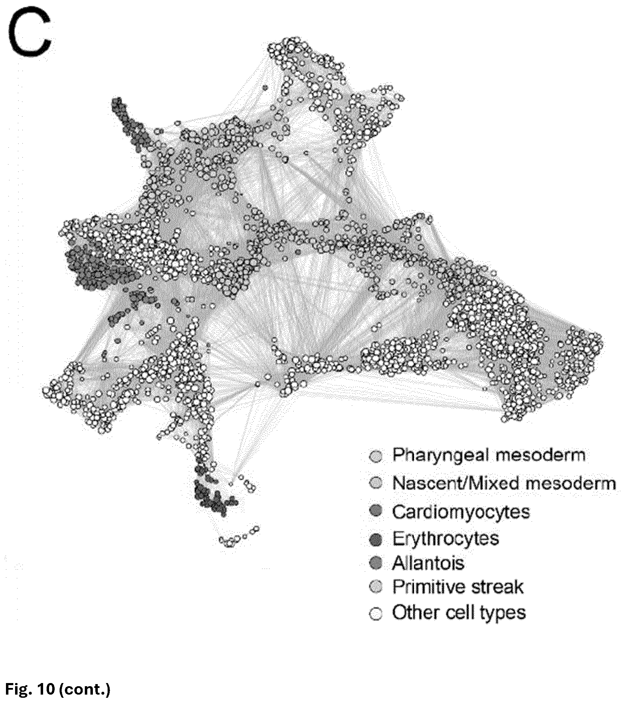

**Figure 10** **Analysis of cell fate heterogeneity within 144h EBs at the single-cell level after activation of SADEiGEN. A.** UMAP with the identified cell types in our sc-RNAseq 144h EBs dataset based on the mouse embryo atlas (E6.5-E8.5) annotations split by samples and replicates. **B.** UMAP from 144h EBs dataset colored by cell origin (scrambled-derived in orange and SADEiGEN-derived in blue colors). Identified cell types are also included. **C.** Abstracted KNN graph of cells neighborhoods obtained with Milo superimposed on UMAP coloured by identified groups of neighborhoods based on their fold change obtained from the differential abundance analysis and the number of shared cells. Only those groups of interest are coloured mainly composed by PGC and Primitive Streak (group 4, 68% cells), Pharyngeal Mesoderm (Group 11, 62% cells), Nascent/Mixed Mesoderm (Group 13, 83% cells), Cardiomyocytes (Group 14, 92% cells), Erythroid (Group 15, 98% cells) and Allantois (Group 16, 51% cells). Graph edge width is proportional to cell overlap size between neighborhoods. Circle size is proportional to neighborhood size. **D.** Volcano plot obtained from pseudo-bulk differential expression analysis between scrambled and SADEiGEN 144h EBs from cells expressing Kdr exclusively. Dashed vertical lines indicate an absolute shrunken Log 2 Fold Change of 0.5 and dashed horizontal line an adjusted p-value (FDR) of 0.05.

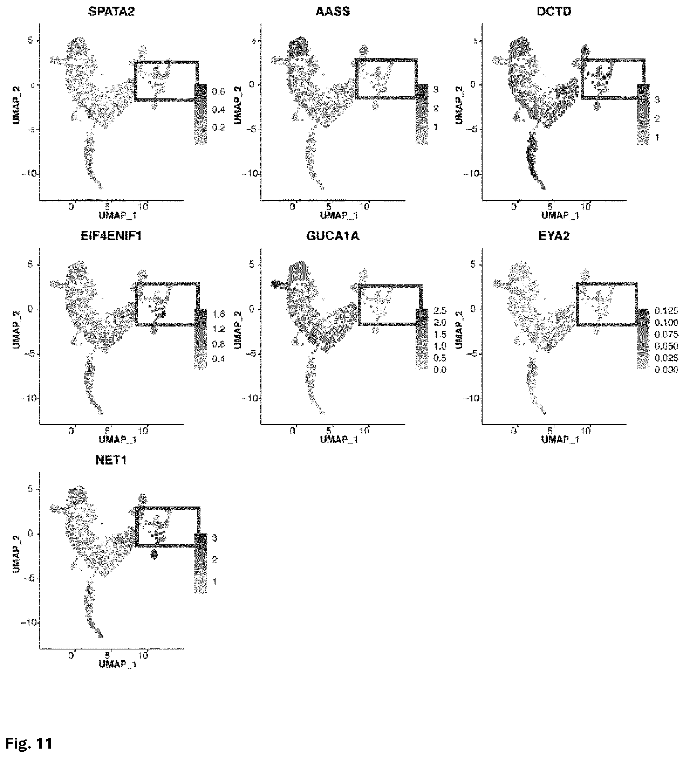

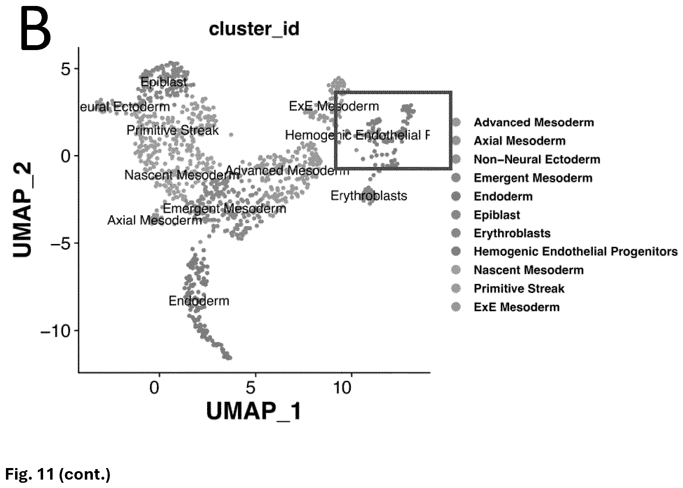

**Figure 11****.** UMAP representation of gene expression of SADEiGEN genes in a human embryo (Carnegee Stage 7) in gestational week 4 plus 5 days (corresponds to between 2 and 3 post-conception weeks) from dataset **(A).** Clusters of identified cell populations are showing in **(B).** Box indicates expression in hemogenic endotelial progenitors.

### DETAILED DESCRIPTION OF THE INVENTION

Here we report a unique experimental strategy for HSC driver discovery, which combines unbiased temporary endogenous gene activation with *in vitro* hematopoietic differentiation and *in vivo* transplantation. This strategy has allowed us to identify a novel combination of 7 genes (*Spata2, Aass, Dctd, Eif4enif1, Guca1a, Eya2* and *Net1*) that imposes HSPC potential to ESCs, here referred as SADEiGEN. Specifically, SADEiGEN induction during mesoderm specification results in KDR⁺ cells with higher ability for *in vivo* engraftment and multilineage reconstitution including the erythroid, myeloid and lymphoid lineages. Previous work indicated that EBs-derived KDR⁺ progenitors resemble primitive hematopoiesis and cannot repopulate the blood in vivo^{29,30}. **Here we demonstrate that, by guiding specific mesoderm cells towards intraembryonic fates, we can generate KDR⁺ progenitors that give rise to definitive HSPCs.** Our results demonstrate that *in vivo* KDR⁺ progenitors are the precursors of, among other tissues, definitive HSCs³¹. Moreover, SADEiGEN induction leads to the formation of a relatively high number of immunophenotypic HSPCs in primary recipients *in vivo,* where approximately 0.04% of total bone marrow cells are LSK CD150⁺ CD48⁻ (Figure 2G). Importantly, SADEiGEN-induced HSPCs, albeit at very low frequency, are also detected 18 weeks after the secondary transplantation (Figures 21, 3A). These results indicate that SADEiGEN-induced blood progenitors maintain their self-renewal potential. Moreover, these cells can reconstitute erythroid, myeloid and lymphoid compartments, although the proportion of cell lineages varies between primary and secondary recipients, with T cells detected only in secondary recipients (Figures 2D-E and 2H-I). A putative explanation maybe the generation of different clones of SADEiGEN-induced HSPCs, each with a different potential to self-renew and generate different blood cell lineages in vivo. In addition, the animal model used in this study makes it difficult to distinguish between clonal engraftment and multi-clonal reconstitution, as the NSG mice show a strong B-cell bias after transplantation ^{32,33}. Analysis of differentiated engrafted blood cells demonstrated that both myeloid (monocytes) and B cells exhibit features of functional maturation, with monocytes capable of engulfing particles and B cells exhibiting different patterns of immunoglobulin expression from primary to secondary recipients (Figures 3D-E and 31). Whether SADEiGEN-derived T-cells undergo rearrangements of their T cell receptor remains unknown.

Previous studies have demonstrated the importance of properly polarizing early mesodermal progenitors and primitive streak to obtain definitive hematopoiesis ^{34,35}. Here, we show that SADEiGEN activation biases embryoid bodies towards more intraembryonic lineages (Figure 5). Specifically, transient induction of SADEiGEN increases the proportion of primitive streak cells and nascent/mixed mesoderm, which ultimately give rise to, among other tissues, definitive blood ²⁸. Regarding the mechanisms by which SADEiGEN promotes HSC generation, it should be noted that it constitutes a very heterogeneous gene cocktail, not composed mainly of transcription factors, but rather by genes with different functions. SPATA2 is involved in several processes, and it acts as an adaptor protein in inflammatory pathways ³⁶, whereas AASS, DCTD, GUCA1A and NET1 are implicated in processes related to nucleotide and amino acid metabolism ³⁷⁻⁴⁰. EIF4ENIF1 is required for P-body formation ⁴¹, which has been previously linked to regulate stem cell function and myeloid leukemias ^{42,43}. EYA2 is a protein phosphatase and transcriptional coactivator ^{44,45}. Our results suggest that programming complex stem cell fates require the tight coordination of different physiological processes within cells, not only to direct action on chromatin conformation by transcription factors, but also by genes that fine-tune cellular processes that are crucial for stem cell maintenance.

On the other hand, the fact that most of the SADEiGEN cocktail is higher expressed in the *in vivo* EHT transition might suggest a direct function into the blood stem cell specification within the AGM region. In fact, mice lacking either *Spata2, Eya2* or *Aass* have been reported to display different hematopoietic-related phenotypes (The Jackson Laboratory MGI:2146885, MGI:109341, MGI:1353573, respectively), suggesting that they are required for a normal hematopoietic development. Moreover, Eya2 is differentially expressed in HSCs ⁴⁶, and its upregulation increases HSPCs via E2A-HLF binding⁴⁷.

The final goal of regenerative medicine is generating HSPCs at high yield for their use in regenerative medicine, not only for transplantation of progenitors but also for using their cell derivatives for other applications such as immunotherapy. In this sense, reproducing this protocol in human pluripotent stem cells is of great importance.

Therefore, a first aspect of the invention refers to an in vitro method for activating a subset of genes in a KDR⁺ cell population, preferably in an enriched or substantially homogeneous KDR⁺ cell population, wherein the activation of said subset of genes promotes hematopoietic stem cell (HSC) generation, said subset comprising at least one, two, three, four, five, six, or preferably all, of the following 7 genes selected from the group consisting of: Spata2 (spermatogenesis-associated 2), Aass (aminoadipate-semialdehyde synthase), Dctd (Gene ID: 1642), Eif4enif1 (eukaryotic translation initiation factor 4E nuclear import factor 1), Guca1a (guanylate cyclase activator 1A), Eya2 (Eyes Absent Homolog 2) and Net1 (Neuroepithelial Cell Transforming 1), and wherein the method comprises inducing temporary endogenous activation of the genes in the KDR⁺ cell population.

As used herein, "activating a subset of genes in a KDR⁺ cell population" refers to any process or intervention that results in upregulation, induction, or enhancement of the transcription, translation, or functional output of one or more target genes within cells that express KDR (kinase insert domain receptor). This activation may be achieved by, for example:
1. Direct Modification of Genetic Regulatory Elements
2. Introducing or modifying promoter or enhancer sequences to increase gene expression levels.
3. Using CRISPR/Cas or other nuclease-based systems (e.g., CRISPR activation constructs) to target and activate transcription at specific loci.
4. Epigenetic Alteration
5. Employing epigenetic modifiers such as histone acetyltransferases, histone demethylases, DNA methyltransferase inhibitors, or DNA demethylases to modulate chromatin accessibility and enhance gene transcription.
6. Delivery of Transcriptional Activators or Regulatory RNAs
7. Introducing transcription factors, co-activators, or guide RNAs designed to recruit activation domains to specific gene loci.
8. Using viral vectors, viral-like particles (VLPs), ribonucleoprotein (RNP) complexes, or non-viral gene delivery systems to carry these activating molecules into KDR⁺ cells.

### Other Mechanisms

Altering signaling pathways (e.g., by adding growth factors or small molecules) that indirectly enhance expression of the target genes in KDR⁺ cells.

Such gene activation is preferably detectable or quantifiable via standard molecular biology or cell-based assays (e.g., qPCR, RNA-Seq, Western blot, reporter assays, or flow cytometry for downstream protein markers). The degree of activation may vary depending on the method employed but is generally sufficient to produce a measurable increase in the expression or activity of the targeted gene(s) within the KDR⁺ cell population.

The following genes can be readily identified by their commonly accepted names, as they are well-known in the art. Indeed, the mere name of each gene is sufficient for a person skilled in the art to locate the corresponding sequence in publicly available databases. For additional clarity, references and database identifiers are also provided herein.

As used herein, SPATA2 (spermatogenesis-associated 2) is a human gene originally implicated in testicular development and spermatogenesis. It also plays a regulatory role in the TNF receptor signaling pathway, where it interacts with deubiquitinating enzymes (e.g., CYLD) to influence NF-κB-mediated inflammatory responses.

SPATA2 gene sequence in humans can be found in NM_006038 (Homo sapiens spermatogenesis associated 2 (SPATA2), transcript variant 1, mRNA). HGNC:14681, NCBI gene: 9825, Ensembl: ENSG00000158480**OMIM**: 607662 UniprotKB: Q9UM82
Chromosome location: Chromosome 20 (*20q13.13).*

A non-limiting short list of ways to increase or enhance SPATA2 expression or function includes:
- Cloning the SPATA2 CDS into a plasmid under a strong promoter (e.g., CMV) and transfect or transduce cells to boost SPATA2 protein levels.
- Using lentiviral or adenoviral systems to achieve stable or high-efficiency SPATA2 expression in various cell types.
- Employing a catalytically inactive Cas9 fused to a transcriptional activator (e.g., VP64) targeting the SPATA2 promoter to increase endogenous gene transcription.

These approaches can be combined or optimized depending on the cell type and experimental goals.

As used herein, AASS (aminoadipate-semialdehyde synthase) is a bifunctional enzyme that catalyses the first two steps of the lysine degradation pathway in humans. It comprises lysine-ketoglutarate reductase (LKR) and saccharopine dehydrogenase (SDH) activities, playing a key role in amino acid catabolism. HGNC: 17366; NCBI Gene: 10157; Ensembl: ENSG00000008311; OMIM^{®}: 605113; UniProtKB/Swiss-Prot: Q9UDR5
Chromosome location: chromosome 7 (7q31.32).

A non-limiting method to enhance AASS expression is to clone its coding sequence into an expression vector (e.g., a plasmid under a strong promoter like CMV). This plasmid can be transfected into target cells, resulting in increased transcription and translation of the AASS gene product and thereby boosting its functional output.

As used herein, DCTD (HGNC: 2710; NCBI Gene: 1635; Ensembl: ENSG00000129187; OMIM^{®}: 607638; UniProtKB/Swiss-Prot: P32321) in humans encodes dCMP deaminase, an enzyme that catalyzes the deamination of deoxycytidylate (dCMP) to deoxyuridylate (dUMP). This step is critical in the pyrimidine nucleotide salvage pathway, influencing DNA synthesis and repair.
Official Symbol: DCTD
Official Full Name: dCMP deaminase
Location: Chromosome 4 (4q35.1)

A straightforward, non-limiting approach to upregulate or enhance DCTD expression in cultured cells is to clone the DCTD coding sequence (CDS) into an expression vector (e.g., a plasmid) under the control of a strong constitutive promoter such as the CMV (cytomegalovirus) promoter. Transfect or transduce the cells of interest with this recombinant plasmid/vector. The robust promoter will drive higher levels of DCTD transcription, increasing mRNA levels and, consequently, protein expression and functional output. This method can be adapted for different cell types and experimental settings by selecting appropriate transfection reagents, viral vectors (e.g., lentivirus, adenovirus), or inducible promoter systems.

As used herein, EIF4ENIF1 (eukaryotic translation initiation factor 4E nuclear import factor 1), sometimes referred to as 4E-T, encodes a protein involved in regulating mRNA metabolism by interacting with the cap-binding protein eIF4E. This interaction can influence the nuclear import and subcellular localization of eIF4E, thereby affecting cap-dependent translation and RNA stability.

The mRNA sequence of this gene can be found in RefSeq NM_019843.4 from the NCBI database.
Chromosome location: chromosome 22 (22q12.2).
HGNC: 16687 , NCBI Gene: 56478, Ensembl: ENSG00000184708, OMIM: 607445, Uniprot/Swiss-Prot: Q9NRA8

A straightforward, non-limiting approach to upregulate or enhance EIF4ENIF1 expression in cultured cells is to clone the EIF4ENIF1 coding sequence into a mammalian expression vector (e.g., one with a CMV promoter) and transfect this construct into target cells. The strong promoter drives higher transcription, thereby increasing mRNA and ultimately boosting the level of the EIF4ENIF1 protein.

As used herein, GUCA1A (guanylate cyclase activator 1A) encodes guanylyl cyclase-activating protein 1 (GCAP1), a calcium-binding protein primarily expressed in retinal photoreceptors. GCAP1 modulates retinal guanylyl cyclase activity in response to calcium levels, playing an important role in phototransduction and normal vision.

The NCBI Reference Sequence is NM_001384910.1 (Homo sapiens guanylate cyclase activator 1A (GUCA1A), mRNA) for the human GUCA1A gene.
HGNC: 4678 , NCBI Gene: 2978, Ensembl: **ENSG00000048545,** OMIM: 600364, Uniprot/Swiss-Prot: P43080.

A straightforward, non-limiting example to upregulate or enhance GUCA1A expression in cultured cells is to clone the GUCA1A Coding Sequence into a Mammalian Expression Vector using a strong, constitutive promoter (e.g., CMV) or an inducible promoter (e.g., Tet-On system) upstream of GUCA1A's coding region. Transfect or Transduce the Construct into Target Cells, Deliver the plasmid via lipid-based transfection or use a viral vector (lentivirus, AAV, etc.) to achieve stable integration or expression in the cell line of interest. By placing GUCA1A downstream of a robust promoter in a suitable vector and introducing it into cells, one can reliably enhance the gene's transcription, translation, and overall functional output.

As used herein, EYA2 (Eyes Absent Homolog 2) is a human gene that encodes a protein functioning both as a transcriptional coactivator and a protein phosphatase. It is part of the EYA family (EYA1-EYA4) originally characterized in Drosophila. EYA2 is implicated in several developmental processes (including organogenesis) and has been studied for its potential roles in cell differentiation and tumorigenesis.
Location: Chromosome 20q13.12
HGNC:3520, NCBI Gene ID: 2139, Ensembl: ENSG00000064655, OMIM: 601654, Uniprot/Swiss-Prot: 000167
*REFSEQ mRNAs :* NM_005244.5, NM_172110.4, NM_172111.1, NM_172112.1, and NM_172113.1

A straightforward non limiting way to increase the expression (transcription, translation, or functional output) of EYA2 in a cell is to clone the EYA2 coding sequence into a mammalian expression vector under the control of a strong promoter (e.g., CMV, EF1α) and then transfect or transduce the target cells. This approach ensures higher transcriptional activity of EYA2, leading to elevated protein production and enhanced functional output.

As used herein, the NET1 (Neuroepithelial Cell Transforming 1) gene encodes a Rho-specific guanine nucleotide exchange factor (RhoGEF). RhoGEFs activate Rho GTPases by catalyzing the exchange of GDP for GTP, thereby influencing key cellular processes such as cytoskeletal organization, cell shape, and motility. Dysregulation or altered expression of NET1 has been implicated in various pathological conditions, including certain cancers.

In humans, NET1 is located on chromosome 10. Like many human genes, NET1 has multiple transcript variants.
Location: Chromosome 10p15.1
RefSeq (mRNA): NM_001047160.3 and NM_005863.5
HGNC: 14592, NCBI Gene: 10276, Ensembl: ENSG00000173848, OMIM: 606450, Uniprot/Swiss-Prot: Q7Z628

A straightforward, non-limiting example to increase NET1 expression in a cell line is loning into an Expression Vector. Insert the full-length NET1 coding sequence into a mammalian expression plasmid (e.g., under a strong promoter such as CMV or EF1α). Transfect the plasmid into the target cells using a standard transfection reagent (e.g., lipofection) to drive high-level NET1 transcription. Such an approach can robustly elevate NET1 mRNA and protein, thereby enhancing its functional output in research or therapeutic settings.

The previous disclosure enables those skilled in the art to readily identify and isolate the above-described 7 human genes based on the provided information. Armed with this information, a person of ordinary skill in the art can readily locate and obtain the human gene sequence for each of these genes. Furthermore, the murine sequences for each of the genes are provided at the end of this description as additional information.

It is noted that the KDR⁺ cell population or the enriched or substantially homogeneous KDR⁺ cell population is preferably a mesodermal KDR⁺ cell population or a precursor of a mesodermal KDR⁺ cell population. Accordingly, a KDR⁺ cell population meets the criteria for being considered "mesodermal" if it displays hallmarks of mesodermal commitment (molecular, functional, or both), whereas it is deemed a "precursor of a mesodermal KDR⁺ cell population" if it is poised for mesodermal differentiation but may not have fully manifested the complete mesodermal gene-expression profile. For the KDR⁺ cell population (or the enriched or substantially homogeneous KDR⁺ cell population) to be deemed "mesodermal," at least about 15%, 20%, 30%, 40%, 50%, 60%, 70%, 80%, or 90% of the cells, as determined by flow cytometry, immunostaining, gene expression profiling, or other suitable methods, should demonstrate molecular or functional hallmarks indicative of mesodermal lineage commitment. Such hallmarks may include the expression of early mesodermal transcription factors (e.g., Brachyury (T), Mesp1, Eomes) and/or the capacity to generate downstream mesoderm-derived cell types (e.g., endothelial cells, cardiomyocytes, blood cells).

Conversely, the population is deemed a "precursor of a mesodermal KDR⁺ cell population" if a similar proportion of the cells express KDR and are poised for mesodermal differentiation (e.g., partially expressing or upregulating relevant markers), but may not yet show the full mesodermal transcriptional or functional profile. In other words, these precursor cells have the potential to meet the full mesodermal criteria upon receiving appropriate differentiation cues in vitro or in vivo.

As used herein, the term "cell population" refers to a group of cells that share one or more common characteristics (e.g., morphological traits, genetic markers, protein expression profiles, metabolic features) and are cultured or isolated under conditions that maintain or promote these characteristics. Unless otherwise specified, a cell population may be:
- Heterogeneous, meaning it can contain multiple subtypes of cells but is distinguished by at least one shared, defining feature (such as expression of a particular marker or suite of markers); or
- Enriched or substantially homogeneous, wherein the cells bearing the defining feature comprise at least a specified percentage (e.g., at least about 15%, 20%, 30%, 40%, 50%, 60%, 70%, 80%, or 90% of the total cell population, as determined by a suitable quantitative method (e.g., flow cytometry, immunostaining, or gene expression analysis).

In certain embodiments, the cell population can be expanded or differentiated in vitro from a progenitor or stem cell source, or it can be harvested from a subject (e.g., from peripheral blood, tissue biopsy, or another biological sample) and further processed (such as by magnetic or flow cytometric sorting) to yield cells with the desired phenotype. The population may exhibit specific Marker Expression: For example, in the context of the present invention, cells that express a particular surface marker KDR.

As used herein, the term "KDR⁺ cell population" refers to a group of cells that express the kinase insert domain receptor (KDR) (also known as CD309, FLK1 or VEGFR2) on their surface. In particular, a "mesodermal KDR⁺ cell population" is one that is derived from, committed to, or capable of differentiating into mesodermal lineages and exhibits KDR expression as a distinguishing marker. Such cells are typically identified via immunostaining (e.g., flow cytometry using anti-KDR/VEGFR2 antibodies) or gene expression profiling (e.g., RT-qPCR for KDR mRNA).

### - Key Features of Mesodermal KDR⁺ Cells

Lineage Commitment: Mesodermal KDR⁺ cells are often precursors to multiple mesoderm-derived cell types (e.g., endothelial cells, cardiomyocytes, and blood cells). They can be derived from pluripotent stem cells (PSCs)-including embryonic stem cells (ESCs) or induced pluripotent stem cells (iPSCs)-when differentiated under conditions favoring mesodermal specification (e.g., through modulation of Wnt, Activin/Nodal, or BMP signaling pathways).

Molecular Profile: KDR (FLK1/VEGFR2) expression is used as a positive marker. Depending on the differentiation stage, mesodermal KDR⁺ cells may co-express other mesoderm markers, such as Brachyury (T), Eomesodermin (EOMES), or Mesp1, and may be negative for markers associated with non-mesodermal lineages (e.g., Sox1 for ectoderm).

Functional Capacity: Mesodermal KDR⁺ cells have the potential to give rise to downstream lineages including vascular endothelium, cardiomyocytes, and hematopoietic cells. In vitro, these cells may form endothelial networks or beating cardiomyocytes under appropriate differentiation conditions.

Isolation and Characterization: Typically isolated via FACS (fluorescence-activated cell sorting) or MACS (magnetic-activated cell sorting) using an anti-KDR antibody. Characterized using functional assays (e.g., tube formation assays for endothelial cells, beating assay for cardiomyocytes, or colony-forming assays for hematopoietic cells).

Mesodermal KDR⁺ Precursor Cell Population. A "precursor of a mesodermal KDR⁺ cell population" refers to a cell population that does not yet robustly express KDR but is competent to express KDR under proper differentiation cues (e.g., appropriate culture media, growth factors, or signaling modulators). Exhibits gene expression signatures (e.g., T/Brachyury, Mesp1, Eomes) characteristic of an early mesoderm or mesendoderm stage. It is predestined to generate mesodermal KDR⁺ cells, either in vitro or in vivo, upon continued culture or physiological embryonic signals.

As used herein, the term "enriched or substantially homogeneous KDR⁺ cell population" refers to a group of cells in which at least about 15%, 20%, 30%, 40%, 50%, 60%, 70%, 80%, or 90% of the cells express KDR (also known as FLK1/VEGFR2) at a detectable level. Such detectability may be established using quantitative methodologies, including flow cytometry, immunostaining, gene expression analysis (e.g., qPCR), or any other suitable technique recognized by those skilled in the art. In certain embodiments, the enriched or substantially homogeneous population is obtained by positive selection: For example, using fluorescence-activated cell sorting (FACS) or magnetic-activated cell sorting (MACS) based on KDR expression; or in vitro expansion or differentiation: Culturing cells under conditions that promote the outgrowth of KDR⁺ cells (e.g., specific growth factors, serum-free media formulations, or other differentiation protocols); or genetic Modification: using transgenic or gene-editing approaches to enrich for cells that express KDR or to eliminate populations lacking KDR.

The term "enriched" indicates that KDR⁺ cells constitute a higher proportion compared to the starting population, whereas "substantially homogeneous" suggests that the majority of cells within the population (e.g., at least about 60%, 70%, 80% or 90%) consistently express KDR at a level that is functionally relevant.

As used herein, the phrase "consistently express KDR at a level that is functionally relevant" refers to the stable and reproducible expression of KDR (kinase insert domain receptor) by cells at a threshold quantity sufficient to:
- Enable or enhance KDR-mediated signaling pathways (e.g., in response to VEGF or other ligands),
- Correlate with functional attributes typical of KDR⁺ cells, such as proliferation, differentiation potential, or lineage commitment, and/or
- Be measured by standard assays-for instance, flow cytometry, immunostaining, or quantitative molecular methods-in a manner that distinguishes these cells from those with low or undetectable KDR expression.

By "consistently," it is meant that a substantial majority of the cells expressing KDR do so at or above the functionally relevant threshold over time or under the specified culture conditions, thus ensuring that the population can reliably exhibit the biological behaviours associated with KDR expression.

As generally used herein, the term "KDR⁺ cell population(s)" includes enriched or substantially homogeneous KDR⁺ cell populations, which are preferably mesodermal KDR⁺ cell populations or precursors of mesodermal KDR⁺ cell populations.

In an embodiment of the first aspect of the invention, the activation of said subset of genes is achieved by transforming, transfecting, or transducing the KDR⁺ cell population with one or more gene delivery systems, such as vectors (preferably viral vectors), viral-like particles (VLPs), or ribonucleoprotein (RNP) complexes, comprising sequences or components that indirectly activate the subset of genes. In particular, said sequences or components include, but are not limited to, guide RNAs targeting promoter regions, epigenetic modifiers (e.g., histone acetyltransferases or DNA demethylases), or regulatory elements that enhance the endogenous expression of the subset of genes.

In an embodiment of the first aspect of the invention, the activation of said subset of genes is achieved by transforming, transfecting, or transducing the KDR⁺ cell population with one or more vectors, preferably viral vectors, comprising:
a. sequences encoding transcriptional activators specific to the subset of genes;
b. other sequences that indirectly activate the subset of genes, wherein said other sequences include guide RNAs targeting promoter regions, epigenetic modifiers such as histone acetyltransferases or DNA demethylases, or regulatory elements that enhance endogenous expression of the subset of genes;
c. sequences encoding one or more of the genes within the subset;

In addition, inducible CRISPR activator systems (such as iVPR) to get the activation of the mentioned genes could be used.

In an embodiment of the first aspect of the invention, the viral vectors are lentiviral vectors modified to allow transient expression of the sequences, such as through the use of self-inactivating (SIN) vectors or inducible promoters that can be deactivated to provide temporary activation of the subset of genes in the KDR⁺ cell population.

In another embodiment of the first aspect of the invention, the viral vectors are adenoviral vectors, allowing transient expression of the sequences without genomic integration, thereby providing temporary activation of the subset of genes in the KDR⁺ cell population.

In another embodiment of the first aspect of the invention, the activation of said subset of genes is achieved using CRISPR activation (CRISPRa) technology, wherein guide RNAs target promoter or enhancer regions of the genes within the subset, and endogenous expression is upregulated through the recruitment of transcriptional activators or chromatin-modifying complexes. Preferably, the CRISPR activation (CRISPRa) system utilizes a catalytically inactive Cas9 (dCas9) fused to a transcriptional activator, such as VP64, p300, or a synergistic activation mediator (SAM) complex, to enhance the expression of the subset of genes in the KDR⁺ cell population. Preferably, the guide RNAs are optimized to target multiple regulatory elements within the promoter and enhancer regions of the genes in the subset, providing synergistic upregulation of endogenous gene expression.

In another embodiment of the first aspect of the invention, the activation of said subset of genes is achieved by delivering synthetic mRNAs encoding said genes to the KDR⁺ cell population.

In another embodiment of the first aspect of the invention, optionally in combination with any previous or subsequent embodiment of the invention, the method further comprises culturing the KDR⁺ cells under conditions conducive to HSC maturation following gene activation to obtain a cell population comprising HSCs.

A second aspect of the invention refers to a cell population comprising HSCs obtained or obtainable by the method of the first aspect of the invention, preferably in accordance with any of its preferred embodiments or any combinations thereof, wherein the HSCs exhibit the capacity for *in vivo* engraftment and multilineage reconstitution, including the erythroid, myeloid, and lymphoid lineages.

For the purposes of this invention, the term "HSCs" (hematopoietic stem cells) refers to multipotent precursor cells that possess both self-renewal capacity and the potential to differentiate into the various lineages of blood cells (including red blood cells, white blood cells, and platelets). Functionally, HSCs can reconstitute the hematopoietic system of a recipient host (e.g., in vivo transplantation assays), thus confirming their ability to engraft and generate the full spectrum of mature blood cell lineages.

In another embodiment of the second aspect of the invention, the hematopoietic stem cells (HSCs) constitute at least 1% of the total cell population obtained or obtainable by the method of the first aspect of the invention, preferably wherein the hematopoietic stem cells (HSCs) constitute between 1% and 10% of the total cell population, preferably wherein the hematopoietic stem cells (HSCs) constitute between 10% and 50% of the total cell population, preferably wherein the hematopoietic stem cells (HSCs) constitute greater than 50% of the total cell population, resulting in an enriched HSC composition.

Preferably, wherein at least about 10%, 25%, 50%, 75%, 80%, 90% or more of this cell population (as determined by flow cytometry or another suitable quantitative assay) is phenotypically characterized by the expression of one or more of the following hematopoietic stem cell (HSC) markers: Lin- Sca1+ ckit+ (LSK). This phenotypic signature correlates with the cells' ability to engraft in vivo and contribute to the formation of multiple blood cell lineages.

As used herein, the phrase "phenotypically characterized by the expression of one or more hematopoietic stem cell (HSC) markers: Lin⁻ Sca1⁺ cKit⁺ (LSK)" refers to a cell population that:
Lacks expression of a panel of lineage markers (Lin⁻):
   "Lineage markers" typically include cell-surface proteins associated with differentiated blood cell types (e.g., CD3, CD19, CD11b, Gr-1, Ter119, or other lineage-specific antigens depending on species and experimental setup).
   A cell is considered Lin⁻ if it does not exhibit detectable levels of these markers, as determined by standard methods such as flow cytometry or immunostaining.
Expresses Sca1 (Sca1⁺):
   Sca1 (Stem cell antigen-1) is a surface protein commonly used to enrich for primitive hematopoietic stem or progenitor cells in certain species (e.g., mouse).
   A cell is considered Sca1⁺ if it displays Sca1 at or above a threshold level, as measured by suitable detection techniques (e.g., flow cytometry).
Expresses cKit (cKit⁺):
   cKit (also known as CD117) is a receptor tyrosine kinase typically associated with hematopoietic stem and progenitor cells.
   A cell is considered cKit⁺ if it expresses cKit at a level distinguishable from background or isotype controls (again, often determined via flow cytometry).

Taken together, Lin⁻ Sca1⁺ cKit⁺ (LSK) designates a cell population enriched for hematopoietic stem cells and/or multipotent progenitors, known to those skilled in the art as having the capacity for self-renewal and multi-lineage reconstitution (e.g., erythroid, myeloid, and lymphoid) when transplanted in vivo or cultured under appropriate conditions.

A third aspect of the invention refers to a vector configured to induce hematopoietic stem cell (HSC) generation by transforming or transducing KDR⁺ cell populations in *vivo* or *in vitro,* wherein said vector is capable of activating at least a subset of genes selected from the group consisting of Spata2, Aass, Dctd, Eif4enif1, Guca1a, Eya2 and Net1, and wherein the activation promotes the differentiation of KDR⁺ cells into hematopoietic stem cells with the ability for multilineage reconstitution.

In a preferred embodiment of the third aspect of the invention, the vector is a lentiviral vector modified as a self-inactivating (SIN) vector, ensuring transient gene activation in KDR⁺ cells.

In another preferred embodiment of the third aspect of the invention, the vector is an adenoviral vector configured for transient expression of the subset of genes without genomic integration.

In another preferred embodiment of the third aspect of the invention, the vector utilizes a CRISPR activation (CRISPRa) system, comprising a catalytically inactive Cas9 (dCas9) fused to a transcriptional activator and guide RNAs targeting the promoter regions of the subset of genes for temporary endogenous activation.

In another preferred embodiment of the third aspect of the invention, optionally in accordance with any previous embodiment or combination of embodiments, the vector further comprises sequences encoding epigenetic modifiers, including histone acetyltransferases or DNA demethylases, to transiently enhance endogenous gene activation in KDR⁺ cells.

In another preferred embodiment of the third aspect of the invention, optionally in accordance with any previous embodiment or combination of embodiments, the vector is designed to activate the subset of genes specifically in mesodermal KDR⁺ cells by incorporating tissue-specific regulatory elements.

A fourth aspect of the invention relates to a cell population comprising HSCs of the second aspect of the invention (or any of its preferred embodiments) or the vector of the third aspect of the invention (or any of its preferred embodiments), for use in a method of treatment aimed at reconstituting erythroid, myeloid, and/or lymphoid compartments in a subject suffering from hematopoietic failure.

As used herein, the term "subject" generally refers to a mammalian individual, preferably a human patient, who is diagnosed with or susceptible to hematopoietic dysfunction.

In some embodiments, the composition or vector is used to treat or ameliorate bone marrow aplasia, a condition wherein the bone marrow fails to produce adequate functional blood cells (including red blood cells, white blood cells, and/or platelets).

In other embodiments, the composition or vector is administered to enhance hematopoietic recovery in subjects undergoing myeloablative therapy, such as high-dose chemotherapy or radiation treatment, which ablates or severely compromises the bone marrow. More preferably, the composition or vector is indicated for the treatment of cytopenic disorders, including but not limited to:
1. Anemia - a deficiency in red blood cells or hemoglobin;
2. Neutropenia - an abnormally low count of neutrophils (a type of white blood cell);
3. Thrombocytopenia - a reduced number of platelets;
4. Pancytopenia - a simultaneous deficiency of red blood cells, white blood cells, and platelets.

By generating lineage-committed progenitors, the cell composition or vector can restore cellular deficits in one or more of these affected compartments, thereby addressing the underlying cytopenia and improving hematopoietic function.

### Routes of Administration

Administration of the cell composition or vector to the subject can be performed by various medically acceptable routes. Examples include:
- Intravenous (IV) Infusion: Infusing the composition directly into the bloodstream, facilitating systemic distribution and engraftment in the bone marrow.
- Intraperitoneal (IP) Injection: Introducing cells or vectors into the peritoneal cavity, which may be advantageous in specific research models or clinical scenarios.
- Intramuscular (IM) or Subcutaneous (SC) Injection: In some cases, may be used for delivering supporting factors or for localized cell engraftment strategies.
- Direct Bone Marrow Injection: In specialized procedures, delivering the composition directly to the marrow compartment.
- Ex Vivo Gene Therapy Approaches: Harvesting the patient's own cells, modifying them with the vector in vitro (e.g., transduction, transfection), and then reintroducing the modified cells via IV infusion.

Such methods of administration can be performed alone or in combination with adjuvant therapies, such as cytokine support, growth factors, or other agents that enhance engraftment, proliferation, or differentiation of the transplanted HSCs. The precise route, timing, and dosage of administration may vary depending on the clinical condition, patient's health status, and physician's assessment.

The following examples are provided merely for illustrative purposes and do not limit the present invention.

### EXAMPLES

### RESULTS

Genome-wide CRISPRa screening in differentiating mESCs identifies a combination of seven genes with potential to induce long-term hematopoietic engraftment.

To uncover new factors that can induce HSC activity from mESCs, we performed a genome-wide CRISPR activation (CRISPRa) screening combining mesodermal/hemogenic differentiation with in vivo hematopoietic repopulating assays in NSG mice (Figure 1A). We engineered a doxycycline-inducible dCas9-VPR (iVPR CRISPRa) system ¹⁸ into mESCs (E14 cell line) (Figure 6A), and tested the induction potential of gene expression, excluding any leakiness. We then transduced the iVPR-mESCs with a genome-wide CRISPRa gRNA library ²⁰ at a high multiplicity of infection (MOI). We repeated the process four several times (with a MOI=2.5 in each round), in order to obtain a high representation of the gRNAs per cell (see material and methods section). Single-clone-gRNA sequencing showed that about 43 % of mESCs contained between 10-20 different gRNAs, 28% more than 20 gRNAs and 28% contained less than 10 gRNAs (Figure 6C). Next, we differentiated the transduced iVPR-mESCs towards KDR⁺ mesodermal progenitors in embryoid bodies (EBs), and at 72h the gRNA library was activated with doxycycline. After 144h of differentiation, KDR⁺ progenitors were purified, and 6.5x10⁵ KDR⁺ cells were transplanted intravenously (together with 2x10⁴ total bone marrow cells as a support) into sublethally irradiated NSG mice. We followed the outcome of transplanted mESCs-KDR-derived cells in vivo in mice using the blue fluorescent protein (BFP) reporter included in the gRNA library vector (Figures 1A). A high representation of the gRNA library was maintained during the in vitro differentiation from mESCs to KDR⁺ progenitors (based on the BFP marker), with a 98.66% gRNAs detected in KDR⁺ progenitors at the time of transplantation (Figures 6D-F).

Six weeks after transplantation of KDR⁺ progenitors, mice were sacrificed and analyzed for the presence of BFP⁺CD45⁺ mESC-KDR⁺-derived hematopoietic cells. We detected a small fraction of these cells (0.024-0.67%) in the bone marrow (BM) of 87.5% of mice (17/20 primary transplanted mice) from 3 independent experiments. Upon secondary transplantation, total BM cells from primary engrafted mice were able to reconstitute 80% of the secondary mice (13/15) and persisted after 16 weeks of transplantation (Figures 1B-C and supplementary table 1). These results indicated that mESC-derived KDR⁺ progenitors after 72 hours of gRNA library induction acquired long-term hematopoietic engraftment potential. To identify the candidate genes determined the gRNAs that were specifically enriched in the mESC-derived (BFP+) hematopoietic cells that were persisting in primary and secondary transplantations. We isolated CD45⁺ BFP⁺ cells from 1 primary (after 6 weeks) and 4 secondary (3 after 12 weeks and 1 at 16 weeks) transplanted mice (mice at each time point from independent experiments) (Figure 1C), and we determined the gRNAs abundance in those cells by DNA sequencing. Although many gRNAs were detected at low levels in the BM from the different samples, we identified 7 gRNAs, each one targeting a different gene, that were highly represented in all the samples (Figures 1D-E). Interestingly, we detected a gradual decrease in the number of detected gRNAs from KDR⁺cells (>10⁵ gRNAs) to primary (960 gRNAs at 6 weeks) or secondary (86 gRNAs at 12 weeks and 42 gRNAs at 16 weeks) transplantations (Figure 6F). These results suggest a strong in vivo selection of the progenitors carrying these 7 gRNAs, which were targeting the gene promoter of *Spata2, Aass, Dctd, Eif4enif1, Guca1a, Eya2 and Net1.* We will refer to this combination of genes as SADEiGEN. To investigate the biological significance of the SADEiGEN gene combination in specifying HSCs in vivo, we took advantage of scRNA-seq data from highly purified hemogenic and hematopoietic progenitors from Aorta-Gonad-Mesonephros region (AGM) from E10.5-E11.5 mouse embryos ²¹ (Figure 6G). We specifically analyzed the expression pattern of SADEiGEN in the precursors of HSC fate (hemogenic endothelium or HE) and in the early HSCs. Most of the 7 SADEiGEN genes (except *Spata2*) were more highly expressed in HSCs compared to HE (Figures 1F and 6H). Thus, our experimental strategy for in vivo genome-wide CRISPRa screen from mESCs identified a combination of seven novel genes that are expressed in hemogenic endothelium and HSCs specification in vivo, and they are selectively enriched in long-term repopulating hematopoietic cells derived from mESCs-derived KDR⁺ progenitors.

SADEiGEN activation induces the generation of HSPCs with potential to serial multilineage engraftment.

To validate the hematopoietic potential conferred by SADEiGEN, we generated clones of iVPR-mESCs exclusively containing the corresponding 7 gRNAs (SADEiGEN iVPR-mESCs), and clones containing non-targeting scrambled-gRNA as controls. Cells were processed as in the CRISPRa screening assay, and KDR⁺ progenitors were purified and injected in the tibia of sublethally irradiated NSG mice (6.5x10⁵ KDR⁺ cells injected with 2x10⁴ NSG BM cells as a support). Mice were bled every month to assess the percentage of mESCs-derived chimerism, as determined by the percentage of BFP⁺CD45⁺ cells in peripheral blood (Figure 2A). We consistently detected chimerism in the blood of mice up to 12 weeks of primary transplantation (Figures 2B and 7A). The percentage of SADEiGEN-derived chimerism in primary recipients was at least 200 times higher compared to scrambled-gRNA samples in primary recipients (2.5±2.8% BFP⁺CD45⁺ at 4 weeks, 4.8±7.2% BFP⁺CD45⁺ at 8 weeks and 5.3±3.80% BFP⁺CD45⁺ at 12 weeks compared to 0.01% CD45⁺ BFP⁺ cells at 4 weeks and 0.00001% at 12 weeks from scrambled-gRNA) (Figures 2B and 7A). After 8 weeks, a group of primary transplanted mice were euthanized and 10⁵ CD45⁺ BFP⁺ BM cells (without support cells) were transplanted into sublethally irradiated secondary recipients. BM cells from the SADEiGEN-transduced group were able to maintain around 1% engraftment in peripheral blood during 16 weeks in secondary recipients (Figures 2C and 7B) whereas no engraftment was detected in mice transplanted with control (from scrambled-gRNA transplanted mice) cells. Analysis of the different blood lineages in peripheral blood and bone marrow at the end point (in the secondary recipients) demonstrated that CD45⁺ BFP⁻ cells from the NSG recipients were mainly restricted to the myeloid and erythroid lineages, in agreement with defective T cell differentiation in the NSG background, while SADEiGEN-induced blood progenitors (CD45⁺ BFP⁺) gave rise to multilineage engraftment, including erythroid, myeloid, B and T lymphoid cells (Figures 2D-E and 7C-F). However, we did not detect CD45⁺ BFP⁺ T cell lymphopoiesis in the BM of primary recipients (Figures 2D-E). We further characterized the blood progenitors that were contributing to in vivo multilineage engraftment of CD45⁺ BFP⁺ cells by analyzing 8 weeks primary transplanted bone marrow in comparison with the recipient cells (BFP⁻). Our analysis indicated that most of the LSK CD150⁺CD48⁻ HSPCs were BFP⁺ indicating that SADEiGEN-transduced mESCs-derived cells mainly replaced the HSPC compartment of the recipients (Figures 2F-G). Then, we isolated CD45⁺BFP⁻ (recipient-derived) and CD45⁺BFP⁺ (mESCs-derived) cells from 3 independent 18-week secondary transplanted bone marrow, and performed single-cell RNA sequencing (sc-RNAseq) analysis. Cells were represented in a Uniform Manifold Approximation and Projection (UMAP) and different clusters were identified. We found that CD45⁺ BFP⁺ hematopoietic cells mainly overlapped with normal recipient hematopoietic cells (CD45⁺ BFP⁻), indicating the molecular similarity of cells from both origins (Figures 2H and 8A). As expected, lymphoid clusters (T and B cells) were composed exclusively of CD45⁺ BFP⁺ cells, due to the lack of lymphopoiesis in NSG mice ²² (Figures 2I and 8A). We then analyzed the gene signatures of the different mESCs-derived hematopoietic cell types at the single-cell level.

Characterization of the small cluster of CD45⁺ BFP⁺ HSPCs detected in the secondary recipients confirmed the expression of previously described HSCs markers ^{23,24} including *Hlf,* Mecom, *Procr, Hoxa9* and *Mycn* (Figures 3A and 8B), confirming the presence of HSC-like cells even at 18 weeks after secondary transplantation. Since HSPC enrichment was not performed for these experiments, only few cells were detected in the HSPC cluster derived from SADEiGEN-induced cells, however they were overlapping with the HSPC recipient cells in the UMAP indicating their molecular correspondence. Similarly, CD45⁺ BFP⁺ cells in the erythrocytes cluster expressed definitive and mature typical erythroid genes, such as *Klf1* and *Sox6,* as well as adult hemoglobin genes such as *Hbb-bs, Hba-a1, Hbq1b, Hbb-bt* and *Hba-a2* (Figures 3B and 8C), indicative of successful mESCs-derived erythropoiesis. We also detected the presence of a cell cluster corresponding to Innate Lymphoid Cells (ILCs), which were expressing markers from the three different ILC subtypes (ILC1, ILC2, ILC3; Figure 8D), and a cell cluster expressing a T-lymphocytes signature (Figure 3C). One of the cell types that were mainly represented by SADEiGEN-derived blood in vivo, apart from the ILCs and T cells, corresponded to B cells (Figure 21). By flow cytometry, we analyzed the maturation markers of these B cells isolated from the bone marrow and the spleen. We observed that B cell progenitors detected in the bone marrow started to acquire IgM on the membrane that increased in the spleen (2.4±1.3% B-220 IgM in bone marrow vs 32.5±19.7% in spleen; Figure 3D). Indeed, B cells acquired a higher maturation degree in secondary recipients, since they expressed other maturation makers such as Cd80 and Cd86, as well as *Ighm, Ighd* and *Ighg3* (Figure 3E). SADEiGEN-induced mESCs also contributed to the myeloid lineage as indicated by the presence of neutrophil and basophil cell clusters at 18 weeks of secondary transplantation (Figures 3F-G). Lastly, we detected a cluster of CD45⁺ BFP⁺ macrophage-like cells that were present in the secondary transplanted bone marrow cells (Figure 3H). To test whether these macrophages were functional, we isolated them by cell sorting (CD45⁺ MAC-1⁺) and addressed their ability to engulf fluorescent beads. Macrophages generated by SADEiGEN-induced mESCs cells displayed a similar phagocytosis potential compared to recipient bone marrow macrophages (Figure 31). Overall, our results demonstrated that activation of the SADEiGEN signature during mesodermal specification generates KDR⁺ progenitors that can give rise to HSPCs with multilineage engraftment capacity in vivo. Importantly, blood cells derived from SADEiGEN-induced mESCs display features of normal maturity with expression patterns specific to each mature blood cell type. Moreover, both B cells and macrophages derived from these in vitro-generated progenitors produce immunoglobulins and show engulfment capacity, respectively, confirming the functional features in vivo.

In vitro generation of HSPCs is enriched after induction of SADEiGEN expression in differentiating mESCs.

We then investigated the potential of SADEiGEN-induced mESCs to generate HSPCs in vitro (Figure 4A). We first characterized the expression dynamics of SADEiGEN upon in vitro activation from 72h to 144h (transplantation time), and we observed that most of the genes had a peak of induction 24h after doxycycline treatment (96h EBs), with a rapid decline of expression. However, *Eya2* maintained a high level of activation in EBs up to 144h EBs (Figure 4B). SADEiGEN activation resulted in only a small increase in the generation of KDR⁺ mesodermal progenitors at 144h compared to the non-targeting scrambled gRNA samples (Figure 4C). Next, test the hematopoietic potential of SADEiGEN-induced KDR⁺ cells (compared with scrambled-derived cells) in Liquid Blast medium cultures, which mimics EHT in vitro ^{25,26} (Figure 4D upper panel, and material and methods section). Under these culture conditions, clusters of hemogenic endothelial-like cells evolved into floating hematopoietic round-shaped cells (Figure 9B). We observed that the number of blood progenitors (KIT⁺ CD41^{low} CD45⁺) was significantly increased in the cultures from SADEiGEN-induced KDR+ progenitors compared to scrambled-induced samples (Figures 4D medium and bottom panels and 9B). These results indicate that SADEiGEN induction during mesodermal specification primes the mesoderm progenitors for an enhanced potential to undergo EHT. Since HSCs are difficult to obtain and maintain in vitro, we investigated the capacity of KDR⁺ cells to develop into HSPCs in vitro upon inducing SADEiGEN expression during mesoderm specification. We cultured 3x10⁵ KDR⁺ cells in serum-free StemSpan media (Iscove's MDM medium supplemented with HSC-specific cytokines) for 96h, and analyzed the cell output by flow cytometry (Figure 4E upper panel). We initially observed a higher growth of floating round-shaped hematopoietic cells in SADEiGEN-induced cells compared to scrambled-induced samples (Figure 9C). When we analyzed the different blood progenitor populations, we detected a higher number of lineage-negative (Figure 9D) and a slightly increased population of LIN⁻SCA-1⁺KIT⁺ (LSK) progenitors in the SADEiGEN-induced cells (Figure 4E medium panel). Importantly, phenotypically defined HSPCs (LSK CD150⁺CD48⁻), which contain the long-term HSCs, were highly enriched in the SADEiGEN-induced cells compared to scrambled-induced cultures (Figure 4E bottom panel). Finally, we determined the capacity of KDR+-derived cells to differentiate into different types of progenitors by colony forming units (CFU): CFU-GEMM, CFU-GM, CFU-M, CFU-G and Erythroid (Figures 4F upper panel and 9E; see materials and methods). We seeded 2x10⁵ KDR⁺ progenitors in methylcellulose media and counted the number and type of colonies that were formed after 8 days. We found that SADEiGEN-induced cells generated higher diversity of multipotent and oligopotent progenitors, including the most undifferentiated CFU-GEMM and CFU-GM progenitors, whereas scrambled-induced samples were mainly showing erythroid potential (Figure 4F).

We further studied whether transduction of iVPR-mESCs with a different set of gRNAs targeting SADEiGEN (see Materials a Methods) led to a comparable hematopoietic phenotype. These new clones were successful in inducing SADEiGEN expressions with similar dynamics as the original gRNAs (Figure 9A and 9F). Activation of the new set of SADEiGEN gRNAs also conferred higher EHT potential, with increased number of blood progenitors (KIT⁺ CD41^{low} CD45⁺) (Figure 9G), and a significant increase in the number of HSPCs (LSK CD150⁺ CD48⁻) (Figure 9H). Finally, the proportion of multipotent (GEMM) and oligopotent (GM) progenitors was also increased compared to scrambled gRNAs, as similarly observed with the original set of gRNAs (Figure 91).

Collectively, these results indicate that induction of SADEiGEN cocktail during differentiation of EBs, and prior to the specification of KDR⁺ progenitors, results in the acquisition of a hemogenic/HSPC potential in mesoderm progenitors in vitro and in vivo.

### SADEiGEN activation during differentiation favors the formation of intraembryonic mesodermal precursors rather than extraembryonic tissue

To further understand the changes imposed by SADEiGEN induction in KDR⁺ mesodermal progenitors, we performed scRNA-seq analysis from 144h EBs derived from SADEiGEN-induced and scrambled-gRNA-induced KDR+ cells. We performed scRNA-seq from 3 independent EBs differentiation experiments and cells were represented in a UMAP to identified the different cell clusters present. Comparative analysis of our data with a scRNA-seq dataset of E6.5-E8.5 mouse embryo ²⁷ allowed the identification of different cell clusters, including those resembling the epiblast cells that evolve towards gastrulation through the primitive streak giving rise to different endodermal, mesodermal and ectodermal lineages. We also identified clusters that overlap with different layers of extraembryonic tissues such as extraembryonic mesoderm, ectoderm and allantois (Figures 5A and 10A). Unexpectedly, scrambled and SADEiGEN-derived 144h EBs were quite similar in terms of cell type composition (Figures 5B), but they differ primarily in the proportion of different cell fates (Figures 5C-D and 10B). Specifically, the extraembryonic allantois cluster was strongly reduced in the SADEiGEN-induced EBs (Figures 5C-D and 10C), whereas cell types associated to intraembryonic mesoderm fates such as Primitive Streak, Pharyngeal Mesoderm, Cardiomyocytes and Nascent/Mixed Mesoderm (NMM) were highly enriched (Figures 5D and 10C). These results suggest that activation of SADEiGEN during germ layer specification biases the differentiation towards intraembryonic mesoderm fates which are precursors of the definitive HSCs ²⁸. To further characterize the type of mesoderm generated after SADEiGEN induction, we performed a pseudo-bulk differential expression analysis of the single-cell KDR⁺ transcriptome (Figure 5E, see materials and methods section). SADEiGEN-induced KDR⁺ progenitors presented higher expression of mesoderm-specific genes (*Eomes, Mixl1, Msx2, Lhx1, Wnt3*)*,* while displaying lower levels of extraembryonic markers compared to scrambled samples (Figure 5F). In fact, genes upregulated in SADEiGEN-induced KDR⁺ progenitors belong to biological processes related to Aorta-Gonad-Mesonephros (AGM) specification (Figure 5G), which is the niche where the first blood stem cells are specified ¹⁵.

Collectively, our results demonstrate that SADEiGEN induction during germ layer specification biases differentiating cells toward a more homogeneous intraembryonic mesodermal lineage (Figure 5H), with higher capacity to generate definitive HSPC potential with multilineage engraftment and self-renewal potential in vivo.

### METHODS

### Cell culture

mESC line ES-E14TG2a (ATCC; Cat #CRL-1821) was cultured on plastic dishes precoated with 0.1% (w/v) Gelatin (Sigma; Cat #G2500-100G) in Serum/LIF medium. Serum/LIF is composed by DMEM basal medium (Sigma-Aldrich; Cat #D5796) supplemented with 15% FBS (ESC-qualified; Gibco Cat #26140079), 1X Glutamax (Gibco; Cat #35050061), 1X NEAA (Gibco; Cat#11140050), 1mM Sodium Pyruvate (Gibco; Cat #11360070), 1000U/mL Leukemia Inhibitory Factor (LIF) (Millipore, Cat #ESG1107), 100U/mL Penicillin/Streptomycin (Gibco; Cat #15140122) and 0.125mM 2-mercaptoethanol (Gibco; Cat#31350010). HEK293T cell line (ATCC; Cat #CRL-3216) was cultured in DMEM basal medium supplemented with 15% FBS (Gibco, Cat #A5256701) 1X Glutamax, 1X NEAA, 1mM Sodium Pyruvate, 100U/mL penicillin/Streptomycin and 0.125mM 2-mercaptoethanol. MS-5 (DSMZ; Cat #ACC-441) and MS-5-hDLL1-EGFP (MERCK, Cat #SCC167) cell lines were cultured in alpha-MEM medium (Stem Cell Technologies; Cat #36450) supplemented with 20% FBS (ESC-qualified; Gibco, Cat #36450) and 100U/mL Penicillin/Streptomycin. Medium was changed every day, and cells were splitted every other day using TrypLE Express (Gibco; Cat #12605010). Cells were maintained in a 5% CO₂ incubator at 37°C.

### Embryoid body differentiation

EB differentiation was established as described in ²⁴ with some modifications specified in ⁴⁹. Briefly, mESCs were split twice in Serum/LIF medium before inducing differentiation. Once mESCs are relatively high confluent (around 80%), cells were collected using TrypLE Express. One well of 6-well plate was split in an entire 6-well plate in IMDM-ES medium for 48h. IMDM-ES medium is composed of Iscove's Modified Dulbecco's Medium (IMDM) (Cytiva, Cat #16SH30259.01) supplemented with 20% FBS (ESC-qualified; Gibco Cat #26140079), 1X Glutamax (Gibco; Cat #35050061), 1X NEAA (Gibco; Cat#11140050), 1mM Sodium Pyruvate (Gibco; Cat#11360070), 10ng/mL Leukemia Inhibitory Factor (LIF) (Millipore, Cat #ESG1107) and 0.125mM 2-mercaptoethanol (Gibco; Cat#31350010). For embryoid body induction, mESCs were harvested, and they were rinsed twice with DPBS (Gibco; Cat #14190144). Cells were very well disaggregated into single-cells, and 1.2 x 10⁴ cells/mL embryoid body differentiation (EB^{diff}) medium were resuspended to a total volume of 25mL of EB^{diff} medium. EB^{diff} medium is composed by IMDM supplemented with 15% FBS (ESC-qualified; Gibco Cat #26140079), 1X Glutamax (Gibco; Cat #35050061), 50 µg/mL ascorbic acid (Sigma; Cat #A-4544), 180 µg/mL Transferrin (Roche; Cat #10652202001) and 0.45 mM alpha-monothioglycerol (MTG) (Sigma; Cat #M6145). EBs were formed in suspension for 6 days (144h). On day 6, EBs were collected and dislodged, and KDR⁺ cells were purified by MACS cell separation (Miltenyi Biotec) or Flow Activated Cell Sorting (FACS) for further experiments.

### Doxycycline-inducible CRISPRa (VPR) cell line generation

ES-E14TG2a mESCs were engineered with the doxycycline-inducible CRISPRa system VPR ¹⁸. mESCs were co-transfected with PB-TRE-dCas9-VPR (Addgene, #63800) and PiggyBac transposase (System Biosciences, #PB210PA-1) plasmids using Lipofectamine 2000 (Invitrogen, #11668027) following manufacturer's instructions. Opti-MEM medium (Gibco, #11058021) was used during the transfection procedure. iVPR-mESCs were selected by treatment with 100µg/mL of Hygromycin B Gold (Invivogen, #ant-hg-1) for 7 days, and single-cell clones were tested for endogenous gene activation. The best iVPR-mESC clone having the best leaky-free and gene activation activities was selected to perform further experiments. List of gRNAs and primers used are listed in supplementary table 4.

### gRNA library preparation and sequencing

Mouse Genome-wide CRISPRa-v2 gRNA library ²⁰ was purchased from Addgene (Cat #83996). gRNA library targets promoters in a genome-wide fashion (19939 genes) with 5 gRNAs per gene (107105 total gRNAs). gRNA library was amplified as described in ⁵⁰. Briefly, NEB 10-beta electrocompetent cells (NEB, Cat #C3020K) were electroporated in five simultaneous reactions, each one containing 1µL of gRNA library (at 20ng/µL concentration) and 20 µg of cells, and the mix was recovered 1 hour at 37°C in 1mL of SOC medium. Reactions were then pooled and electroporated cells were grown overnight at 37°C in 1 liter of 2XTY medium (5 g/L NaCl, 16 g/L tryptone, 10 g/L yeast extract) supplemented with 10µg/mL ampicillin (Sigma-Aldrich, Cat #A9393). gRNA library was purified using QIAGEN plasmid maxi kit (QIAGEN, Cat #12165) following manufacturer's instructions. gRNA library DNA concentration was determined using Nanodrop (Thermo Fisher Scientific, Cat #ND-1000).

gRNA library was sequenced as reported in ⁵¹. More in detail, 20 ng pooled gRNA library were PCR-amplified using PrimeSTAR HS DNA Polymerase (TAKARA Bio, Cat #R010A). Illumina NGS primers were incorporated in two rounds of PCR, where initially gRNAs-containing amplicon was obtained by using common primers placed in the gRNA backbone. Illumina adapters were coupled in a second PCR reaction. DNA was purified using QIAEX II Gel Extraction Kit (QIAGEN, Cat #20051) following manufacturer's extraction. List of primers used for next generation sequencing (NGS) are included in supplementary table 4. 3x10⁷ reads per sample were sequenced by single read 50 bp in HiSeq2500 (Illumina).

### Lentiviral vector production and titering

2x10⁷ HEK293T cells were seeded in a 146X21mm (Techno Plastic Products; Cat #93150) plastic petri dish a day before transfection. Cells were transfected with 7.5µg pCMV-dR8.2 dvpr (Addgene, Cat #8455), 3µg pMD2.G (Addgene; Cat#12259) and 10µg gRNA library prep using 4µL/µg DNA of Polyethylenimine (Sigma-Aldrich, Cat #764892) and they were incubated in DMEM basal medium (without penicillin/streptomycin nor serum supplementation) for 5 hours. Medium was then replaced by regularly supplemented DMEM medium. Cell supernatant was collected 48 hours after transfection, and lentiviral particles were concentrated by Lenti-X-Concentrator (TAKARA Bio, Cat #631232) following manufacturer's instructions (supernatant::Lenti-X-Concentrator mix incubated for 5 hours at 4°C before centrifuge for 45 minutes at 1500g). Lentiviral pellet was resuspended in DPBS 1X and it was frozen down at -80°C before its use.

gRNA library lentiviral titering was performed by seeding 10⁵ iVPR-mESCs per 1.86 cm² well (24-well plate), and increasing amount of lentiviral concentrate was added into the cells. Blue Fluorescent Protein (BFP) expression was used as a marker of gRNA library incorporation into the cells, and number of lentiviral particles was calculated based on percentage of BFP⁺ iVPR-mESCs.

### CRISPRa genome-wide screen

### Lentiviral gRNA library infection of iVPR-mESCs

10⁵ iVPR-mESCs were seeded in 0.1% Gelatin-precoated 1.86cm² wells (24-wells plate), and incubated with the lentiviral gRNA library at a Multiplicity of Infection (MOI) of 2.5 for 16 hours and in Serum/LIF medium supplemented with 10 µg/mL Polybrene (Sigma-Aldrich, Cat #28728-55-4), lentivirus-containing medium was then washed with DBSP and regular Serum/LIF medium was added into cells for another 24 hours. Infected cells were selected by 1µg/mL Puromycin treatment (Sigma-Aldrich, Cat #58-58-2) for 96h, using an uninfected control as a reference. iVPR-mESCs were infected 4 times in order to have a high number of gRNAs per cell. To know the gRNA distribution per cell, independent infected single-cell clones were sorted using FACS (BFP expression, since BFP cassette is contained in the gRNA construct) and their gRNAs were sequenced by NGS.

### gRNA library-iVPR-mESCs differentiation towards KDR⁺ mesodermal progenitors

4 round-infected iVPR-mESCs were differentiated towards KDR⁺ mesodermal progenitors as described above. 72 hours EBs were supplemented with 1 µg/mL doxycycline (Tocris, Cat #4090) for another 72 hours, in order to activate the gRNA library.

### Transplantation of KDR⁺ cells into NSG mice (Primary and Secondary Transplants)

144 hours EBs were disaggregated by TrypLE Express (3 minutes incubation at 37°C), and cells were stained with 10 µL/10⁷ cells of Anti-mouse CD309-Biotin (Invitrogen, Cat #13-5821-82) for 20 min at room temperature and dark. Cells were then washed and incubated with either 10µL/10⁷ cells of anti-biotin microbeads (Miltenyi Biotec, Cat #130-090-485) when Magnetic-activated cell sorting (MACS) is used for purifying KDR⁺ (CD309⁺) cells or 1:200 Streptavidin-PE (BD Biosciences, Cat #554061) for Flow Activated Cell Sorting (FACS).

6.5x10⁵ KDR⁺ cells were isolated and transplanted into sublethally irradiated (2Gy) NSG (NOD.Cg-Prkdc^{scid} Il2rg^{tm1Wjl}/SzJ) mice (The Jackson laboratory, Strain #005557). 2x10⁴ bone marrow cells (BFP⁻) from NSG mice were transplanted together as support. Transplantation of cells were performed by retro orbital injection with a 27.5Gx12mm syringe, and cells (KDR⁺ and support) were resuspended in 150µL DPBS. For secondary transplantation, bone marrow from primary transplants were isolated by flushing, and half of cells was transplanted into a secondary recipient (sublethally irradiated NSG mouse), whereas the other half was harvested for gRNA sequencing. List of mice used and the engraftment of mESCs-derived KDR⁺ progenitors are listed in supplementary table 1.

### Sample preparation for gRNA library sequencing in CD45⁺/BFP⁺ transplanted cells

Bone marrow from gRNA-library KDR⁺-transplanted NSG mice was isolated by sacrificing mice and crushing long-bones ⁵². Bone marrow cells were erythrocyte-depleted by incubation with 10 times more of ACK lysis buffer (Gibco, Cat #A1049201) than volume of cells; they are incubated for 10 minutes at room temperature, and cells were centrifuged at 300g and resuspended in DPBS supplemented with 10% FBS for further manipulation. A second round of lysis is performed when the bone marrow pellet remains highly red; this second lysis is performed for 3 minutes at room temperature. Sample was stained with 1:200 of FITC Rat anti-mouse CD45 (BD Pharmingen, Cat #553079) for 15 minutes at room temperature in the dark and washed once with DPBS. CD45⁺ BFP⁺ cells (hematopoietic cells derived from mESCs) were isolated by FACS using BD FACSAria II Cell Sorter (BD Bioscience). Genomic DNA (gDNA) was isolated using 30µL/500 cells of lysis buffer (100mM Tris-HCl pH8, 5mM EDTA, 0.1%SDS, 200mM NaCl and 200 µg/mL Proteinase K) and samples were incubated 3 hours at 55°C and 1 hour at 85°C. gDNA solution was diluted 1:5 and gRNAs were sequenced as described above.

### CRISPRa screening gRNA library analysis

Data from Genome-wide CRISPRa screening was analyzed using the MAGeCK algorithm⁵³ and following the MAGeCKFlute pipeline ⁵⁴. gRNA abundance comparison was determined by comparing gRNA distribution in KDR⁺ cells (time 0) against CD45⁺ BFP⁺ from bone marrow of primary and secondary transplantations. Common gRNAs detected across primary and secondary transplants from three independent experiments were considered as putative hits. List of CRISPRa screen hits are listed in supplementary table 2.

### CRISPRa screening validation

2 different gRNAs targeting Spata2, Aass, Dctd, Eif4enif1, Guca1a, Eya2 and Net1 promoters were cloned into the pKLV-U6gRNA(BbsI)-PGKpuro2ABFP plasmid (Addgene, plasmid #50946). gRNAs were introduced by lentiviral vectors into iVPR-mESCs, and single-cell clones containing the 7 gRNAs in the same cell (each one targeting one of the seven genes: SADEiGEN cocktail) were screened by PCR and used for further experiments. Validation experiments were performed using iVPR-mESCs containing either non-targeting scrambled gRNA or SADEiGEN gRNAs. mESCs were differentiated towards KDR⁺ progenitors as described above, and they were purified by MACS or FACS.

### In vivo transplantation of KDR⁺ cells (Primary and Secondary recipients)

For primary transplantation, 6.5x10⁵ KDR⁺ cells from scrambled or SADEiGEN conditions were injected intratibially into sublethally irradiated (2Gy) NSG mice, together with 2x10⁴ bone marrow cells from NSG mice as a support. KDR and support cells were centrifuged together at 300g for 5 minutes, and pellet was resuspended into 30 µl DPBS and injected using 29Gx12.7mm syringe (Becton Dickinson, Cat #324892). For secondary transplants, CD45⁺ BFP⁺ bone marrow cells from 8 weeks primary recipient were isolated, and 10⁵ of them were transplanted intratibial into a secondary recipient.

### Chimerism analysis in bone marrow and peripheral blood

Peripheral blood of transplanted mice were analyzed at 4, 8 and 12 weeks in primary and 4, 8, 12 and 16 weeks in secondary recipients by BD LSRFortessa Cell Analyzer or BD LSR II Flow Cytometer (BD Bioscience). Blood was incubated with ACK lysis buffer to remove the erythrocytes when erythroid lineage was not analyzed. The presence of donor chimerism (KDR⁺-derived cells) was considered when CD45⁺ BFP⁺ cells were detected in peripheral blood. Blood lineages analysis in either peripheral blood (8 weeks in primary transplants and 14 weeks in secondary transplants) or bone marrow (8 weeks in primary transplants and 14 weeks in secondary transplants) was done by flow cytometry and data was analysis using FlowJo v10.0.7 (BD Bioscience). List of antibodies used are found in supplementary table 5. Engraftment quantifications per mouse in validation experiments are found in supplementary table 3.

### Animal experiments

2-4 months old NOD.Cg-Prkdc^{scid} Il2rg^{tm1Wjl}/SzJ mice (The Jackson Laboratories, Strain #005557) were used for experiments, regardless of their gender, and similar numbers of female and male mice were used in experiments. Animals were kept under pathological-free conditions, and all animal-related work was approved by the Animal Care Committee of the Barcelona Biomedical Research Park (PRBB) and Catalan Government (Generalitat de Catalunya), with the license number 9309. Animal care and use is approved by the PRBB-Ethics committee and accredited by AAALAC International, following European (2010/63/UE) and Spanish (RD 53/2013) legislation.

### In vitro Hematopoietic Stem Cell specification

3x10⁵ EB-derived KDR⁺ cells were seeded in 9.6cm² wells (6-well plate) and cultured in HSC expansion medium for 96 hours. HSC expansion medium is composed by StemSpan Serum-Free Expansion Medium (Stem Cell Technologies, Cat #09600) supplemented with 50 ng/mL recombinant murine SCF (Peprotech, Cat #250-03), 25ng/mL recombinant murine TPO (Peprotech, Cat #315-14), 30ng/mL recombinant murine Flt3l (Peprotech, Cat #250-31L) and 1X Glutamax. Non-adherent cells were collected for flow cytometry analysis.

### In vitro Endothelial-to-Hematopoietic (EHT) differentiation from mESCs

EHT differentiation has been previously described in ²⁴. EB-derived KDR⁺ cells were seeded in 9.6cm² wells (6-well plate), precoated with 0.1% Gelatin, and in liquid blast medium for 72 hours. Liquid blast medium is composed by IMDM basal medium supplemented with 10% FBS (ESC-qualified, Gibco Cat #26140079), 1X Glutamax, 25 µg/mL L-Ascorbic Acid (Sigma-Aldrich, Cat #A4544), 0.45 mM Alpha-Monothioglycerol (MTG) (Sigma-Aldrich, Cat #M6145), 180 µg/mL Transferrin, 10 ng/mL recombinant murine interleukin 6 (R&D systems, Cat #406-ML), 5 ng/mL recombinant murine VEGF (Peprotech, Cat #450-32) and 15% conditioned medium from D4T cell line (Keller 1995). Non-adherent and adherent cells were collected after 72 hours for further analysis by flow cytometry.

### In vitro myeloid and lymphoid differentiation from mESCs

Myeloid and lymphoid in vitro differentiation has been previously described ⁵⁵ mESCs were differentiated towards KDR⁺ mesodermal progenitors as described above. Either scrambled or SADEiGEN 72 hours EBs were supplemented with 1 µg/mL doxycycline for 48 hours. 120 hours EBs were disaggregated to single cells, KDR⁺ cells were purified and 5x10⁵ KDR⁺ cells were seeded on MS5-precoated 56.7cm² plates for 72 hours in alpha-MEM medium supplemented with 20% FBS (ESC-qualified), 100U/mL penicillin/Streptomycin and 5ng/mL recombinant murine Flt3l. Floating hematopoietic progenitors were then seeded in 9.6cm² wells in alpha-MEM medium supplemented with 20% FBS (ESC-qualified), 100U/mL penicillin/Streptomycin, 1 ng/mL recombinant murine IL-7 (Peprotech, Cat #217-17) and 5ng/mL recombinant murine Flt3 ligand. Wells were pre-coated with either MS5 or MS5-DLL1 feeder cells depending of the desired blood lineage to be obtained: i) for erythroid, monocytic or B cells, wells were coated with MS5, and ii) for T cells, MS5-DLL1 were used. Medium was changed every other day, and the feeder layer was replaced every 4 days.

### Colony forming unit assays

2.5x10⁵ EBs-derived KDR⁺ cells were seeded in 1.9cm² wells (24-well plate) and cultured in MethoCult GF M3434 (Stem Cell Technologies, Cat #03434) for 192 hours (8 days). Formed colonies were screened and classified according to morphology in colony-forming-unit (CFU) erythroid (Erythroid), CFU-Macrophage (CFU-M), CFU-Granulocyte (CFU-G), CFU-Granulo/Macrophages (CFU-GM) and CFU multipotent (CFU-GEMM). Number of colonies was quantified to estimate the proportion of each CFU type.

### RNA isolation, cDNA synthesis and quantitative RT-PCR

Total RNA isolation from cells was performed using the RNeasy Plus Mini Kit (Qiagen; Cat #74136) or RNeasy Micro Kit (Qiagen; Cat #74004) following manufacturer's instructions. Amount of RNA was quantified with Nanodrop (Thermo Fisher; Cat #ND2000CLAPTOP), and 2 µg of total RNA was retro-transcribed using Transcriptor First Strand cDNA Synthesis Kit (Roche; Cat #04897030001) following the manufacturer's instructions.

Quantitative RT-PCR was performed in triplicates for each sample, and SYBR Green I Master Kit (Roche; Cat #04887352001) was used to carry out the reaction. qRT-PCR was performed using the LightCycler 480 system (Roche). Relative expression levels were calculated as 2^{-ΔCT} normalized with the average CT of the housekeeping *Tbp* or *Gapdh.* List of oligos sequences is found in supplementary table 4.

### Blood functional assays

### B cell maturation analysis

Bone marrow and spleen from primary transplanted mice (8 weeks post-transplantation) were isolated, and maturation analysis was based on the protein expression of B-220 (pan B cell marker) and B cell receptor (IgM). Further maturation markers (CD80, CD86, IgD, IgG) were analyzed by RNA expression using the sc-RNA-seq data generated from secondary transplanted mice at 18 weeks.

### Phagocytosis assays

Primary macrophages were isolated from a primary transplanted bone marrow (12 weeks post-transplantation) by purifying CD45⁺ MAC-1⁺ using BD FACSAria II (BD Biosciences). Cells were seeded at a confluency of 3x10⁴ cells/cm² for 72h in macrophage-amplifying culture medium (Mac-Medium; IMDM supplemented with 10% mESC-qualified FBS, 100U/mL Penicillin/Streptomycin, 1X Glutamax, 1X NEAA, 125µM 2-mercaptoethanol, 1X Na Pyruvate and 25ng/mL M-CSF[Peprotech, Cat #315-02]). Macrophages were splitted twice in Mac-Medium before phagocytosis assays were performed. For phagocytosis assays, macrophages were seeded at three times higher confluency (9x10⁴ cells/cm²) a day before performing the assay. Cells were incubated for 3 hours with 5µL of green-fluorescent latex beads (Sigma-Aldrich, Cat #L1030), then they were washed three times with PBS 1X and fixed for 10 minutes at room temperature using 4%PFA. Wash three more times with PBS and incubate for 1 at room temperature with 1:500 phalloidin-rhodamine solution (Invitrogen, Cat #R415). Cells were mounted using Fluoromont-G (Southern Biotech, Cat #0100-01). Immunofluorescent images were acquired using Leica TCS SP5 Confocal microscope (Leica Microsystems), and images were analyzed using ImageJ software ⁵⁶. Phagocytosis efficiency was calculated by counting the average number of engulfed beads per CD45⁺ MAC1⁺ cell (either recipient- or SADEiGEN-derived).

### Statistical analysis

Statistical analysis was performed with GraphPad Prism v.8.0.1. (GraphPad Software, Inc.). Unless specified, the comparison between two groups was performed with unpaired two-sided t-test. A p-value < 0.05 was considered significant. For multiple comparisons, 2-way ANOVA was applied.

### Single-cell RNA sequencing

Single-cell RNA sequencing 3' (sc-RNAseq) was performed using the Chromium Controller from 10X Genomics according to the manufacturer's instructions (CG000315 Rev E). For bone marrow sc-RNAseq, 4.5 months post-transplantation bone marrow cells were isolated by flushing the long-bones of 3 independent mice, pooling together all the bone marrow cells. Cells were processed as previously reported, and they were stained with rat anti-mouse FITC-CD45 antibody (1:200). Cells were isolated by FACS based on their origin: i) recipient (CD45+ BFP-) or SADEiGEN-derived cells (CD45+ BFP+). For sc-RNAseq EBs, 144h EBs were dislodged byTrypLE, and alive cells were isolated by FACS. Cellular suspension was counted manually with a hemocytometer, and visualized under a ZEISS Primovert Microscope. Cells were resuspended in PBS + BSA 0.04% to a final concentration of 700-1200 cells/µl per sample. Number of cells encapsulated varied from 10000 to 14000 depending on the sample, to achieve a final target cell recovery of 5000-7000 cells. Resulting libraries were quantified and quality checked by using the 2200 TapeStation^{®} (Agilent) and sequenced on a NovaSeq X Plus instrument (Illumina), in a 150 bp paired-end run strategy with a total of 60 G raw data per sample.

### Data preprocessing

10x sequencing raw reads were demultiplexed and aligned with 10x Genomics CellRanger v7.2.0 (cellranger count) under default parameters which includes intronic reads ⁵⁷. Sequences were aligned against the mouse pre-built reference transcriptome refdata-gex-mm10-2020-A provided by 10x Genomics. Features, barcodes and expression matrices were separately obtained per sample (3 scrambled and 3 SADEiGEN). Respectively for scrambled and SADEiGEN conditions, (i) the average of recovered cells was 7,894 and 7,832 cells, (ii) the median genes per cell was 3,848 and 3,773 genes and, (iii) the mean reads per cell was 26,952 and 28,615 reads.

### Quality control

Generated filtered matrices were imported and merged into R (v4.3.1) using Seurat package (v5.0.0) ⁵⁸. A Seurat object with a total of 32,285 genes and 47,177 cells was available prior to quality control (QC). Doublets were separately detected by sample with scDblFinder package (v1.14.0) ⁵⁹. For this purpose, random and cluster-based approaches, implemented in the same package, were used. Additionally, the latter was considered with the clusters identified by CellRanger software (graph-based). Those cells detected as doublets by two out of the three cases were finally labeled as doublets and removed from the dataset. Next, cells were filtered based on the number of genes removing those with less than 300 or more than 7k genes. Cells with more than 10% of mitochondrial gene content or with less than 1kcounts were also discarded. Genes not present in at least 10 cells were filtered out. Ribosomal genes were as well discarded. After QC, a Seurat object with a total of 22,492 genes and 34,950 cells was used for downstream analysis.

### Clustering

Samples were normalized using the SCTransform function regressing the mitochondrial content out and with the method glmGamPoi (package v1.12.2). No sample integration was required. For dimensionality reduction and visualization, runPCA and runUMAP functions were executed considering 38 principal components which corresponded to a cumulative captured data variance > 85%. Cell phase score was computed with the CellCycleScoring function and available S and G2M genes in the Seurat package. Clustering analysis was performed with FindNeighbors (38 dimensions) and FindClusters functions with default parameters except for the resolution (0.4). Expression values were imputed and smoothed with the MAGIC algorithm through Rmagic package (v2.0.3.999) for gene expression visualization purposes ⁶⁰.

### Cell type annotation

Cell types were individually annotated by means of the SingleR package (v2.2.0) ⁶¹. For this purpose, the mouse gastrulation and early organogenesis dataset from Pijuan-Sala et al. was considered as the reference ²⁶. This dataset was retrieved using the MouseGastrulationData R package (1.14.0) (Griffiths & Lun, 2024). Briefly, we used 34 samples out of the 36 available after excluding samples labeled as 'mixed gastrulation' in their stage. Samples included developmental stages from E6.5 to E8.5. After removing cells with NAs in their cell type, a total of 108,857 cells, distributed in 37 different cell types, were used as reference. For automated labeling, both sets (test and reference) were log normalized with scuttle (v1.10.3) ⁶². SingleR was executed with default parameters except for de.method, set to wilcox. Those cell types identified in <0.1% total cells were re-labelled as underrepresented (125 cells). A total of 466 cells remained unlabeled.

### Differential abundance analysis

Differential abundance analysis (DAA) was conducted with miloR (v1.8.1) which is the R implementation for the milo method ⁶³. Briefly, the k-nearest neighbor graph was built considering k=30 and 38 dimensions. Experimental design only included sample conditions (SADEiGEN vs scrambled) to be tested. Neighborhood connectivity was computed for further p-values corrections (Spatial FDR). Differentially abundant neighborhoods were considered if corrected p-value (Spatial FDR) < 0.1%. Each neighborhood was assigned to the annotated cell types. Those neighborhoods with a mix of cell types (fraction of main cell type < 70%) were relabeled to 'Mixed' population. Finally, neighborhoods were grouped considering minimum overlap of 3 and maximum delta log fold change of 2.

### Pseudo-bulk differential expression analysis

A subset of cells from the processed dataset was considered for differential expression analysis, specifically, those cells with at least 1 raw count assigned to KDR⁺ were selected. A total of 5,954 cells across the six initial samples were included. Raw counts were aggregated per sample with the AggregateExpression function from Seurat. Experimental design only included sample conditions (SADEiGEN vs scrambled) to be tested. Prior to statistical analysis, those genes with less than 5 raw counts across the 6 samples under test were removed. For Principal Component Analysis (PCA), counts were normalized by the variance-stabilizing transformation method as implemented in DESeq2 R package (v1.40.2) (Love, Huber, and Anders 2014). Differential expression analysis (DEA) was conducted with DESeq2. Obtained log2 fold change values were shrunken with apeglm shrinkage estimator R package (v1.22.1) ⁶⁴. Raw p-values were adjusted for multiple testing using the Benjamini-Hochberg False Discovery Rate (FDR) ⁶⁵. Differentially Expressed Genes (DEGs) between SADEiGEN and scrambled samples were called with adjusted p-values (FDR) < 0.05 and absolute shrunken log₂ Fold change > 0.5.

### Functional analysis

Overrepresentation analysis was conducted over DEGs from the pseudo-bulk differential expression analysis against the Gene Ontology (GO) Biological Process (BP) ⁶⁷. For this purpose, gene Entrez identifiers were used. Statistically significant GO BP terms (adjusted p-values FDR < 0.01) were simplified using Go-Figure! ⁶⁸ with default parameters.

### Bone marrow 18 weeks secondary transplantation

Same methodology and tools were applied as detailed above unless specified below.

### Data pre-processing

Features, barcodes and expression matrices were separately obtained per sample (1xCD45 BPF⁻ and 1xCD45 BFP⁺, coming from the pool of three different mice after 18 weeks of a secondary transplantation). Respectively for CD45 BPF⁻ and CD45 BFP⁺ samples, (i) the number of recovered cells was 8,461 and 5,635 cells, (ii) the median genes per cell was 1,405 and 1,714 genes and, (iii) the mean reads per cell was 24,338 and 34,029 reads.

### Quality control

A Seurat object with a total of 32,285 genes and 14,096 cells was available prior to QC. Cells labeled as doublets, with a number of genes less than 300 or more than 10000, with more than 12% of mitochondrial gene content or with less than 500 counts were discarded. Genes not present in at least 5 cells were filtered out. Ribosomal genes were as well discarded. After QC, a Seurat object with a total of 17,741 genes and 12,530 cells was available for analysis.

### Sample integration and clustering

After SCTransform normalization, both samples were integrated using the anchor-based CCA integration method implemented in Seurat. The integrated dataset was used for downstream analysis. A total of 35 principal components were considered for dimensionality reduction. Clustering analysis was performed at a resolution of 0.4 identifying an initial set of 17 clusters. After sub-clustering of specific clusters (originally labeled as 11, 12, 13 and 15), a final set of 21 clusters was annotated. Normalized expression values, prior to integration, were imputed and smoothed with the MAGIC algorithm and used for gene expression visualization.

### Cell type annotation

A combination of automated and manual annotation was applied. Firstly, cells were individually annotated with the SingleR package (v2.4.1) ⁶¹ selecting the Bone Marrow samples from Mouse Cell Atlas (MCA 2.0) dataset as reference ²². Briefly, this reference dataset included 9,049 cells from three bone marrow samples and 17 annotated cell types. SingleR was executed with default parameters. Automated labelling was manually curated mainly because we observed that 'Neutrophil Mpo high' cell type was assigned to cells which did not express Mpo gene or other related neutrophil progenitors such as Elane or Ms4a3. Manual curation was based on lineage known marker genes, used by Kucinski et al. ⁶⁸ but not limited to, specifically: (i) Innate Lymphoid cells, ILC, (Gata3, Id2, Il7r, Il2rb, Il18r1, Icos, Rorc, Ahr, Csf2), (ii) Hematopoietic Stem Cells, HSC, (Hlf, Mecom, Procr, Mycn), (iii) Neutrophil Progenitors (Mpo, Elane, Prtn3, Ms4a3), (iv) Basophil cells (Prss34, Mcpt8, Cd200r3, Il4, Itga2b), (v) T-cells (Ccr6, Tbx21, Il2ra, Klrb1f), (vi) B-cells (Cd80, Cd38, Cd86, Cd19, Ighd, Ighg3, Ighm), (vi) Macrophages (Tnsfsf13, Tnfrsf21, Adgre1, Ms4a6c, Ms4a4a, Irf5, Irf7, Irf8, Irf9) and (vii) Erythroid (Hbb-bs, Hba-a1, Hbq1b, Hbb-bt, Hba-a2, Klf1, Sox6). Expression of these markers was inspected and corresponding cell types assigned to previous identified clusters. Limited number of cells were left as 'Undetermined Macrophages/Neutrophils' (0.81% in CD45 BPF⁻and 1.0%. in CD45 BFP⁺) as no consistent patter was observed. Cell type representative genes for each condition were independently obtained from SCT assay with FindAllMarkers function (Wilcoxon test, Bonferroni adjusted p-value < 0.05, logFC > 0.25, cell percentage in cluster of interest > 0.25).

### REFERENCES

1 Ema, H., Morita, Y. & Suda, T. Heterogeneity and hierarchy of hematopoietic stem cells. Exp. Hematol. 42, 74-82.e72 (2014). https://doi.org:10.1016/j.exphem.2013.11.004
2 Zhang, Y., Gao, S., Xia, J. & Liu, F. Hematopoietic Hierarchy - An Updated Roadmap. Trends Cell Biol. 28, 976-986 (2018). https://doi.org:10.1016/j.tcb.2018.06.001
3 Bigas, A., Galán Palma, L., Kartha, G. M. & Giorgetti, A. Using Pluripotent Stem Cells to Understand Normal and Leukemic Hematopoietic Development. Stem Cells Transl. Med. 11, 1123-1134 (2022). https://doi.org:10.1093/stcltm/szac071
4 Amabile, G. et al. In vivo generation of transplantable human hematopoietic cells from induced pluripotent stem cells. Blood 121, 1255-1264 (2013). https://doi.org:10.1182/blood-2012-06-434407
5 Suzuki, N. et al. Generation of engraftable hematopoietic stem cells from induced pluripotent stem cells by way of teratoma formation. Mol. Ther. 21, 1424-1431 (2013). https://doi.org:10.1038/rnt.2013.71
6 Sandler, V. M. et al. Reprogramming human endothelial cells to haematopoietic cells requires vascular induction. Nature 511, 312-318 (2014). https://doi.org:10.1038/nature13547
7 Riddell, J. et al. Reprogramming committed murine blood cells to induced hematopoietic stem cells with defined factors. Cell 157, 549-564 (2014). https://doi.org:10.1016/j.cell.2014.04.006
8 Lis, R. et al. Conversion of adult endothelium to immunocompetent haematopoietic stem cells. Nature 545, 439-445 (2017). https://doi.org:10.1038/nature22326
9 Sugimura, R. et al. Haematopoietic stem and progenitor cells from human pluripotent stem cells. Nature 545, 432-438 (2017). https://doi.org:10.1038/nature22370
10 Palma, L. G. & Bigas, A. Making Human Hematopoietic Stem Cells Without Transgenes. Cell. Reprogram. 26, 43-45 (2024). https://doi.org:10.1089/cell.2024.0020
11 Piau, O. et al. Generation of transgene-free hematopoietic stem cells from human induced pluripotent stem cells. Cell Stem Cell 30, 1610-1623.e1617 (2023). https://doi.org:10.1016/j.stem.2023.11.002
12 Ng, E. S. et al. Long-term engrafting multilineage hematopoietic cells differentiated from human induced pluripotent stem cells. Nat. Biotechnol. (2024). https://doi.org:10.1038/s41587-024-02360-7
13 Medvinsky, A. & Dzierzak, E. Definitive hematopoiesis is autonomously initiated by the AGM region. Cell 86, 897-906 (1996). https://doi.org:10.1016/s0092-8674(00)80165-8
14 de Bruijn, M. F. T. R. et al. Hematopoietic stem cells localize to the endothelial cell layer in the midgestation mouse aorta. Immunity 16, 673-683 (2002). https://doi.org:10.1016/s1074-7613(02)00313-8
15 Dzierzak, E. & Bigas, A. Blood Development: Hematopoietic Stem Cell Dependence and Independence. Cell Stem Cell 22, 639-651 (2018). https://doi.org:10.106/j.stem.2018.04.015
16 Kasbekar, M., Mitchell, C. A., Proven, M. A. & Passegué, E. Hematopoietic stem cells through the ages: A lifetime of adaptation to organismal demands. Cell Stem Cell 30, 1403-1420 (2023). https://doi.org:10.1016/j.stem.2023.09.013
17 Calvanese, V. & Mikkola, H. K. A. The genesis of human hematopoietic stem cells. Blood 142, 519-532 (2023). https://doi.org:10.1182/blood.2022017934
18 Chavez, A. et al. Highly efficient Cas9-mediated transcriptional programming. Nat. Methods 12, 326-328 (2015). https://doi.org:10.1038/nmeth.3312
19 Pickar-Oliver, A. & Gersbach, C. A. The next generation of CRISPR-Cas technologies and applications. Nat. Rev. Mol. Cell Biol. 20, 490-507 (2019). https://doi.org:10.1038/s41580-019-0131-5
20 Horlbeck, M. A. et al. Compact and highly active next-generation libraries for CRISPR-mediated gene repression and activation. Elife 5 (2016). https://doi.org:10.7554/eLife.19760
21 Thambyrajah, R. et al. Cis inhibition of NOTCH1 through JAGGED1 sustains embryonic hematopoietic stem cell fate. Nat. Commun. 15, 1604 (2024). https://doi.org:10.1038/s41467-024-45716-y
22 Katano, I. et al. NOD-Rag2null IL-2Rγnull mice: an alternative to NOG mice for generation of humanized mice. Exp. Anim. 63, 321-330 (2014). https://doi,org:10.1538/expanim.63.321
23 Rodriguez-Fraticelli, A. E. et al. Clonal analysis of lineage fate in native haematopoiesis. Nature 553, 212-216 (2018). https://doi.org:10.1038/nature25168
24 Kucinski, I. et al. A time- and single-cell-resolved model of murine bone marrow hematopoiesis. Cell Stem Cell 31, 244-259.e210 (2024). https://doi.org:10.1016/j.stem.2023.12.001
25 Sroczynska, P., Lancrin, C., Pearson, S., Kouskoff, V. & Lacaud, G. In vitro differentiation of mouse embryonic stem cells as a model of early hematopoietic development. Methods Mol. Biol. 538, 317-334 (2009). https://doi.org:10.1007/978-1-59745-418-6_16
26 Lancrin, C. et al. The haemangioblast generates haematopoietic cells through a haemogenic endothelium stage. Nature 457, 892-895 (2009). https://doi.org:10.1038/nature07679
27 Pijuan-Sala, B. et al. A single-cell molecular map of mouse gastrulation and early organogenesis. Nature 566, 490-495 (2019). https://doi.org:10.1038/s41586-019-0933-9
28 Murry, C. E. & Keller, G. Differentiation of embryonic stem cells to clinically relevant populations: lessons from embryonic development. Cell 132, 661-680 (2008). https://doi.org:10.1016/j.cell.2008.02.008
29 McKinney-Freeman, S. et al. The transcriptional landscape of hematopoietic stem cell ontogeny. Cell Stem Cell 11, 701-714 (2012). https://doi.org:10.1016/j.stem.2012.07.018
30 Pearson, S., Cuvertino, S., Fleury, M., Lacaud, G. & Kouskoff, V. In vivo repopulating activity emerges at the onset of hematopoietic specification during embryonic stem cell differentiation. Stem Cell Reports 4, 431-444 (2015). https://doi.org:10.1016/j.stemcr.2015.01.003
31 Lugus, J. J., Park, C., Ma, Y. D. & Choi, K. Both primitive and definitive blood cells are derived from Flk-1+ mesoderm. Blood 113, 563-566 (2009). https://doi.org:10.1182/blood-2008-06-162750
32 Rongvaux, A. et al. Human hemato-lymphoid system mice: current use and future potential for medicine. Annu. Rev. Immunol. 31, 635-674 (2013). https://doi.org:10.1146/annurev-immunol-032712-095921
33 Sippel, T. R., Radtke, S., Olsen, T. M., Kiem, H.-P. & Rongvaux, A. Human hematopoietic stem cell maintenance and myeloid cell development in next-generation humanized mouse models. Blood Adv 3, 268-274 (2019). https://doi.org:10.1182/bloodadvances.2018023887
34 Sturgeon, C. M., Ditadi, A., Awong, G., Kennedy, M. & Keller, G. Wnt signaling controls the specification of definitive and primitive hematopoiesis from human pluripotent stem cells. Nat. Biotechnol. 32, 554-561 (2014). https://doi.org:10.1038/nbt.2915
35 Fowler, J. L. et al. Lineage-tracing hematopoietic stem cell origins in vivo to efficiently make human HLF+ HOXA+ hematopoietic progenitors from pluripotent stem cells. Dev. Cell 59, 1110-1131.e1122 (2024). https://doi.org:10.1016/j.devcel.2024.03.003
36 Elliott, P. R. et al. SPATA2 Links CYLD to LUBAC, Activates CYLD, and Controls LUBAC Signaling. Mol. Cell 63, 990-1005 (2016). https://doi.org:10.1016/j.molcel.2016.08.001
37 Sacksteder, K. A. et al. Identification of the alpha-aminoadipic semialdehyde synthase gene, which is defective in familial hyperlysinemia. Am. J. Hum. Genet. 66, 1736-1743 (2000). https://doi.org:10.1086/302919
38 Fugger, K. et al. Targeting the nucleotide salvage factor DNPH1 sensitizes BRCA-deficient cells to PARP inhibitors. Science 372, 156-165 (2021). https://doi.org:10.1126/science.abb4542
39 Vinberg, F., Turunen, T. T., Heikkinen, H., Pitkänen, M. & Koskelainen, A. A novel Ca2+-feedback mechanism extends the operating range of mammalian rods to brighter light. J. Gen. Physiol. 146, 307-321 (2015). https://doi.org:10.1085/jgp.201511412
40 Qin, H. et al. Characterization of the biochemical and transforming properties of the neuroepithelial transforming protein 1. J. Biol. Chem. 280, 7603-7613 (2005). https://doi.org:10.1074/jbc.M412141200
41 Räsch, F., Weber, R., Izaurralde, E. & Igreja, C. 4E-T-bound mRNAs are stored in a silenced and deadenylated form. Genes Dev. 34, 847-860 (2020). https://doi.org:10.1101/gad.336073.119
42 Di Stefano, B. et al. The RNA Helicase DDX6 Controls Cellular Plasticity by Modulating P-Body Homeostasis. Cell Stem Cell 25, 622-638.e613 (2019). https://doi.org:10.1016/j.stem.2019.08.018
43 Kodali, S. et al. RNA sequestration in P-bodies sustains myeloid leukaemia. Nat. Cell Biol. (2024). https://doi.org:10.1038/s41556-024-01489-6
44 Fougerousse, F. et al. Six and Eya expression during human somitogenesis and MyoD gene family activation. J. Muscle Res. Cell Motil. 23, 255-264 (2002). https://doi.org:10.1023/a:1020990825644
45 Patrick, A. N. et al. Structure-function analyses of the human SIX1-EYA2 complex reveal insights into metastasis and BOR syndrome. Nat. Struct. Mol. Biol. 20, 447-453 (2013). https://doi.org:10.1038/nsmb.2505
46 Forsberg, E. C. et al. Differential expression of novel potential regulators in hematopoietic stem cells. PLoS Genet. 1, e28 (2005). https://doi.org:10.1371/journal.pgen.0010028
47 Maharjan, B. D., Ono, R. & Nosaka, T. Eya2 is critical for the E2A-HLF-mediated immortalization of mouse hematopoietic stem/progenitor cells. Int. J. Oncol. 54, 981-990 (2019). https://doi.org:10.3892/ijo.2019.4673
48 Kyba, M., Perlingeiro, R. C. R. & Daley, G. Q. HoxB4 confers definitive lymphoid-myeloid engraftment potential on embryonic stem cell and yolk sac hematopoietic progenitors. Cell 109, 29-37 (2002). https://doi.org:10.1016/s0092-8674(02)00680-3
49 Yokomizo, T. et al. Independent origins of fetal liver haematopoietic stem and progenitor cells. Nature 609, 779-784 (2022). https://doi.org:10.1038/s41586-022-05203-0
50 Palma, L. G. et al. Epigenetic modifications driving ground state pluripotency exit require an NF-κB-independent chromatin IκBα function. bioRxiv (2023). https://doi.org:10.1101/2023.07.28.550934
51 Barrero, M. et al. The interferon gamma pathway enhances pluripotency and X-chromosome reactivation in iPSC reprogramming. Sci Adv 10, eadj8862 (2024). https://doi.org:10.1126/sciadv.adj8862
52 Joung, J. et al. Genome-scale CRISPR-Cas9 knockout and transcriptional activation screening. Nat. Protoc. 12, 828-863 (2017). https://doi.org:10.1038/nprot.2017.016
53 Wilkinson, A. C., Ishida, R., Nakauchi, H. & Yamazaki, S. Long-term ex vivo expansion of mouse hematopoietic stem cells. Nat. Protoc. 15, 628-648 (2020). https://doi.org:10.1038/s41596-019-0263-2
54 Li, W. et al. MAGeCK enables robust identification of essential genes from genome-scale CRISPR/Cas9 knockout screens. Genome Biol 15, 554 (2014). https://doi.org:10.1186/s13059-014-0554-4
55 Wang, B. et al. Integrative analysis of pooled CRISPR genetic screens using MAGeCKFlute. Nat. Protoc. 14, 756-780 (2019). https://doi.org:10.1038/s41596-018-0113-7
56 Kucerova-Levisohn, M. et al. Derivation of T cells in vitro from mouse embryonic stem cells. J Vis Exp, e52119 (2014). https://doi.org:10.3791/52119
57 Schneider, C. A., Rasband, W. S. & Eliceiri, K. W. NIH Image to ImageJ: 25 years of image analysis. Nat. Methods 9, 671-675 (2012). https://doi.org:10.1038/nmeth.2089
58 Zheng, G. X. Y. et al. Massively parallel digital transcriptional profiling of single cells. Nat. Commun. 8, 14049 (2017). https://doi,org:10.1038/ncomms14049
59 Hao, Y. et al. Dictionary learning for integrative, multimodal and scalable single-cell analysis. Nat. Biotechnol. 42, 293-304 (2024). https://doi.org:10.1038/s41587-023-01767-y
60 Germain, P.-L., Lun, A., Garcia Meixide, C., Macnair, W. & Robinson, M. D. Doublet identification in single-cell sequencing data using scDblFinder. F1000Res. 10, 979 (2021). https://doi.org:10.12688/f1000research.73600.2
61 van Dijk, D. et al. Recovering Gene Interactions from Single-Cell Data Using Data Diffusion. Cell 174, 716-729.e727 (2018). https://doi.org:10.1016/j.cell.2018.05.061
62 Aran, D. et al. Reference-based analysis of lung single-cell sequencing reveals a transitional profibrotic macrophage. Nat Immunol 20, 163-172 (2019). https://doi.org:10.1038/s41590-018-0276-y
63 McCarthy, D. J., Campbell, K. R., Lun, A. T. L. & Wills, Q. F. Scater: pre-processing, quality control, normalization and visualization of single-cell RNA-seq data in R. Bioinformatics 33, 1179-1186 (2017). https://doi.org:10.1093/bioinformatics/btw777
64 Dann, E., Henderson, N. C., Teichmann, S. A., Morgan, M. D. & Marioni, J. C. Differential abundance testing on single-cell data using k-nearest neighbor graphs. Nat. Biotechnol. 40, 245-253 (2022). https://doi.org:10.1038/s41587-021-01033-z
65 Zhu, A., Ibrahim, J. G. & Love, M. I. Heavy-tailed prior distributions for sequence count data: removing the noise and preserving large differences. Bioinformatics 35, 2084-2092 (2019). https://doi.org:10.1093/bioinformatics/bty895
66 Benjamini, Y. & Hochberg, Y. Controlling the False Discovery Rate: A Practical and Powerful Approach to Multiple Testing. J. R. Stat. Soc. Series B Stat. Methodol. 57, 289-300 (1995).
67 Ashburner, M. et al. Gene ontology: tool for the unification of biology. The Gene Ontology Consortium. Nat Genet 25, 25-29 (2000). https://doi.org:10.1038/75556
68 Reijnders, M. J. M. F. & Waterhouse, R. M. Summary Visualizations of Gene Ontology Terms With GO-Figure! Front Bioinform 1, 638255 (2021). https://doi.org:10.3389/fbint.2021.638255
69 Wang, R. et al. Construction of a cross-species cell landscape at single-cell level. Nucleic Acids Res. 51, 501-516 (2023). https://doi.org:10.1093/nar/gkac633

Sequence listing

Spata2 spermatogenesis associated 2 [ Mus musculus (house mouse) ]

Gene: Spata2 ENSMUSG00000047030 Gene ID: 263876
[Source:MGI Symbol;Acc:MGI:2146885]
Location
Chromosome 2: 167,323,053-167,334,807 reverse strand.
GRCm39:CM000995.3
85.4% identity in 515 residues overlap; Score: 2294.0; Gap
frequency: 0.2%
Q9UM82|SPA 7 MDTKFKDDLFRKYVQFHESKVDTTTSRQRPGSDECLRVAASTLLSLHKVDPFYRFRLIQF
Q8K004|SPA 1 MDTKYKDDLFRKYVQFHEGKVDTTPGNQQPGSDEYLRVAAATLLSLHKVDPLYRFRLIQF
**** ************* ***** * ***** ***** ********** ********

Q9UM82|SPA 67 YEVVESSLRSLSSSSLRALHGAFSMLETVGINLFLYPWKKEFRSIKTYTGPFVYYVKSTL
Q8K004|SPA 61 YEVVESSLRSLSSSSLSALHCAFSMLETMAINLFLFPWKKEFRSIKTYTGPFVYYVKSTL
**************** *** ******* ***** ************************

Q9UM82|SPA 127 LEEDIRAILSCMGYTPELGTAYKLRELVETLQVKMVSFELFLAKVECEQMLEIHSQVKDK
Q8K004|SPA 121 LEKDIRAILRFMGYEPELGTVYKLKELVESLQVKMVSFELFLAKVECEQMLGIHSQVKDK
** ****** *** ***** *** **** ******************** ********

Q9UM82|SPA 187 GYSELDIVSERKSSAEDVRGCSDALRRRAEGREHLTASMSRVALQKSASERAAKDYYKPR
Q8K004|SPA 181 GYSELDVVAERKGSTEDARGCSDALRRRAESREHLTTSMARVALQKSASERAAKDYYKPR
****** * *** * ** ************ ***** ** ********************

Q9UM82|SPA 247 VTKPSRSVDAYDSYWESRKPPLKASLSLRKEPVATDVGDDLKDEIIRPSPSLLTMASSPH
Q8K004|SPA 241 VTKPSRSVDAYDSYWESRKPPSKASLSLRKEPLAMDVGEDLKDEIIRPSPSLLAMSSSPH
********************* ********** * *** ************** * ****

Q9UM82|SPA 307 GSPDVLPPASPSNGPALLRGTYFSTQDDVDLYTDSEPRATYRRQDALRPDVWLLRNDAHS
Q8K004|SPA 301 GSPDDLSSISSINGLGLLRSTYFSTQDDVDLYTDSEPRATYRRQDALRPDVWLVKNDTHP
**** * * ** *** ********************************* ** *

Q9UM82|SPA 367 LYHKRSPPAKESALSKCQSCGLSCSSSLCQRCDSLLTCPPASKPSAFPSKASTHDSLAHG
Q8K004|SPA 361 IYHKRSPPTKESALSKCQNCGLSCSSSLCQRCDSVLVCPSASKPSAFPSKASVHDSLAHG
******* ********* *************** * ** ************ *******

Q9UM82|SPA 427 ASLREKYPG-QTQGLDRLPHLHSKSKPSTTPTSRCGFCNRPGATNTCTQCSKVSCDACLS
Q8K004|SPA 421 APMREKYVGHQTQGLDRLAPVHSKPKPSTTATSRCGFCNRAGATNTCTQCSKVSCDACLG
* **** * ******** *** ***** ********* *****************

Q9UM82|SPA 486 AYHYDPCYKKSELHKFMPNNQLNYKSTQLSHLVYR
Q8K004|SPA 481 AYHYDPCCRKSELHKFLPNSQLNYKSAPFSQLVYR
******* ******* ** ****** * ****

Aass aminoadipate-semialdehyde synthase [ Mus
musculus (house mouse) ]
Gene ID: 30956, updated on 4-Jan-2025 Gene: Aass ENSMUSG00000029695

[Source:MGI Symbol;Acc:MGI:1353573]Chromosome 6: 23,072,172-23,132,985 reverse strand.

87.6% identity in 925 residues overlap; Score: 4247.0; Gap
frequency: 0.0%

Q9UDR5|AAS 1 MLQVHRTGLGRLGVSLSKGLHHKAVLAVRREDVNAWERRAPLAPKHIKGITNLGYKVLIQ
Q99K67|AAS 1 MLRAQRPRLARLRACLSRGLHHKPVMALRREDVNAWERRAPLAPKHIKGITKLGYKVLIQ
** * * ** ** ***** * * ********************** ********

Q9UDR5|AAS 61 PSNRRAIHDKDYVKAGGILQEDISEACLILGVKRPPEEKLMSRKTYAFFSHTIKAQEANM
Q99K67|AAS 61 PSNRRAIHDKEYVRAGGILQEDITEACLILGVKRPPEEKLMSKKTYAFFSHTIKAQEANM
********** ** ********* ****************** *****************

Q9UDR5|AAS 121 GLLDEILKQEIRLIDYEKMVDHRGVRVVAFGQWAGVAGMINILHGMGLRLLALGHHTPFM
Q99K67|AAS 121 NLLDEVLKQEIRLIDYEKMVDHRGSRIVAFGQWAGVAGMINILHGMGLRLLALGHHTPFM
**** ****************** * ********************************

Q9UDR5|AAS 181 HIGMAHNYRNSSQAVQAVRDAGYEISLGLMPKSIGPLTFVFTGTGNVSKGAQAIFNELPC
Q99K67|AAS 181 HLGMAHNYRNSSQAVQAVRDAGYEISLGLMPKSIGPLTFVFTGTGNVSKGAQEVFNELPC
* ************************************************** ******

Q9UDR5|AAS 241 EYVEPHELKEVSQTGDLRKVYGTVLSRHHHLVRKTDAVYDPAEYDKHPERYISRFNTDIA
Q99K67|AAS 241 EYVEPHELREVSKTGDLRKVYGTVLSRHHHLVRKTDGVYDPVEYEKYPERYTSRFNTDIA
******** *** ********************** **** ** * **** ********

Q9UDR5|AAS 301 PYTTCLINGIYWEQNTPRLLTRQDAQSLLAPGKFSPAGVEGCPALPHKLVAICDISADTG
Q99K67|AAS 301 PYTTCLINGIYWEQNTPRLLTRQDAQSLLVPVKSSVVPVEGCPELPHKLVAICDISADTG
***************************** * * * ***** ***************

Q9UDR5|AAS 361 GSIEFMTECTTIEHPFCMYDADQHIIHDSVEGSGILMCSIDNLPAQLPIEATECFGDMLY
Q99K67|AAS 361 GSIDFMTECTTIERPFCMYDADQQIIHDSVEGSGILMCSIDNLPAQLPIEATEYFGDMLY
*** ********* ********* ***************************** ******

Q9UDR5|AAS 421 PYVEEMILSDATQPLESQNFSPVVRDAVITSNGTLPDKYKYIQTLRESRERAQSLSMGTR
Q99K67|AAS 421 PYVEEMLLSDASQPLESQNFSPVVRDAVITSNGLLTDKYKYIQKLRESRERIQFLSMSTK
****** **** ********************* * ******* ******* * *** *

Q9UDR5|AAS 481 RKVLVLGSGYISEPVLEYLSRDGNIEITVGSDMKNQIEQLGKKYNINPVSMDICKQEEKL
Q99K67|AAS 481 KKVLVLGSGYVSGPVLEYLSRDNNIEITLGSDMTNQMQQLSKKYNINPVSLTVGKQEAKL
********* * ********* ***** **** ** ** ********* *** **

Q9UDR5|AAS 541 GFLVAKQDLVISLLPYVLHPLVAKACITNKVNMVTASYITPALKELEKSVEDAGITIIGE
Q99K67|AAS 541 QSLVESQDLVISLLPYVLHPVVAKACIESRVNMVTASYITPAMKELEKSVDDAGITVIGE
** ************** ****** ************ ******* ***** ***

Q9UDR5|AAS 601 LGLDPGLDHMLAMETIDKAKEVGATIESYISYCGGLPAPEHSNNPLRYKFSWSPVGVLMN
Q99K67|AAS 601 LGLDPGLDHMLAMETIDTAKELGATVESYVSYCGGLPAPEHSDNPLRYKFSWSPVGVLMN
***************** *** *** *** ************ *****************

Q9UDR5|AAS 661 VMQSATYLLDGKVVNVAGGISFLDAVTSMDFFPGLNLEGYPNRDSTKYAEIYGISSAHTL
Q99K67|AAS 661 IMQPASYLLNGKVVNVTGGVSFLNSVTPMDYFPGLNLEGYPNRDSIKYAEIYGISSAHTL
** * *** ****** ** *** ** ** *************** **************

Q9UDR5|AAS 721 LRGTLRYKGYMKALNGFVKLGLINREALPAFRPEANPLTWKQLLCDLVGISPSSEHDVLK
Q99K67|AAS 721 LRGTLRYKGYSKALNGFVKLGLINREAYPALRPEANPLTWKQLLCDLVGISRSSPCEKLK
********** **************** ** ******************** ** **

Q9UDR5|AAS 781 EAVLKKLGGDNTQLEAAEWLGLLGDEQVPQAESILDALSKHLVMKLSYGPEEKDMIVMRD
Q99K67|AAS 781 EVVFTKLGGDNTQLEAAEWLGLLGDEQVPQAESIVDAFSKHLVSKLSYGPEEKDMIVMRD
* * ***************************** ** ***** ****************

Q9UDR5|AAS 841 SFGIRHPSGHLEHKTIDLVAYGDINGFSAMAKTVGLPTAMAAKMLLDGEIGAKGLMGPFS
Q99K67|AAS 841 SFGIRHPSGHLENKTIDLVVYGDFNGFSAMAKTVGLPTAMAAKMLLDGEIEAKGLMGPFT
************ ****** *** ************************** ********

Q9UDR5|AAS 901 KEIYGPILERIKAEGIIYTTQSTIK
Q99K67|AAS 901 KEIYGPILERIKAEGIVFNTQSTIK
**************** ******

Dctd dCMP deaminase [ Mus musculus (house mouse) ]
Gene ID: 320685, updated on 13-Jan-2025
Gene: Dctd ENSMUSG00000031562

Description
dCMP deaminase [Source:MGI Symbol;Acc:MGI:2444529]
Gene Synonyms
6030466N05Rik
Location
Chromosome 8: 48,552,127-48,606,268 forward strand.

92.7% identity in 178 residues overlap; Score: 887.0; Gap
frequency: 0.0%

P32321|DCT     1 MSEVSCKKRDDYLEWPEYFMAVAFLSAQRSKDPNSQVGACIVNSENKIVGIGYNGMPNGC
Q8K2D6|DCT     1 MSDISCKKRDDYLEWPEYFMAVAFLSAQRSKDPSSQVGACIVNTENKIVGIGYNGMPNGC
                 **  ***************************** ********* ****************

P32321|DCT    61 SDDVLPWRRTAENKLDTKYPYVCHAELNAIMNKNSTDVKGCSMYVALFPCNECAKLIIQA
Q8K2D6|DCT    61 SDDLLPWRRTAENKLDTKYPYVCHAELNAIMNKNSADVKGCSMYVALFPCNECAKLIIQA
                 *** ******************************* ************************

P32321|DCT   121 GIKEVIFMSDKYHDSDEATAARLLFNMAGVTFRKFIPKCSKIVIDFDSINSRPSQKLQ
Q8K2D6|DCT   121 GIKEVIFMSDKYHDSEETTAARLLFKLAGVTFRKFTPKYSKIVIDFDSINSRPSQKPQ
                 *************** * *******  ******** ** ***************** *

[…]

Guca1a guanylate cyclase activator 1a (retina) [ Mus
musculus (house mouse) ]
Gene ID: 14913, updated on 4-Jan-2025
Gene: Guca1a ENSMUSG00000023982

Description
guanylate cyclase activator 1a (retina) [Source:MGI Symbol;Acc:MGI:102770]
Gene Synonyms
Guca1, mGCAP1
Location
Chromosome 17: 47,705,483-47,711,509 reverse strand.

91.6% identity in 202 residues overlap; Score: 953.0; Gap
frequency: 0.5%

P43080|GUC     1 MGNVMEGKSVEELSSTECHQWYKKFMTECPSGQLTLYEFRQFFGLKNLSPSASQYVEQMF
P43081|GUC     1 MGNIMEGKSVEELSSTECHQWYKKFMTECPSGQLTLYEFRQFFGLKNLSPSASQYVEQMF
                 *** ********************************************************

P43080|GUC    61 ETFDFNKDGYIDFMEYVAALSLVLKGKVEQKLRWYFKLYDVDGNGCIDRDELLTIIQAIR
P43081|GUC    61 ETFDFNKDGYIDFMEYVAALSLVLKGKVEQKLRWYFKLYDVDGNGCIDRDELLTIIRAIR
                 ******************************************************** ***

P43080|GUC   121 AINPCSDTTMTAEEFTDTVFSKIDVNGDGELSLEEFIEGVQKDQMLLDTLTRSLDLTRIV
P43081|GUC   121 TINPWSDSSMSAEEFTDTVFAKIDINGDGELSLEEFMEGVQKDQMLLDTLTRSLDLTGIV
                  *** **  * ********* *** *********** ******************** **

P43080|GUC   181 RRLQNGEQDEEGA-DEAAEAAG
P43081|GUC   181 RRLQNGEHEEAGTGDLAAEAAG
                 *******  * *  * ******

Eya2 EYA transcriptional coactivator and
phosphatase 2 [ Mus musculus (house mouse) ]
Gene ID: 14049, updated on 4-Jan-2025
Gene: Eya2 ENSMUSG00000017897 [Source:MGI Symbol;Acc:MGI:109341]

Chromosome 2: 165,436,952-165,613,647 forward strand.

87.9% identity in 538 residues overlap; Score: 2476.0; Gap
frequency: 1.1%
O00167|EYA 1 MVELVISPSLTVNSDCLDKLKFNRADAAVWTLSDRQGITKSAPLRVSQLFSRSCPRVLPR
O08575|EYA 1 MLEVVTSPSLATSSD------WSEHGAAVGTLSDREGIAKSAALSVPQLFVKSHPRVPPG
* * * **** ** *** ***** ** *** * * *** * *** *

O00167|EYA 61 QPSTAMAAYGQTQYSAGIQQATPYTAYPPPAQAYGIPSYSIKTEDSLNHSPGQSGFLSYG
O08575|EYA 55 QSSTAMAAYGQTQYSTGIQQAPPYTAYPTPAQAYGIPPYSIKTEDSLNHSPSQSGFLSYG
* ************* ***** ****** ******** ************* ********

O00167|EYA 121 SSFSTSPTGQSPYTYQMHGTTGFYQGGNGLGNAAGFGSVHQDYPSYPGFPQSQYPQYYGS
O08575|EYA 115 PSFSTAPAGQSPYTYPVHSTAGLYQGANGLTNTAGFGSVHQDYPSYPSFSQNQYPQYFSP
**** * ******* * * * *** *** * ************* * * *****

O00167|EYA 181 SYNPPYVPASSICPSPLSTSTYVLQEASHNVPNQSSESLAGEYNTHNGPSTPAKEGDTDR
O08575|EYA 175 SYNPPYVPASSLCSSPLSTSTYVLQEAPHNVPSQSSESLAGDYNTHNGPSTPAKEGDTER
*********** * ************* **** ******** **************** *

O00167|EYA 241 PHRASDGKLRGRSKRSSDPSPAGDNEIERVFVWDLDETIIIFHSLLTGTFASRYGKDTTT
O08575|EYA 235 PHRASDGKLRGRSKRNSDPSPAGDNEIERVFVWDLDETIIIFHSLLTGTFASRYGKDTTT
*************** ********************************************

O00167|EYA 301 SVRIGLMMEEMIFNLADTHLFFNDLEDCDQIHVDDVSSDDNGQDLSTYNFSADGFHSSAP
O08575|EYA 295 SVRIGLMMEEMIFNLADTHLFFNDLEDCDQIHVDDVSSDDNGQDLSTYNFSTDGFHSTAP
*************************************************** ***** **

O00167|EYA 361 GANLCLGSGVHGGVDWMRKLAFRYRRVKEMYNTYKNNVGGLIGTPKRETWLQLRAELEAL
O08575|EYA 355 GASLCLGTGVHGGVDWMRKLAFRYRRVKEMYNTYRNNVGGLIGAPKRETWLQLRAELEAL
** **** ************************** ******** ****************

O00167|EYA 421 TDLWLTHSLKALNLINSRPNCVNVLVTTTQLIPALAKVLLYGLGSVFPIENIYSATKTGK
O08575|EYA 415 TDLWLTHSLKALNLINSRPNCVNVLVTTTQLIPALAKVLLYGLGSVFPIENIYSATKTGK
************************************************************

O00167|EYA 481 ESCFERIMQRFGRKAVYVVIGDGVEEEQGAKKHNMPFWRISCHADLEALRHALELEYL
O08575|EYA 475 ESCFERIMQRFGRKAVYIVIGDGVEEEQGAKKHNMPFWRISCHADLEALRHALELEYL
***************** ****************************************



## Claims

1. An *in vitro* method for activating a subset of genes in a, preferably human, KDR⁺ cell population, preferably in an enriched or substantially homogeneous, preferably human, KDR⁺ cell population, wherein the activation of said subset of genes promotes hematopoietic stem cell (HSC) generation, said subset comprising at least the following 7, preferably human, genes selected from the group consisting of: Spata2 ((spermatogenesis-associated 2), Aass (aminoadipate-semialdehyde synthase), Dctd (Gene ID: 1642), Eif4enif1 (eukaryotic translation initiation factor 4E nuclear import factor 1), Guca1a (guanylate cyclase activator 1A), Eya2 (Eyes Absent Homolog 2) and Net1 (Neuroepithelial Cell Transforming 1), and wherein the method comprises inducing temporary endogenous activation of the subset of genes in the KDR⁺ cell population.

2. The method of claim 1, wherein the activation of said subset of genes is achieved by transforming, transfecting, or transducing the KDR⁺ cell population with one or more vectors, preferably viral vectors, comprising:
a. sequences encoding transcriptional activators specific to the subset of genes;
b. other sequences that indirectly activate the subset of genes, wherein said other sequences include guide RNAs targeting promoter regions, epigenetic modifiers such as histone acetyltransferases or DNA demethylases, or regulatory elements that enhance endogenous expression of the subset of genes; or
c. sequences encoding one or more of the genes within the subset;

3. The method of claim 2, wherein the viral vectors are lentiviral vectors modified to allow transient expression of the sequences, such as through the use of self-inactivating (SIN) vectors or inducible promoters that can be deactivated to provide temporary activation of the subset of genes in the KDR⁺ cell population.

4. The method of claim 2, wherein the viral vectors are adenoviral vectors, allowing transient expression of the sequences without genomic integration, thereby providing temporary activation of the subset of genes in the KDR⁺ cell population.

5. The method of claim 1, wherein the activation of said subset of genes is achieved using CRISPR activation (CRISPRa) technology, wherein guide RNAs target promoter or enhancer regions of the genes within the subset, and endogenous expression is upregulated through the recruitment of transcriptional activators or chromatin-modifying complexes.

6. The method of claim 5, wherein the CRISPR activation (CRISPRa) system utilizes a catalytically inactive Cas9 (dCas9) fused to a transcriptional activator, such as VP64, p300, or a synergistic activation mediator (SAM) complex, to enhance the expression of the subset of genes in the KDR⁺ cell population.

7. The method of claim 5, wherein the guide RNAs are optimized to target multiple regulatory elements within the promoter and enhancer regions of the genes in the subset, providing synergistic upregulation of endogenous gene expression.

8. The method of claim 1, wherein the activation of said subset of genes is achieved by delivering synthetic mRNAs encoding said genes to the KDR⁺ cell population.

9. The method according to any one of claims 1 to 8, wherein the method further comprises culturing the KDR⁺ cells under conditions conducive to HSC maturation following gene activation to obtain a cell population comprising HSCs.

10. A cell population comprising HSCs obtained or obtainable by the method of claim 9, wherein at least about 10%, 25%, 50%, 75%, 80%, 90% or more of this cell population (as determined by flow cytometry) is phenotypically **characterized by** the expression of the following hematopoietic stem cell (HSC) markers: Lin- Sca1+ ckit+ (LSK).

11. A vector configured to induce hematopoietic stem cell (HSC) generation by transforming or transducing KDR⁺ cell populations *in vivo* or *in vitro,* wherein said vector is capable of activating at least a subset of genes selected from the group consisting of Spata2, Aass, Dctd, Eif4enif1, Guca1a, Eya2 and Net1, and wherein the activation promotes the differentiation of KDR⁺ cells into hematopoietic stem cells with the ability for multilineage reconstitution.

12. The vector of claim 11, wherein the vector is selected from the list consisting of:
a. a lentiviral vector modified as a self-inactivating (SIN) vector, ensuring transient gene activation in KDR⁺ cells;
b. an adenoviral vector configured for transient expression of the subset of genes without genomic integration.

13. The vector of claim 11, wherein the vector utilizes a CRISPR activation (CRISPRa) system, comprising a catalytically inactive Cas9 (dCas9) fused to a transcriptional activator and guide RNAs targeting the promoter regions of the subset of genes for temporary endogenous activation.

14. The composition according to claim 10 or the vector according to any one of claims 11 to 13, for use in a method of treatment for reconstituting erythroid, myeloid, and/or lymphoid compartments in a subject suffering from hematopoietic failure.

15. The composition according to any one of claims 10 to 11 or the vector according to any one of claims 12 to 17, wherein the composition or vector is for use in treating bone marrow aplasia, or for used in enhancing hematopoietic recovery in subjects undergoing myeloablative therapy.
